# EUROPEAN PATENT SPECIFICATION

(11) **EP 2 964 758 B1**
(45) Date of publication and mention of the grant of the patent: **27.05.2020**
(21) Application number: 14718161.4
(22) Date of filing: 05.03.2014
(51) Int. Cl.: C12N 9/16, A61K 38/00, C12Q 1/34, G01N 33/68

(54) **PRODUCTION OF CATALYTICALLY ACTIVE TYPE I SULFATASE**
HERSTELLUNG VON KATALYTISCH AKTIVER TYP-I-SULFATASE
PRODUCTION DE SULFATASE DE TYPE I CATALYTIQUEMENT ACTIVE

(30) Priority: 05.03.2013 US 201361773034 P; 15.03.2013 US 201361790530 P
(43) Date of publication of application: 13.01.2016
(73) Proprietor: Oxyrane UK Limited, Manchester M1 2AP (GB)
(72) Inventor: VERVECKEN, Wouter, B-9860 Landskouter (BE); RYCKAERT, Stefan Simonne Prudent Eugène Christine, B-9040 Belgium (BE); VALEVSKA, Albena Vergilieva, B-9800 Belgium (BE)
(74) Representative: Michalík, Andrej
(86) International application number: PCT/IB2014/059464
(87) International publication number: WO 2014/136065

(56) References cited:
- AU-A1- 2012 206 984
- FR-A1- 2 954 349
- US-A1- 2009 186 011
- ALEXANDER RODRIGUEZ ET AL: "Production of recombinant human N-acetylgalactosamine-6-sulfate sulfatase enzyme in Pichia pastoris", MOLECULAR GENETICS AND METABOLISM, vol. 108, no. 2, 1 February 2013 (2013-02-01), pages S79-S80, XP055139790, ISSN: 1096-7192, DOI: 10.1016/j.ymgme.2012.11.211
- CARLSON B L ET AL: "Function and structure of a prokaryotic formylglycine-generating enzyme", JOURNAL OF BIOLOGICAL CHEMISTRY, AMERICAN SOCIETY FOR BIOCHEMISTRY AND MOLECULAR BIOLOGY, US, vol. 283, no. 29, 18 July 2008 (2008-07-18), pages 20117-20125, XP002712380, ISSN: 0021-9258, DOI: 10.1074/JBC.M800217200 [retrieved on 2008-04-04]
- KAREN DE POURCQ ET AL: "Engineering the yeast Yarrowia lipolytica for the production of therapeutic proteins homogeneously glycosylated with Man8GlcNAc2 and Man5GlcNAc2", MICROBIAL CELL FACTORIES, BIOMED CENTRAL, LONDON, NL, vol. 11, no. 1, 1 May 2012 (2012-05-01), page 53, XP021129012, ISSN: 1475-2859, DOI: 10.1186/1475-2859-11-53
- ALMECIGA CARLOS J ET AL: "Production of an active recombinant human N-acetylgalactosamine-6-sulfate sulfatase enzyme in Pichia pastoris", MOLECULAR GENETICS AND METABOLISM, vol. 111, no. 2, 27 January 2014 (2014-01-27), XP028821615, 27.01.2014 ISSN: 1096-7192, DOI: 10.1016/J.YMGME.2013.12.023
- LANDGREBE J ET AL: "The human SUMF1 gene, required for posttranslational sulfatase modification, defines a new gene family which is conserved from pro- to eukaryotes", GENE, ELSEVIER, AMSTERDAM, NL, vol. 316, 16 October 2003 (2003-10-16), pages 47-56, XP004464790, ISSN: 0378-1119, DOI: 10.1016/S0378-1119(03)00746-7

## Description

### TECHNICAL FIELD

This document relates to methods and materials, including genetically engineered fungal cells, useful for the production of type I sulfatase enzymes or functional fragments thereof, in their catalytically active form.

### BACKGROUND

Sulfatases catalyze the hydrolysis of sulfate esters (e.g., sulfates) of substrates including steroids, complex cell surface carbohydrates and proteins. The absence of an active individual type I sulfatase has been implicated in a number of pathophysical conditions, namely lysosomal storage disorders which includes mucopolysaccharidoses (MPS), such as MPSII, MPSIIA, MPSIVA, MPSVI, and metachromatic leukodystrophy.

Thus, a method of making type I sulfatase with a high level of activity for use in such disorders would be extremely valuable.

### SUMMARY

This document provides methods and materials based on, *inter alia*, the discovery by the inventors that catalytically active type I sulfatases can be produced in recombinant fungi expressing type I sulfatase-activating enzymes (FGEs) from a variety of species.

The present invention provides subject-matter as set forth in any one and all of (1) to (7) below:
(1) A method for making a type I sulfatase in an active form,
   (I) the method comprising:
      a) providing a fungal cell genetically engineered to produce a protein with the type I sulfatase activating activity of a Formylglycine Generating Enzyme (FGE); and
      b) introducing into the cell a nucleic acid encoding a type I sulfatase, wherein the encoded type I sulfatase, without an activation step, is in an inactive form,
         wherein, after the introduction, the cell produces, or produces at an increased level, the type I sulfatase in an active form, and wherein, after step (b), the cell, or the progeny thereof, is cultivated at a pO₂ of 5% - 40%; or
   (II) the method comprising:
      a) providing a fungal cell genetically engineered to produce a type I sulfatase, wherein the type I sulfatase, without an activation step, is in an inactive form; and
      b) introducing into the cell a nucleic acid encoding a protein with the type I sulfatase activating activity of a Formylglycine Generating Enzyme (FGE),
         wherein, after the introduction, the cell produces, or produces at an increased level, the type I sulfatase in an active form, and wherein, after step (b), the cell, or the progeny thereof, is cultivated at a pO₂ of 5% - 40%.
(2) The method as set forth in (1) above, wherein the fungal cell is a *Yarrowia lipolytica* cell.
(3) The method as set forth in (1) above, wherein:
   (AA) the protein with the type I sulfatase activating activity of a FGE comprises:
      (a) a mature wild type FGE polypeptide;
      (b) a functional fragment of a mature wild type FGE polypeptide comprising at least 50 consecutive amino acids of the mature wild type FGE;
      (c) a polypeptide with at least 80% identity to (a);
      (d) a polypeptide with at least 90% identity to (b);
      (e) (a) but with no more than 10 conservative substitutions; or
      (f) (b) but with no more than 5 conservative substitutions; or
   (AB) the method is as defined in (AA) and the mature wild type FGE polypeptide is mature wild type protein SCO7548; or
   (AC) the method is as defined in (AA) and the mature wild type FGE polypeptide is mature wild type protein Rv0712; or
   (AD) the method is as defined in (AA) and the mature wild type FGE polypeptide is mature wild type sulfatase modifying factor 1; or
   (AE) the method is as defined in (AA) and the mature wild type FGE polypeptide is mature wild type C-alpha-formylglycine-generating enzyme; or
   (AF) the method is as defined in (AA) and the mature wild type FGE polypeptide is mature wild type sulfatase-modifying factor 1; or
   (AG) the method is as defined in any of (1) or (2) above or in (AA) and the protein with the type I sulfatase activating activity of a FGE is a prokaryotic protein with the type I sulfatase activating activity of a FGE; or
   (AH) the method is as defined in (AG) and the prokaryote is *Mycobacterium tuberculosis* or *Streptomyces coelicolor*; or
   (AI) the method is as defined in any of (1) or (2) above or in (AA) and the protein with the type I sulfatase activating activity of a FGE is a protein with the type I sulfatase activating activity of a eukaryotic FGE; or
   (AJ) the method is as defined in (AI) and the eukaryote is *Homo sapiens*, *Bos taurus*, *Hemicentrotus pulcherrimus, Tupaia chinensis, Monodelphis domestica, Gallus gallus, Dendroctonus ponderosa*, or *Columba livia*; or
   (AK) the method is as defined in any of (1) or (2) above or in any of (AA) to (AJ) and the protein with the type I sulfatase activating activity of a FGE further comprises an ER targeting motif; or
   (AL) the method is as defined in (AK) and the ER targeting motif is fused to the C-terminus of the protein with the type I sulfatase activating activity of a FGE polypeptide; or
   (AM) the method is as defined in (AK) and the ER targeting motif is fused to the N-terminus of the protein with the type I sulfatase activating activity of a FGE polypeptide; or
   (AN) the method is as defined in any one of (AK) to (AM) and the ER targeting motif comprises HDEL (SEQ ID NO: 1); or
   (AO) the method is as defined in any one of (AK) to (AM) and the ER targeting motif comprises KDEL (SEQ ID NO: 3); or
   (AP) the method is as defined in any one of (AK) to (AM) and the ER targeting motif comprises DDEL (SEQ ID NO: 4) or RDEL (SEQ ID NO: 33); or
   (AQ) the method is as defined in any one of (AK) to (AM) and the ER targeting motif comprises a yeast MNS1 transmembrane anchor polypeptide; or
   (AR) the method is as defined in (AQ) and the yeast MNS1 transmembrane anchor polypeptide comprises the *Yarrowia lipolytica* MNS 1 transmembrane anchor polypeptide; or
   (AS) the method is as defined in any one of (AK) to (AM) and the ER targeting motif comprises a yeast WBP1 transmembrane anchor polypeptide; or
   (AT) the method is as defined in (AS) and the yeast WBP1 transmembrane anchor polypeptide comprises the *Yarrowia lipolytica* WBP1 transmembrane anchor polypeptide; or
   (AU) the method is as defined in any one of (1) or (2) above or in any one of (AA) to (AT) and the type I sulfatase or the protein with the type I sulfatase activating activity of a FGE further comprises a leader or signal sequence; or
   (AV) the method is as defined in (AU) and the leader or signal sequence is an exogenous leader or signal sequence; or
   (AW) the method is as defined in (AU) and the leader or signal sequence is an endogenous leader or signal sequence; or
   (AX) the method is as defined in any one of (AU) to (AW) and the leader or signal sequence is Lip2pre; or
   (AY) the method is as defined in any one of (1) or (2) above or in any one of (AA) to (AX) and the method further comprises introducing into the cell a nucleic acid encoding a polypeptide capable of effecting mannosyl phosphorylation, or a functional fragment thereof; or
   (AW) the method is as defined in (AY) and the polypeptide capable of effecting mannosyl phosphorylation is selected from the group consisting of MNN4, PNO1, and MNN6; or
   (AZ) the method is as defined in any one of (1) or (2) above or in any one of (AA) to (AW) and the method further comprises introducing into the cell a nucleic acid encoding a mannosidase, or a functional fragment thereof, capable of hydrolyzing a terminal mannose-1-phospho-6-mannose moiety to a terminal phospho-6-mannose; or
   (BA) the method is as defined in (AZ) and the mannosidase is the family 92 glycoside hydrolase CcMan5 from *Cellulosimicrobium cellulans*; or
   (BB) the method is as defined in (AZ) and the mannosidase is also capable of removing a mannose residue bound by an alpha 1,2 linkage to the underlying mannose in the terminal mannose-1-phospho-6-mannose moiety; or
   (BC) the method is as defined in (BB) and the mannosidase is a family 38 glycoside hydrolase selected from the group consisting of a *Canavalia ensiformis* (Jack Bean) mannosidase and *Yarrowia lipolytica* AMS1 mannosidase; or
   (BD) the method is as defined in (AZ) and the method further comprises introducing into the cell a nucleic acid encoding a mannosidase, or a functional fragment thereof, that is capable of removing a mannose residue bound by an alpha 1,2 linkage to the underlying mannose in the terminal mannose-1-phospho-6-mannose moiety; or
   (BE) the method is as defined in (BD) and the mannosidase is selected from the group consisting of the family 38 glycoside hydrolase *Canavalia ensiformis* (Jack Bean) mannosidase, the family 38 glycoside hydrolase *Yarrowia lipolytica* AMS1 mannosidase, the family 47 glycoside hydrolase *Aspergillus satoi* As mannosidase, and the family 92 glycoside hydrolase *Cellulosimicrobium cellulans* CcMan4 mannosidase; or
   (BF) the method is as defined in any one of (1) or (2) above or in any one of (AA) to (BE) and the method further comprises introducing into the cell a nucleic acid encoding a trafficking protein, or a functional fragment thereof, wherein the trafficking protein or functional fragment thereof, directs the protein with the type I sulfatase activating activity of a FGE to the endoplasmic reticulum (ER) of the cell; or
   (BG) the method is as defined in (BF) and the trafficking protein is Protein Disulfide Isomerase (PDI); or
   (BH) the method is as defined in (BF) and the trafficking protein is Endoplasmic Reticulum Protein 44 (Erp44) or human SUMF2; or
   (BI) the method is as defined in (BF) and the trafficking protein, or functional fragment thereof, binds to the protein with the type I sulfatase activating activity of a FGE; or
   (BJ) the method is as defined in any one of (1) or (2) above or in any one of (AA) to (BI) and the fungal cell is a yeast cell; or
   (BK) the method is as defined in (BJ) and the yeast cell is a cell of a methylotrophic yeast; or
   (BL) the method is as defined in (BK) and the methylotrophic yeast is selected from the group comprising *Pichia pastoris, Pichia methanolica, Ogataea minuta*, and *Hansenula polymorpha*; or
   (BM) the method is as defined in any one of (1) or (2) above or in any one of (AA) to (BI) and the fungal cell is a cell of a filamentous fungus; or
   (BN) the method is as defined in (BM) and the filamentous fungus is selected from a group consisting of: *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus ustus, Aspergillus versicolor, Trichoderma,* and *Neurospora*; or
   (BO) the method is as defined in any one of (1) or (2) above or in any one of (AA) to (BN) and the type I sulfatase is a human type I sulfatase; or
   (BP) the method is as defined in any one of (1) or (2) above or in any one of (AA) to (BN) and the type I sulfatase is iduronate sulfatase; or
   (BQ) the method is as defined in any one of (1) or (2) above or in any one of (AA) to (BN) and the type I sulfatase is sulfamidase; or
   (BR) the method is as defined in any one of (1) or (2) above or in any one of (AA) to (BQ) and the cell comprises a deficiency in OCH1 activity; or
   (BS) the method is as defined in (AA) and the protein with the type I sulfatase activating activity of a FGE comprises any one of (a) - (f) and the mature wild type FGE polypeptide is a mature wild type *Columba livia* FGE polypeptide and further comprises a yeast MNS 1 transmembrane anchor polypeptide; or
   (BT) the method is as defined in (BS) and the protein with the type I sulfatase activating activity of a FGE comprises the amino acid sequence set forth in SEQ ID NO: 63; or
   (BU) the method is as defined in (AA) and the mature wild type FGE polypeptide is:
      (i) a mature wild type FGE of *Hemicentrotus pulcherrimus* having the amino acid sequence set forth in SEQ ID NO: 13, a mature wild type FGE of *Gallus gallus* having the amino acid sequence set forth in SEQ ID NO: 47, a mature wild type FGE of *Dendroctonus ponderosa* having the amino acid sequence set forth in SEQ ID NO: 49, or a mature wild type FGE of *Columba livia* having the amino acid sequence set forth in SEQ ID NO: 51; or
      (ii) a functional mature FGE having an amino acid sequence that is at least 80% identical to any one of the amino acid sequences of (i).
(4) The method as set forth in any one of (1)-(3) above, wherein the method results in the production of a type I sulfatase in which greater than 10%, 20%, 30%, 40%, 50%, 60%, 70%, 80%, or 90% of the molecules of the type I sulfatase comprise a formylglycine residue in the active site.
(5) The method as set forth in any one of (1)-(3) above, wherein the method results in the production of a type I sulfatase in which greater than 95% of the molecules of the type I sulfatase comprise a formylglycine residue in the active site.
(6) The method as set forth in any one of (1)-(3) above, wherein the method results in the production of a type I sulfatase in which 100% of the molecules of the type I sulfatase comprise a formylglycine residue in the active site.
(7) The method as set forth in (1) above, wherein the protein with the type I sulfatase activating activity of a FGE is encoded by a nucleotide sequence comprising
   (i) the nucleic acid sequence set out in any one of SEQ ID NOs: 14, 48, 50 or 52; or
   (ii) a nucleic acid sequence that is at least 80% identical to any one of the nucleic acid sequences of (i) and encodes a mature functional FGE; or
   (iii) a nucleic acid sequence that hybridizes to a complement of any one of the nucleic acid sequences of (i) under high stringency and encodes a mature functional FGE.

The present document also describes a first method for making a type I sulfatase, or a functional fragment of a type I sulfatase, in an active form. The method includes: (a) providing a fungal cell genetically engineered such that, when transformed with a polynucleotide encoding a type I sulfatase, or a functional fragment of a type I sulfatase, the cell has the ability to produce the type I sulfatase, or the functional fragment of the type I sulfatase in an active form, or an increased level of the type I sulfatase, or the functional fragment of the type I sulfatase in an active form; and (b) introducing into the cell a nucleic acid encoding the type I sulfatase, or a functional fragment of the type I sulfatase. The encoded type I sulfatase, or functional fragment of the type I sulfatase, without an activation step, is in an inactive form. After the introduction, the cell produces, or produces at an increased level, the type I sulfatase, or a functional fragment of the type I sulfatase, in an active form.

The document also features a second method for making a type I sulfatase, or a functional fragment of a type I sulfatase, in an active form. The method includes: (a) providing a fungal cell genetically engineered to produce a produce a protein with the type I sulfatase activating activity of a Formylglycine Generating Enzyme (FGE); and (b) introducing into the cell a nucleic acid encoding a type I sulfatase, or a functional fragment of the type I sulfatase. The encoded type I sulfatase, or the encoded functional fragment of the type I sulfatase, without an activation step, is in an inactive form. After the introduction, the cell produces, or produces at an increased level, the type I sulfatase, or the functional fragment of the type I sulfatase, in an active form.

In addition, the document describes a third method for making a type I sulfatase, or a functional fragment of a type I sulfatase, in an active form. The method includes: (a) providing a fungal cell genetically engineered to produce a type I sulfatase, or a functional fragment of the type I sulfatase, the encoded type I sulfatase, or the encoded functional fragment of the type I sulfatase, without an activation step, being in an inactive form; and (b) introducing into the cell a nucleic acid encoding a produce a protein with the type I sulfatase activating activity of a Formylglycine Generating Enzyme (FGE). After the introduction, the cell produces, or produces at an increased level, the type I sulfatase, or functional fragment of the type I sulfatase, in an active form.

In the second and third methods, the protein with the type I sulfatase activating activity of a FGE can include or be any of (a) - (f) as follows: (a) a mature wild type FGE polypeptide; (b) a functional fragment of a mature wild type FGE polypeptide comprising at least 50 (e.g., at least: 60; 70; 80; 90; 100; 125; 150; 175; 200; 225; 250; 275; 300; 325; 350; 400; 450; 500; or more) consecutive amino acids of the mature wild type FGE; (c) a polypeptide with at least 80% (e.g., at least: 85%; 88%; 90%; 92%; 95%; 98%; 99%; or 99.5%) identity to (a); (d) a polypeptide with at least 90% (e.g., at least: 92%; 95%; 98%; 99%; or 99.5%) identity to (b); (e) (a) but with no more than 10 (e.g., no more than 8; 7; 6; 5; 4; 3; 2; or 1) conservative substitution(s); or (f) (b) but with no more than 5 (e.g., no more than 4; 3; 2; or 1) conservative substitutions(s). In all of the methods in which an FGE is involved, the FGE can be the following mature wild type proteins and functional fragments of the mature wild type proteins as well as variants (listed above) of either: mature wild type protein SCO7548; mature wild type protein Rv0712; mature wild type sulfatase modifying factor 1; mature wild type C-alpha-formylglycine-generating enzyme; or mature wild type sulfatase-modifying factor 1. Also useful for the production methods of the disclosure are fusion proteins containing any of the mature wild type proteins, functional fragments, and variants of both. Moreover, the FGE can be a prokaryotic FGE (e.g., a FGE from *Mycobacterium tuberculosis* or *Streptomyces coelicolor*)*.* Alternatively, the FGE can be a eukaryotic FGE (e.g., a FGE of *Homo sapiens*, *Bos taurus, Hemicentrotus pulcherrimus, Tupaia chinensis, Monodelphis domestica, Gallus gallus, Dendroctonus ponderosa,* or *Columba livia*).

In addition, in any of the active type I sulfatase production methods described in the present disclosure, any of the proteins with the type I sulfatase activating activity of a FGE, fusion proteins containing such proteins, can further include a ER targeting motif such as HDEL (SEQ ID NO: 1), KDEL (SEQ ID NO: 3), DDEL (SEQ ID NO: 4), RDEL (SEQ ID NO: 33), a yeast MNS1 transmembrane anchor polypeptide (such as the *Yarrowia lipolytica* MNS1 transmembrane anchor polypeptide), a yeast WBP1 transmembrane anchor polypeptide (such as the *Yarrowia lipolytica* WBP1 transmembrane anchor polypeptide), or the transmembrane parts of Secretory-12 (SEC12), Glucosidase-1 (GLS1), or STaurosporine Temperature Sensitive-3 (STT3). The ER targeting motif can be fused to the N-terminus or the C-terminus of any of the proteins with the type I sulfatase activating activity of a FGE or fusion proteins containing such proteins.

In all of the active type I sulfatase production methods described herein, the type I sulfatase, or the functional fragment of the type I sulfatase, as well as any of the proteins with the type I sulfatase activating activity of a FGE can be fused in frame to a leader or signal sequence. The leader or signal can be an exogenous or an endogenous leader or signal sequence. The leader or signal sequence can be, for example, the yeast Lip2pre leader sequence.

All the active type I sulfatase production methods described herein can further include introducing into the cell a nucleic acid encoding a polypeptide capable of effecting mannosyl phosphorylation (e.g., MNN4, PNO1, MNN6, or a functional fragment of such a polypeptide).

All the active type I sulfatase production methods described herein can also include introducing into the cell a nucleic acid encoding a mannosidase, or a functional fragment of a mannosidase, capable of hydrolyzing a terminal mannose-1-phospho-6-mannose moiety to a terminal phospho-6-mannose; this mannosidase can be, for example, the family 92 glycoside hydrolase CcMan5 from *Cellulosimicrobium cellulans.* The mannosidase, or the functional fragment of the mannosidase, can also be capable of removing a mannose residue bound by an alpha 1,2 linkage to the underlying mannose in the terminal mannose-1-phospho-6-mannose moiety; such a mannosidase can be a family 38 glycoside hydrolase selected from the group consisting of a *Canavalia ensiformis* (Jack Bean) mannosidase and *Yarrowia lipolytica* AMS1 mannosidase. Alternatively, or in addition, these methods can further include introducing into the cell a nucleic acid encoding a mannosidase, or a functional fragment of the mannosidase, that is capable of removing a mannose residue bound by an alpha 1,2 linkage to the underlying mannose in the terminal mannose-1-phospho-6-mannose moiety; this mannosidase can be the family 38 glycoside hydrolase *Canavalia ensiformis* (Jack Bean) mannosidase, the family 38 glycoside hydrolase *Yarrowia lipolytica* AMS1 mannosidase, the family 47 glycoside hydrolase *Aspergillus satoi* (AS) mannosidase, or the family 92 glycoside hydrolase *Cellulosimicrobium cellulans* CcMan4 mannosidase.

All of the active type I sulfatase production methods described herein can further include introducing into the cell a nucleic acid encoding a trafficking protein, or a functional fragment of the trafficking protein, which can direct any of the proteins with the type I sulfatase activating activity of a FGE to the endoplasmic reticulum (ER) of the cell. The trafficking protein can be Protein Disulfide Isomerase (PDI), Endoplasmic Reticulum Protein 44 (Erp44), or the inactive homolog of FGE in humans named SUMF2 (sulfatase modifying factor 2). The trafficking protein, or the functional fragment of the trafficking protein, can bind to the any of the proteins with the type I sulfatase activating activity of a FGE.

In all the active type I sulfatase production methods described herein, the fungal cell can be a yeast cell, e.g., a *Yarrowia lipolytica* cell, an *Arxula adeninivorans* cell, or a cell of another related species of dimorphic yeast. Alternatively, the yeast cell can be *a Saccharomyces cerevisiae* cell or a cell of a methylotrophic yeast (e.g., a cell of *Pichia pastoris, Pichia methanolica, Ogataea minuta,* or *Hansenula polymorpha*). Alternatively, in all the above methods, the fungal cell can be a cell of a filamentous fungus (e.g., *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus ustus, Aspergillus versicolor, Trichoderma,* or *Neurospora*).

In any of the active type I sulfatase production methods described herein, the cell can include a deficiency in Outer Chain elongation (OCH1) protein 1 activity.

In all of the active type I sulfatase production methods described herein, coding sequences encoding type I sulfatase, or the functional fragment of the type I sulfatase coding sequence, any of the proteins with the type I sulfatase activating activity of a FGE, as well as other proteins (such as trafficking proteins, proteins capable of producing mannosyl phosphorylation, mannosidases, or functional fragments and variants of such proteins) can be under the control of a yeast (e.g., *Yarrowia lipolytica*, *Arxula adeninivorans*, or other related dimorphic yeast species) promoter for expression in a yeast cell. Each of the coding sequences can be under the control of the same yeast promoter, or the coding sequences can be under the control of different yeast promoters. For example, the yeast promoter can be hp4d or POX2.

In any of the active type I sulfatase production methods described herein, the coding sequences of the type I sulfatase, the functional fragment of the type I sulfatase, any of the proteins with the type I sulfatase activating activity of a FGE, as well as other proteins (such as trafficking proteins, proteins capable of producing mannosyl phosphorylation, mannosidases, or functional fragments and variants of such proteins) can be present as a single copy or as multiple copies, e.g., 2 copies. Each of the copies can be under the control of the same yeast promoter, or each of the copies can be under the control of different yeast promoters. For example, the yeast promoter for the first copy can be hp4d and the yeast promoter for the second copy can be POX2.

In all of the active type I sulfatase production methods described herein, the sulfatase can a human type I sulfatase. The type I sulfatase can be, for example, iduronate sulfatase (hIDS) or sulfamidase (SGSH).

In all of the three active type I sulfatase production methods described above, after step (b), the cell, or the progeny thereof, can be cultivated at high pO₂. The cell, or the progeny of the cell, can be cultivated at a pO₂ of, for example, 5% - 40% (e.g., 10%, 15%, 20%, 25%, 30%, or 35%).

All of the active type I sulfatase production methods described herein can result in the production of a type I sulfatase, or a functional fragment of the type I sulfatase, in which greater than 10%, 20%, 30%, 40%, 50%, 60%, 70%, 80%, 90%, 95%, or 100% of the molecules of the type I sulfatase or functional fragment contain a formylglycine residue in the active site. It is to be understood that an activation of 100% is detected at a detection limit of 0.5% and therefore includes values from 99.5% to 100%.

In any of the active type I sulfatase production methods described herein, the protein with the type I sulfatase activity of a FGE (i) can include or be any of (a) - (f) as follows: (a) a mature wild type FGE polypeptide; (b) a functional fragment of a mature wild type FGE polypeptide comprising at least 50 (e.g., at least: 60; 70; 80; 90; 100; 125; 150; 175; 200; 225; 250; 275; 300; 325; 350; 400; 450; 500; or more) consecutive amino acids of the mature wild type FGE; (c) a polypeptide with at least 80% (e.g., at least: 85%; 88%; 90%; 92%; 95%; 98%; 99%; or 99.5%) identity to (a); (d) a polypeptide with at least 90% (e.g., at least: 92%; 95%; 98%; 99%; or 99.5%) identity to (b); (e) (a) but with no more than 10 (e.g., no more than 8; 7; 6; 5; 4; 3; 2; or 1) conservative substitution(s); and (f) (b) but with no more than 5 (e.g., no more than 4; 3; 2; or 1) conservative substitutions(s), where the mature wild type FGE polypeptide is a mature wild type *Columba livia* FGE. Moreover, the protein with the type I sulfatase activity of a FGE can further include a yeast MNS1 transmembrane anchor polypeptide. The protein with the type I sulfatase activating activity of a FGE can have or contain the amino acid sequence set forth in SEQ ID NO: 63.

The present document also features an active type I sulfatase, or a functional fragment of an active type I sulfatase, produced by the any of the active type I sulfatase production methods described herein. The document also describes a method of treating a subject having, or suspected of having, a disorder treatable with a type I sulfatase, the method comprising administering to the subject the active type I sulfatase, or a functional fragment of the type I sulfatase, produced by any of the active type I sulfatase production methods described herein. The disorder can be, for example, a lysosomal storage disorder or a disease of some other subcellular compartment or organelle (e.g., the Golgi or microsomes). The disorder can be, without limitation, metachromatic leukodystrophy, Hunter disease, Sanfilippo disease A & D, Morquio disease A, Maroteaux-Lamy disease, X-linked ichthyosis, Chondrodysplasia Punctata 1, and multiple sulfatase deficiency (MSD). Moreover, in these methods, the subject can be a human.

The document also features an isolated fungal cell that contains a nucleic acid encoding a the protein with the type I sulfatase activity of a FGE. The protein with the type I sulfatase activity of a FGE (i) can include or be any of (a) - (f) as follows: (a) a mature wild type FGE polypeptide; (b) a functional fragment of a mature wild type FGE polypeptide comprising at least 50 (e.g., at least: 60; 70; 80; 90; 100; 125; 150; 175; 200; 225; 250; 275; 300; 325; 350; 400; 450; 500; or more) consecutive amino acids of the mature wild type FGE; (c) a polypeptide with at least 80% (e.g., at least: 85%; 88%; 90%; 92%; 95%; 98%; 99%; or 99.5%) identity to (a); (d) a polypeptide with at least 90% (e.g., at least: 92%; 95%; 98%; 99%; or 99.5%) identity to (b); (e) (a) but with no more than 10 (e.g., no more than 8; 7; 6; 5; 4; 3; 2; or 1) conservative substitution(s); and (f) (b) but with no more than 5 (e.g., no more than 4; 3; 2; or 1) conservative substitutions(s) This fungal cell can also contain a nucleic acid encoding a type I sulfatase, a functional fragment of a type I sulfatase or a fusion protein containing a type I sulfatase or a functional fragment thereof. The encoded type I sulfatase, or the encoded functional fragment of the type I sulfatase, without the action of an activating factor on it, is an inactive form.

In all fungal cells containing a nucleic acid encoding an FGE, the FGE can be any of the following mature wild type proteins (or functional fragments thereof) and variants (listed above) of either: mature wild type protein SCO7548; mature wild type protein Rv0712; mature wild type sulfatase modifying factor 1; mature wild type C-alpha-formylglycine-generating enzyme; or mature wild type sulfatase-modifying factor 1. Also useful are fungal cells producing fusion proteins containing any of the mature wild type proteins, functional fragments, and variants of both. Moreover, the FGE can be a prokaryotic FGE (e.g., a FGE from *Mycobacterium tuberculosis* or *Streptomyces coelicolor*). Alternatively, the FGE can be a eukaryotic FGE (e.g., a FGE of *Homo sapiens*, *Bos taurus, Hemicentrotus pulcherrimus, Tupaia chinensis, Monodelphis domestica, Gallus gallus, Dendroctonus ponderosa,* or *Columba livia*).

In addition, in any of the fungal cells of the disclosure, any of the proteins with the type I sulfatase activating activity of a FGE, fusions containing such proteins, can further include a ER targeting motif such as HDEL (SEQ ID NO: 1), KDEL (SEQ ID NO: 3), DDEL (SEQ ID NO: 4), RDEL (SEQ ID NO: 33), a yeast MNS1 transmembrane anchor polypeptide (such as the *Yarrowia lipolytica* MNS1 transmembrane anchor polypeptide), oyeast WBP1 transmembrane anchor polypeptide (such as the *Yarrowia lipolytica* WBP1 transmembrane anchor polypeptide), or the transmembrane parts of Secretory-12 (SEC12), Glucosidase-1 (GLS1), or STaurosporine Temperature Sensitive-3 (STT3). The ER targeting motif can be fused to the N-terminus or the C-terminus of any of the proteins with the type I sulfatase activating activity of a FGE, or fusion proteins containing such proteins

In all of the fungal cells of this disclosure, the type I sulfatase, or the functional fragment of the type I sulfatase, as well as any of the proteins with the type I sulfatase activating activity of a FGE of the can be fused in frame to a leader or signal sequence. The leader or signal can be an exogenous or an endogenous leader or signal sequence. The leader or signal sequence can be, for example, the Lip2pre leader sequence.

All the fungal cells of this disclosure can further include a nucleic acid encoding a polypeptide capable of effecting mannosyl phosphorylation (e.g., MNN4, PNO1, MNN6, or a functional fragment of such a polypeptide).

In addition, all the fungal cells of this disclosure can also contain a nucleic acid encoding a mannosidase, or a functional fragment of a mannosidase, capable of hydrolyzing a terminal mannose-1-phospho-6-mannose moiety to a terminal phospho-6-mannose; this mannosidase can be, for example, the family 92 glycoside hydrolase CcMan5 from *Cellulosimicrobium cellulans.* The mannosidase, or the functional fragment of the mannosidase, can also be capable of removing a mannose residue bound by an alpha 1,2 linkage to the underlying mannose in the terminal mannose-1-phospho-6-mannose moiety; such a mannosidase can be a family 38 glycoside hydrolase selected from the group consisting of a *Canavalia ensiformis* (Jack Bean) mannosidase and *Yarrowia lipolytica* AMS1 mannosidase. Alternatively, or in addition, the fungal cells can further include a nucleic acid encoding a mannosidase, or a functional fragment of the mannosidase, that is capable of removing a mannose residue bound by an alpha 1,2 linkage to the underlying mannose in the terminal mannose-1-phospho-6-mannose moiety; this mannosidase can be the family 38 glycoside hydrolase *Canavalia ensiformis* (Jack Bean) mannosidase, the family 38 glycoside hydrolase *Yarrowia lipolytica* AMS1 mannosidase, the family 47 glycoside hydrolase *Aspergillus satoi* (AS) mannosidase, or the family 92 glycoside hydrolase *Cellulosimicrobium cellulans* CcMan4 mannosidase.

Furthermore, all of the fungal cells of this disclosure can also include a nucleic acid encoding a trafficking protein, or a functional fragment of the trafficking protein, which can direct any of the proteins with the type I sulfatase activating activity of a FGE to the endoplasmic reticulum (ER) of the cell. The trafficking protein can be Protein Disulfide Isomerase (PDI), Endoplasmic Reticulum Protein 44 (Erp44), or the inactive homolog of FGE in humans named SUMF2. The trafficking protein, or the functional fragment of the trafficking protein, can bind to the any of the proteins with the type I sulfatase activating activity of a FGE.

The fungal cell of this disclosure can be a yeast cell, e.g., a *Yarrowia lipolytica* cell, an *Arxula adeninivorans* cell or a cell of another related species of dimorphic yeast. Alternatively, the yeast cell can be a *Saccharomyces cerevisiae* cell or a cell of a methylotrophic yeast (e.g., a cell of *Pichia pastoris, Pichia methanolica, Ogataea minuta,* or *Hansenula polymorpha*). Alternatively, in all the above methods, the fungal cell can be a cell of a filamentous fungus (e.g., *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus ustus, Aspergillus versicolor, Trichoderma,* or *Neurospora*).

In any of the fungal cells of this disclosure, the cell can include a deficiency in Outer Chain elongation (OCH1) protein 1 activity.

In all of the fungal cells of this disclosure, coding sequences encoding type I sulfatase, or the functional fragment of the type I sulfatase coding sequence, any of the proteins with the type I sulfatase activating activity of a FGE, as well as other proteins (such as trafficking proteins, proteins capable of producing mannosyl phosphorylation, mannosidases, or functional fragments and variants of such proteins) can be under the control of a yeast (e.g., *Yarrowia lipolytica, Arxula adeninivorans*, or other related dimorphic yeast species) promoter for expression in a yeast cell. Each of the coding sequences can be under the control of the same yeast promoter, or the coding sequences can be under the control of different yeast promoters. For example, the yeast promoter can be hp4d or POX2. Moreover, any can be present as a single copy or as multiple copies, e.g. 2 copies. Each of the copies can be under the control of the same yeast promoter, or each of the copies can be under the control of different yeast promoters. For example, the yeast promoter for the first copy can be hp4d and the yeast promoter for the second copy can be POX2.

In all of the fungal cells of this disclosure, the sulfatase can a human type I sulfatase. The type I sulfatase can be, for example, iduronate sulfatase (hIDS) or sulfamidase (SGSH).

In any of the fungal cells of this disclosure, the protein with the type I sulfatase activity of a FGE (i) can include or be any of (a) - (f) as follows: (a) a mature wild type FGE polypeptide; (b) a functional fragment of a mature wild type FGE polypeptide comprising at least 50 (e.g., at least: 60; 70; 80; 90; 100; 125; 150; 175; 200; 225; 250; 275; 300; 325; 350; 400; 450; 500; or more) consecutive amino acids of the mature wild type FGE; (c) a polypeptide with at least 80% (e.g., at least: 85%; 88%; 90%; 92%; 95%; 98%; 99%; or 99.5%) identity to (a); (d) a polypeptide with at least 90% (e.g., at least: 92%; 95%; 98%; 99%; or 99.5%) identity to (b); (e) (a) but with no more than 10 (e.g., no more than 8; 7; 6; 5; 4; 3; 2; or 1) conservative substitution(s); and (f) (b) but with no more than 5 (e.g., no more than 4; 3; 2; or 1) conservative substitutions(s),where the mature wild type FGE polypeptide is a mature wild type *Columba livia* FGE. Moreover, the protein with the type I sulfatase activity of a FGE can further include a yeast MNS1 transmembrane anchor polypeptide. The protein with the type I sulfatase activating activity of a FGE can have or contain the amino acid sequence set forth in SEQ ID NO: 63.

The document also describes a substantially pure culture comprising fungal cells which are genetically engineered to comprise a protein with the type I sulfatase activating activity of a FGE. The fungal cells further comprising a nucleic acid encoding a type I sulfatase, or a functional fragment thereof, wherein the encoded type I sulfatase, or functional fragment thereof, without the action of an activating factor on it, is an inactive form. The fungal cells of the culture can have any of the attributes, characteristics, and properties of the fungal cells described above and can express any of the wild type proteins, functional fragments of such proteins, and variants described herein.

In any of the above methods or fungal cells, the mature wild type FGE can be: (i) a mature wild type FGE of *Hemicentrotus pulcherrimus* having the amino acid sequence set forth in SEQ ID NO: 13, a mature wild type FGE of *Gallus gallus* having the amino acid sequence set forth in SEQ ID NO: 47, a mature wild type FGE of *Dendroctonus ponderosa* having the amino acid sequence set forth in SEQ ID NO: 49, or a mature wild type FGE of *Columba livia* having the amino acid sequence set forth in SEQ ID NO: 51; (ii) a functional mature FGE having an amino acid sequence that is at least 80% identical to any one of the amino acid sequences of (i).

Moreover, in any of the above methods or fungal cells, the protein with the type I sulfatase activating activity of a FGE can be encoded by a nucleotide sequence having: (i) the nucleic acid sequence set out in any one of SEQ ID NOs: 14, 48, 50 or 52; or (ii) a nucleic acid sequence that is at least 80% identical to any one of the nucleic acid sequences of (i) and encodes a functional FGE; or (iii) a nucleic acid sequence that hybridizes to a complement of any one of the nucleic acid sequences of (i) under high stringency and encodes a functional FGE.

Unless otherwise defined, all technical and scientific terms used herein have the same meaning as commonly understood by one of ordinary skill in the art to which the embodiments of this document belong. Although methods and materials similar or equivalent to those described herein can be used in the practice or testing of these embodiments, the exemplary methods and materials are described below. The materials, methods, and examples are illustrative only and not intended to be limiting.

Other features and advantages of the materials and methods recited in this disclosure, e.g., methods of activating type I sulfatases or functional fragments thereof, will be apparent from the following detailed description, and from the claims.

### DESCRIPTION OF DRAWINGS

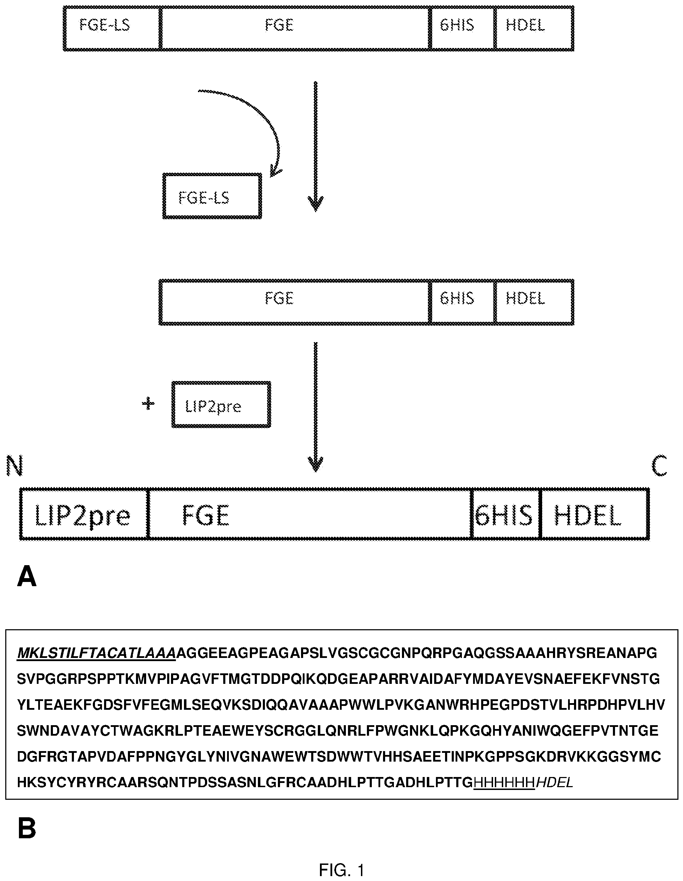

FIG. 1A is a schematic representation of the recombinant Formylglycine Generating Enzyme (rFGE) fusion proteins produced by genetically engineered cells described herein and how their native leader sequence (FGE-LS) is replaced with the LIP2 pre leader (signal) sequence. Each fusion protein contains, N-terminus to C-terminus, the Lip2 pre leader sequence (LIP2pre), a mature FGE (FGE; e.g., mature *Bos taurus* FGE), a hexahistidine tag (6HIS), and a HDEL (SEQ ID NO: 1) tetrapeptide. FIG. 1B is a depiction of the amino acid sequence (SEQ ID NO: 32) of a fusion protein as described for FIG. 1A in which the mature FGE is *Bos taurus* FGE (BtFGE). LIP2pre is in bold italics and underlined, the mature BtFGE is in plain bold text, the 6HIS is in plain text and underlined, and the HDEL is in plain italics text.

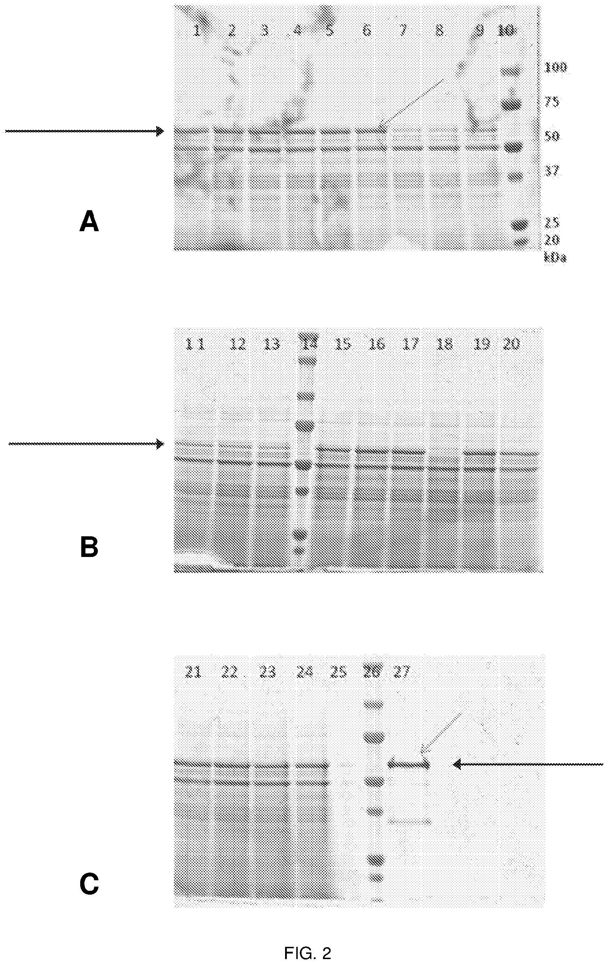

FIGs. 2A, 2B, and 2C are photographs of sodium dodecyl sulfate polyacrylamide gel electrophoresis (SDS-PAGE) analyses detecting recombinant human iduronate-2 sulfatase (rhIDS) as expressed in *Y. lipolytica* at 28°C. The gel depicted in FIG. 2A shows expression of rIDS from T146 (OXYY1828; BtFGE) clones A-F in lanes 1-6 and T147 (OXYY1831; ScFGE) clones A, B and C in lane 7-9, respectively. The gel depicted in FIG. 2B shows expression of rhIDS from T147 (OXYY1831; ScFGE) clones D-F in lanes 11-13 and from T148 (OXYY1801; HpFGE) clones A-F in lanes 15-20. The gel depicted in FIG. 2C shows expression of rhIDS from T126 (OXYY1827; hFGE) clones A-D in lanes 21-24. Molecular weight markers are shown in lanes 10, 14 and 26 of FIG 2A, 2B, and 2C, respectively. Lane 27 contains ELAPRASE® (idursulfase) which is a commercial human IDS preparation. The arrows in the photographs indicate detection of rhIDS protein.

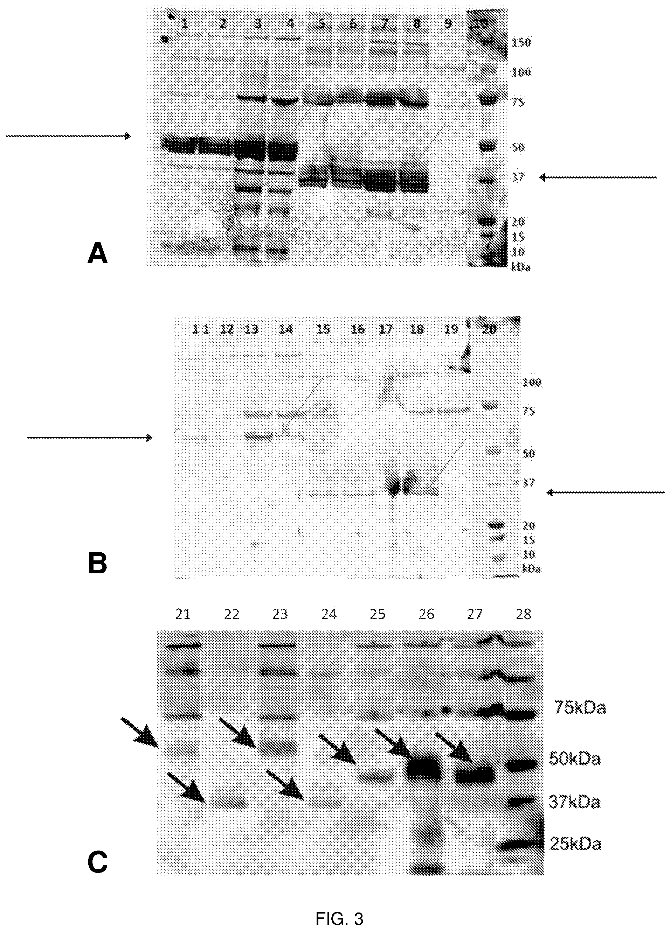

FIG. 3A, 3B, and 3C are digital images of a chemiluminiscent reaction showing the Western blot analysis of rFGE under reducing conditions. The image depicted in FIG. 3A shows expression of rFGE from T146 (OXYY1828; BtFGE) clones A and B at 28°C (lanes 1 and 2) and at 20°C (lanes 3 and 4) in lanes 1-4, and from T147 (OXYY1831; ScFGE) clones A and B at 28°C (lanes 5 and 6) and 20°C (lanes 7 and 8) in lanes 5-8. The image depicted in FIG. 3B shows expression of rFGE from T148 (OXYY1801; HpGFE) clones A and B at 28°C (lanes 11 and 12) and at 20°C (lanes 13 and 14) in lanes 11-14, and from T153 (OXYY1802; MtFGE) clones A and B at 28°C (lanes 15 and 16) and 20°C (lanes 17 and 18) in lanes 15-18. FGE expression for T126 (OXYY1827; hFGE) at 28°C and 20°C is shown in lane 9 of FIG. 3A and lane 19 of FIG. 3B respectively. FIG. 3C shows expression of rFGE from a clone of T148 (OXYY1801; HpGFE) grown at 28°C in lane 21; a clone of T153 (OXYY1802; MtFGE) grown at 28°C in lane 22; a clone of T148 (OXYY1801) grown at 20°C in lane 23; a clone of T153 (OXYY1802; MtFGE) grown at 20°C in lane 24; a clone of T161 (OXYY1798, BtFGE) grown at 28°C in lane 25; a clone of T156 (OXYY1803; BtFGE and hPDI) grown at 28°C in lane 26; and a clone of T146 (OXYY1828; BtFGE) grown at 28°C in lane 27. Molecular weight markers are shown in lanes 10, 20, and 28 of FIG 3A, 3B, and 3C respectively. The arrows in the photographs indicate detection of rFGE protein.

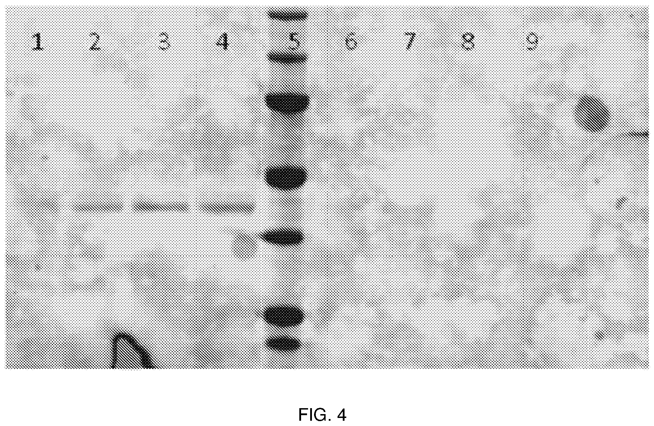

FIG. 4 is a digital image of a chemiluminiscent reaction displaying the Western blot analysis of rFGE under reducing and non-reducing conditions. Expression of rFGE from T126 (OXYY1827; hFGE) clones A and B at 28°C (lanes 1 and 2) and at 20°C (lanes 3 and 4) under reducing conditions are shown in lanes 1-4 and under non-reducing conditions in lanes 6-9. Molecular weight markers are shown in lane 5.

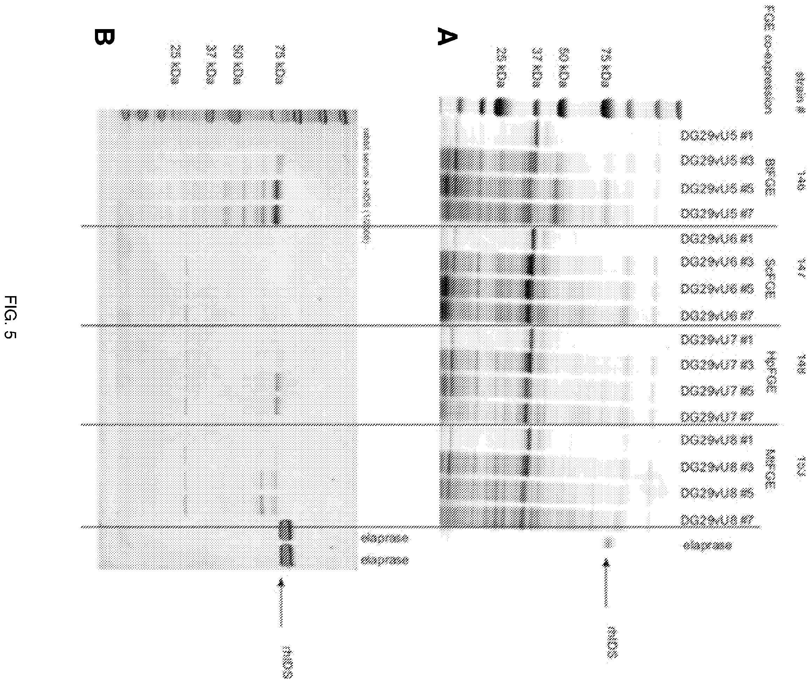

FIGs. 5A and 5B are a photograph of an SDS-PAGE analysis (FIG. 5A) and a digital image of a chemiluminiscent reaction of a Western blot analysis (FIG. 5B) showing rhIDS expression in the presence of FGE co-expression in strains T146 (OXYY1828), T147 (OXYY1831), T148 (OXYY1801) and T153 (OXYY1802) which co-express B*os taurus* FGE (BtFGE), *Streptomyces coelicolor* FGE (ScFGE), *Hemicentrotus pulcherrimus* (HpFGE), and *Mycobacterium tuberculosis* FGE (MtFGE), respectively. The expression of each clone was analyzed at 4 timepoints. The arrows in the images indicate detection of rIDS protein. Molecular weight markers are shown the left-most lane of the photograph and the digital image. ELAPRASE® was included in the indicated lanes.

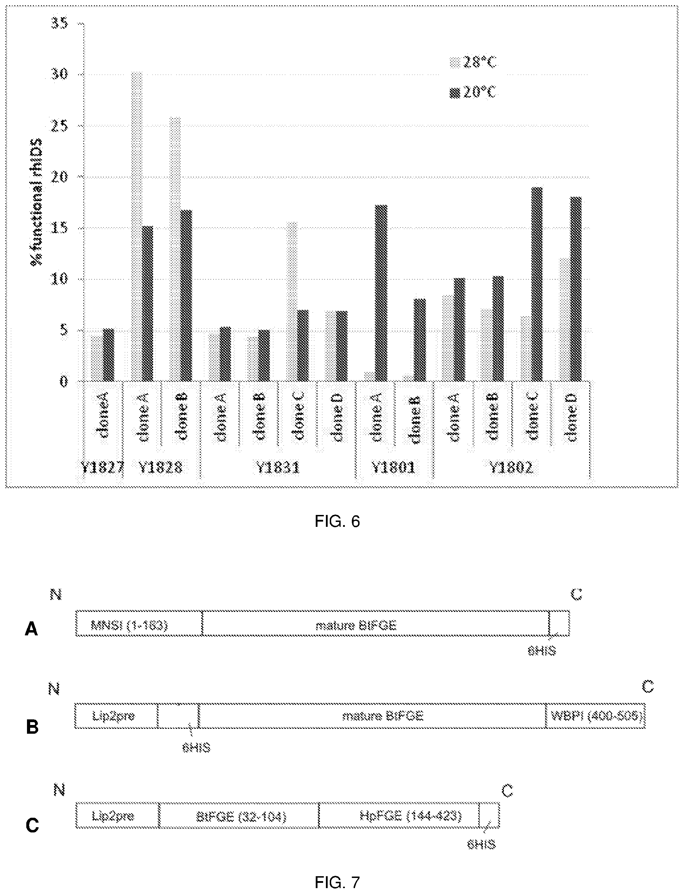

FIG 6 is a bar graph depicting the percentages of total rhIDS produced at 28°C and 20°C in heterologous *Y. lipolytica* cells co-expressing rIDS and rFGE of different origins that are functional.
FIG. 7A is a diagrammatic representation of the rFGE and *Yarrowia lipolytica* MNS1 mannosidase anchorage domain containing fusion proteins described in Example 10. Each fusion protein contains, N-terminus to C-terminus, amino acids 1-163 of MNS1 (SEQ ID NO:26), a mature FGE (e.g., BtFGE), and a hexahistidine (6HIS) tag; FIG. 7B is a diagrammatic representation of the rFGE and *Yarrowia lipolytica* WBP1 oligosaccharyl transferase anchorage domain containing fusion proteins described in Example 11. Each fusion protein contains, N-terminus to C-terminus, the Lip2 signal sequence, a hexahistidine (6HIS) tag, a mature FGE (e.g., BtFGE), and the C-terminal 118 amino acids (amino acids 400-505 ofXP_502492.1) of *Yarrowia lipolytica* WBP1 (SEQ ID NO:28); FIG. 7C is a diagrammatic representation of the chimeric protein consisting of of the N-terminal end of BtFGE (amino acids 32-104 of NP_001069544, fused to the C-terminal end of HpFGE (amino acids 144-423 of BAJ83907) described in Example 12. The Lip2 leader was fused to the N-terminal end of the chimeric coding sequence and at the C-terminus a 6HIS tag was added, followed by the HDEL tetrapeptide.

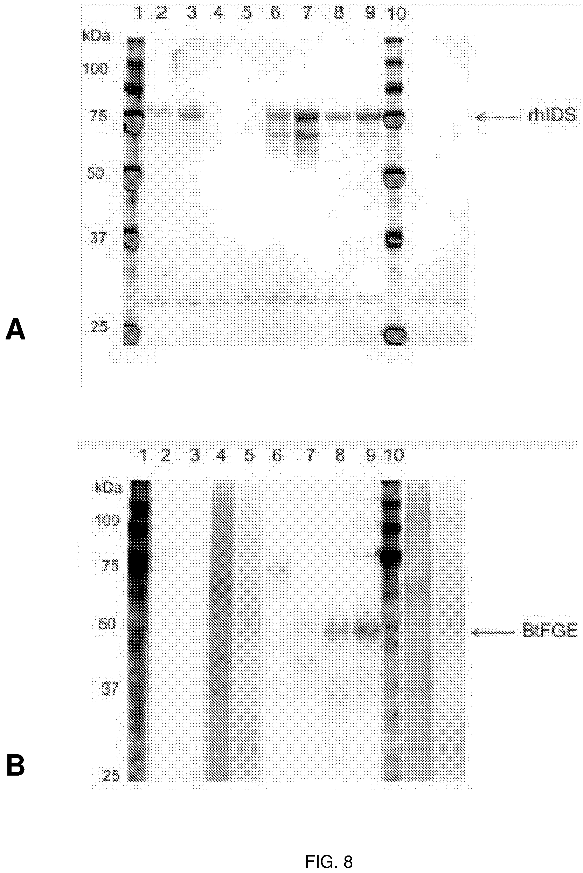

FIG. 8A is a digital image of a chemiluminiscent reaction displaying the Western blot analysis (by Western blot with a rabbit anti-human IDS antiserum) for expression of rhIDS from strains co-expressing rhIDS (1 copy, POX2 driven) and rFGE (1 copy POX2 driven and 1 copy Hp4d driven) grown under fed-batch fermentation. The *Y. lipolytica-*produced IDS is visible at an approximate MW of 76 kDa. The supernatant was analyzed for six rIDS expressing strains at the endpoint of the fermentation. Lane 1 is the MW Marker; lane 2 is ChFGE (the chimeric protein described in Example 12) co-expressed at 20°C; lane 3 is ChFGE co-expressed at 28°C; lane 6 is BtFGE-WBP1 co-expression; lane 7 is BtFGE-MNS1 co-expression; and lanes 8-9 are the control strains co-expressing BtFGE-HDEL (1 copy, POX2 driven). FIG. 8B is a digital image of a chemiluminiscent reaction displaying the Western blot analysis for expression of rFGE using anti-his antibody (A00186-100, Genscript). The contents in each lane correspond to those in FIG. 8A.

### DETAILED DESCRIPTION

Type I sulfatases require a unique co- or post- translational amino acid modification in the active center of the enzyme to enable their activation, specifically, a cysteine in the active site is oxidized to the aldehyde-containing a C_{α}-Formylglycine residue. In humans, a single enzyme, sulfatase modifying factor-1 (SUMF1) or formylglycine generating enzyme (FGE) is responsible for activation of all type I sulfatases. Inactivity of FGE leads to the production of catalytically inactive type I sulfatases, the cause of a rare but fatal lysosomal storage disease called Multiple Sulfatase Deficiency (MSD) (Dierks et al (2003), Cell, 113, 435-444).

The formylglycine (FGly) residue of an activated type I sulfatase is located in a 13 amino acid consensus sequence called the *sulfatase motif.* Formylglycine can be generated from a cysteine residue within the core motif [CX(P/A)XR] or a serine residue within the core motif [S/CXPXR]. Each 'X' in this core motif represents any amino acid. In eukaryotic organisms, the conversion starting from cysteine is the only known route. Conversion starting from serine is predominantly found in anaerobic bacteria as the conversion of the thiol group of cysteine to an aldehyde group catalyzed by FGly-generating enzyme is oxygen-dependent. The mechanism by which FGly is formed by FGE is still unknown. It has been determined that the structure of FGE-substrate complexes includes pentamer and heptamer peptides that mimic the substrate. It was shown that the peptides isolate a cavity that can serve as a binding site for molecular oxygen (Roeser et al (2006), Proceedings of the National Academy of Sciences of the United States of America, 103, 81-86). The inactive homolog of FGE in humans, SUMF2 is also a trafficking protein.

The enzyme acts on the newly synthesized type I sulfatase when it is entering the endoplasmic reticulum (ER) and when it is still in its unfolded form. Once the nascent type I sulfatase is fully folded, the target cysteine becomes incorporated in the active site cleft where it is inaccessible for modification by FGE, resulting in the production of an inactive type I sulfatase. In humans, the FGE lacks a C-terminal ER retrieval signal and is also dependent on interaction with other proteins for its correct localization. Both Protein Disulfide Isomerase (PDI) and Endoplasmic Reticulum Protein (Erp44), two ER resident proteins, have been shown to interact with FGE and are thought to be involved in the control of FGE trafficking and functioning via non-covalent hetero-oligomeric interaction (Fraldi et al (2008), Human molecular genetics, 17, 2610-2621 and Mariappan et al (2008), The Journal of Biological Chemistry, 283, 6375-6383).

The interaction is likely to occur through the N-terminal extension of FGE that confers not only ER localization to FGE but is also indispensable for its *in vivo* catalytic activity.

In humans, a paralog of FGE has also been identified as the SUMF2 gene product. It is catalytically inactive and has substantial expression levels (Gande et al (2008), The FEBS Journal, 275, 1118-1130). There is evidence that FGE and its paralog act in concert by forming heterodimers. Also, *in vivo* the paralog seems to contact nascent type I sulfatases hereby forming ternary complexes with FGE (Zito et al (2005), EMBO Reports, 6, 655-660). The human paralog is retrieved to the ER through a C-terminal KDEL-like signal, but does not seem to act as a standalone retention factor for ER localization of FGE. Conferring ER localization of human FGE through fusion for the HDEL (SEQ ID NO: 1; corresponding nucleic acid sequence set forth in SEQ ID NO: 2) tetrapeptide has been shown to be sufficient and effective. An alternative approach to obtain correct localization of the FGE protein to the ER is to fuse a transmembrane anchor to the FGE. For example, the transmembrane anchor of a yeast α-1,2-mannosidase (MNS1) or a yeast wheat germ agglutinin-binding protein (WBP1) such as those of *Saccharomyces cerevisiae* or *Yarrowia lipolytica* can be used. *Y. lipoytica* MNS1 has Accession No: XP_502939.1 and *Yarrowia lipolytica* WBP1 has Accession No.: XP_502492.1.

Human FGE (hFGE) is encoded by the SUMF1 gene. The immature protein is a protein of 374 residues, including a signal sequence of 33 amino acids (SEQ ID NO: 23) which induces the translocation of the protein into the ER. The amino acid sequence of mature hFGE is designated SEQ ID NO:9. A single N-glycosylation site is also present at Asn141 (residue number is that of the immature hFGE protein). The folding of the protein shows remarkably little secondary structure (Roeser et al (2006), Proceedings of the National Academy of Sciences of the United States of America, 103, 81-86). Human FGE is a compact monomeric molecule that is stabilized by two intramolecular disulfide bridges and two calcium molecules. It has a binding groove for the CXPXR substrate peptide which has two cysteines, Cys₃₃₆ and Cys₃₄₁ (residue numbers are those of the immature hFGE protein), involved in the formation of FGly, as discussed above. SUMF1 homologues have been identified across a large variety of species and are highly conserved (Sardiello et al (2005), Human Molecular Genetics, 14, 3203-3217). However, thus far, no FGE homologues have been identified in *Yarrowia lypolytica* or other fungal species despite the presence of a type I sulfatase gene (Sardiello et al (2005), Human Molecular Genetics, 14, 3203-3217).

In eukaryotes, the minimal canonical sequence CxPxR (where each x is any amino acid) in the active site of type I sulfatases is recognized by an FGly-generating enzyme, which catalyzes the oxidation of the cysteine residue to an aldehyde-bearing Ca-formylglycine residue. This reaction is a multistep redox reaction that involves disulfide bridge formation and requires molecular oxygen and a reducing agent but does not require a cofactor or a metal ion (Roeser et al (2006), Proceedings of the National Academy of Sciences of the United States of America, 103, 81-86). This conversion from cysteine to formylglycine is an activation step that is essential for the type I sulfatase activity of the type I sulfatases.

In general, this document discloses methods and materials for the production and isolation of catalytically active type I sulfatases in recombinant fungal cells. Also described are methods to produce active type I sulfatases in the presence of FGEs and, optionally, other polypeptides, such as trafficking molecules, mannosidases, and polypeptides that effect mannose phosphorylation. The utilization of FGEs from varying sources is included.

Also included in this document are methods and materials for hydrolyzing a terminal mannose-1-phospho-6-mannose linkage or moiety on an N-glycan on a type I sulfatase to phospho-6-mannose (also referred to as "mannose-6-phosphate" herein) ("uncapping") and hydrolyzing a terminal alpha-1,2 mannose, alpha-1,3 mannose and/or alpha-1,6 mannose linkage or moiety of such a phosphate-containing N-glycan ("demannosylating"). Also described are methods of facilitating uptake of a glycoprotein (e.g., an activated type I sulfatase) by a mammalian cell as both uncapping and demannosylation (either by separate enzymes or a single enzyme) are required to achieve mammalian cellular uptake of glycoproteins via mannose-6-phosphate receptors. For further details on these methods, see for example, PCT application PCT/IB2011/002770 or U.S. Application Publication No. US2013/0267473-A1.

The methods and materials described herein are useful for making agents for the treatment of any condition in which it is desired to administer an activated type I sulfatase (e.g., an activated type I sulfatase, or a functional fragment thereof) to a subject (e.g., a human patient with the condition). They are particularly useful for producing agents for treating subjects with lysosomal storage disorders (LSDs) in which one or more type I sulfatases are absent, inactive, or insufficiently active. Moreover, they can be used to treat MSD in which afflicted subjects produce catalytically inactive FGE. LSDs are a diverse group of hereditary metabolic disorders characterized by the accumulation of storage products in the lysosomes due to impaired activity of catabolic enzymes involved in their degradation. The build-up of storage products leads to cell dysfunction and progressive clinical manifestations. Deficiencies in catabolic enzymes can be corrected by enzyme replacement therapy (ERT), provided that the administered enzyme can be targeted to the lysosomes of the diseased cells. Lysosomal enzymes typically are glycoproteins that are synthesized in the ER, transported via the secretory pathway to the Golgi, and then recruited to the lysosomes. Using the methods and materials described herein, a microbe-based production process can be used to obtain therapeutic type I sulfatases. In some embodiments these type I sulfatases have demannosylated phosphorylated N-glycans. Thus, the methods and materials described herein are useful for preparing type I sulfatases for the treatment of disorders such as, for example, LSDs. Relevant disorders include, without limitation, metachromatic leukodystrophy (arylsulfatase A), Hunter disease (iduronate 2-sulfatase), Sanfilippo disease A (N-sulfoglucosamine sulfohydrolase) & D (N-acetylglucosamine-6-sulfatas), Morquio disease A (Galactosamine-6-sulfatase), Maroteaux-Lamy disease (arylsulfatase B), X-linked ichthyosis (steroid sulfatase), Chondrodysplasia Punctata 1 (arylsulfatase E), and MSD. For other relevant disorders, see, for example, Diez-Roux et al. (2005), Annu Rev Genomics Hum Genet, 6,355-379.

As used herein, a type I sulfatase that is in an "active form" is one that has more than 5% (e.g., more than: 7.5%; 10%; 20%; 30%; 40%; 50%; 60%; 70%; 80%; 90%; 100%; or even more) of the type I sulfatase activity of a wild-type type I sulfatase obtained from a mammalian cell with a normal level of FGE with normal activity and with wild type expression levels of sulfatases and with the specificity of the relevant wild type type I sulfatase.

As used herein, the terms "inactive type I sulfatase", "type I sulfatase in an inactive form", "type I sulfatase that is not in an active form", "type I sulfatase that is not active", and similar terms refer to a type I sulfatase that has no more than 5% (e.g., no more than: 2.5%; 1.0%; 0.1%; 0.01%; or none) of the type I sulfatase activity of a wild-type type I sulfatase obtained from a cell with a normal level of FGE with normal activity and with wild type expression levels of sulfatases and with the specificity for the relevant wild type type I sulfatase. This document provides methods that include the use of nucleic acids encoding type I sulfatases and FGEs.

The terms "nucleic acid" and "polynucleotide" are used interchangeably herein, and refer to both RNA and DNA, including cDNA, genomic DNA, synthetic DNA, and DNA (or RNA) containing nucleic acid analogs. Polynucleotides can have any three-dimensional structure. A nucleic acid can be double-stranded or single-stranded (i.e., a sense strand or an antisense strand). Non-limiting examples of polynucleotides include genes, gene fragments, exons, introns, messenger RNA (mRNA), transfer RNA, ribosomal RNA, siRNA, micro-RNA, ribozymes, cDNA, recombinant polynucleotides, branched polynucleotides, plasmids, vectors, isolated DNA of any sequence, isolated RNA of any sequence, nucleic acid probes, and primers, as well as nucleic acid analogs.

"Polypeptide" and "protein" are used interchangeably herein and mean any peptide-linked chain of amino acids, regardless of length or post-translational modification. Typically, a polypeptide described herein (e.g., a type I sulfatase or an FGE) is isolated when it constitutes at least 60%, by weight, of the total protein in a preparation, e.g., 60% of the total protein in a sample. In some embodiments, a polypeptide described herein consists of at least 75%, at least 90%, or at least 99%, by weight, of the total protein in a preparation.

The term "active site" is a defined region of an enzyme where a substrate binds to subsequently undergo a chemical reaction. The active site is the region in which the chemical reaction occurs. The active site of an enzyme can be found in a cleft or pocket that can be lined with amino acid residues that participates in recognition of a substrate. Residues that directly participate in a catalytic reaction mechanism of a substrate are in the active site. In certain instances, as described herein, a residue of the enzyme requires post translational modification. In some instances, the residue is in the active site of the protein (i.e., formylglycine in the active site of type I sulfatase). Substrates bind to the active site of the enzyme through chemical interactions selected from a group comprising hydrogen bonds, hydrophobic interactions, electrostatic interactions, van de Waal's forces, and temporary covalent interactions. In further embodiments, a combination of these to form the enzyme-substrate complex can be used. The active site can modify the reaction mechanism to change the activation energy of the reaction involving the substrate. The consensus active site of an enzyme or the consensus sequence within an active site is the highly homologous region of conserved residues which are shared by a family of proteins (i.e. enzymes).

The term "activation step", as used herein with respect to the production of a type I sulfatase in an active form, or a functional fragment thereof, refers to an intracellular process that occurs before, during, or after the intracellular folding of the type I sulfatase polypeptide, or the functional fragment thereof, that results in the type I sulfatase polypeptide, or the functional fragment thereof, after it is fully folded, being in an active form. Such an activation step can be, but is not necessarily, effected by an activating factor.

As used herein, the term "activating factor" refers to an enzyme (e.g, an FGE), or a functional fragment thereof, that, before, during or after the intracellular folding of a type I sulfatase, or a functional fragment thereof, acts on the type I sulfatase, or functional fragment thereof, such that the fully folded type I sulfatase, or fully folded functional fragment thereof, is in an active form.

As used herein, the term "at an increased level", when used with respect to the production of a type I sulfatase in an active form, or a functional fragment thereof, in a fungal cell expressing an exogenous nucleic acid encoding an activating factor (e.g., an FGE), refers to the increased level of the type I sulfatase in an active form, or the functional fragment thereof, produced in the fungal cell as compared to the level produced by a control fungal cell not expressing an exogenous nucleic acid encoding an activating factor.

An "isolated nucleic acid" refers to a nucleic acid that is separated from other nucleic acid molecules that are present in a naturally-occurring genome, including nucleic acids that normally flank one or both sides of the nucleic acid in a naturally-occurring genome (e.g. a yeast genome). The term "isolated" as used herein with respect to nucleic acids also includes any non-naturally-occurring nucleic acid sequence, since such non-naturally-occurring sequences are not found in nature and do not have immediately contiguous sequences in a naturally-occurring genome. An isolated nucleic acid can be, for example, a DNA molecule, provided one of the nucleic acid sequences normally found immediately flanking that DNA molecule in a naturally-occurring genome is removed or absent. Thus, an isolated nucleic acid includes, without limitation, a DNA molecule that exists as a separate molecule (e.g., a chemically synthesized nucleic acid, or a cDNA or genomic DNA fragment produced by PCR or restriction endonuclease treatment) independent of other sequences as well as DNA that is incorporated into a vector, an autonomously replicating plasmid, a virus (e.g., any paramyxovirus, retrovirus, lentivirus, adenovirus, or herpes virus), or into the genomic DNA of a prokaryote or eukaryote. In addition, an isolated nucleic acid can include an engineered nucleic acid such as a DNA molecule that is part of a hybrid or fusion nucleic acid. A nucleic acid existing among hundreds to millions of other nucleic acids within, for example, cDNA libraries or genomic libraries, or gel slices containing a genomic DNA restriction digest, is not considered an isolated nucleic acid.

The term "functional fragment" as used herein refers to a peptide fragment of a protein that is shorter (in terms of amino acid number) than the corresponding mature, full-length, wild-type protein and has at least 25% (e.g., at least: 30%; 40%; 50%; 60%; 70%; 75%; 80%; 85%; 90%; 95%; 98%; 99%; 100%; or even greater than 100%) of the activity of the corresponding mature, full-length, wild-type protein. The functional fragment can generally, but not always, be comprised of a continuous region of the protein (i.e., be composed of consecutive amino acids of the protein) wherein the region has functional activity. The term "functional fragment" also refers to a peptide fragment of a protein that can be made active or have the ability to be activated by means of an activation step to have the activity of the corresponding activated mature, full-length, wild-type protein. The functional fragment can contain the activation site of type I sulfatase in an active form or not in an active form; the latter type of functional fragment would have the ability to be activated by the action of an FGE. The consensus amino acid sequence of the type I sulfatase active site is described herein. Candidate functional fragments of type I sulfatases can therefore be produced by one skilled in the art using well established methods. Their activity can be confirmed by well-established methods such as those described in the working examples disclosed here. Functional fragments will generally be at least 20 (e.g., at least: 30; 40; 60; 70; 80; 90; 100; 125; 150; 175; 200; 225; 250; 275; 300; 325; 350; 400; 450; 500; or more) amino acids long.

A "functional mature FGE" as used herein with reference to a variant mature FGE polypeptides or a variant nucleic acid encoding a variant FGE polypeptide has at least 25% (e.g., at least: 30%; 40%; 50%; 60%; 70%; 75%; 80%; 85%; 90%; 95%; 98%; 99%; 100%; or even greater than 100%) of the activity of the corresponding mature, full-length, wild-type polypeptide.

This document also describes (i) functional variants of the proteins used in the methods of the document and (ii) functional variants of the functional fragments described above. Functional variants of the proteins and functional fragments can contain additions, deletions, or substitutions relative to the corresponding wild-type sequences. Proteins with substitutions will generally have not more than 50 (e.g., not more than one, two, three, four, five, six, seven, eight, nine, ten, 12, 15,20,25,30,35,40, or 50) conservative amino acid substitutions. This applies to any of the above-mentioned proteins and functional fragments. A conservative substitution is a substitution of one amino acid for another with similar characteristics. Conservative substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The nonpolar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Any substitution of one member of the above-mentioned polar, basic or acidic groups by another member of the same group can be deemed a conservative substitution. By contrast, a nonconservative substitution is a substitution of one amino acid for another with dissimilar characteristics.

Deletion variants can lack one, two, three, four, five, six, seven, eight, nine, ten, 11, 12, 13, 14, 15, 16, 17, 18, 19, or 20 amino acid segments (of two or more amino acids) or non-contiguous single amino acids.

Substitutions and deletions in type I sulfatases will preferably not be in the active site. In particular, a cysteine residue that is converted to a formylglycine upon activation should not be substituted or deleted.

Additions (addition variants) include fusion proteins containing: (a) any of the above-described proteins or a fragment thereof; and (b) internal or terminal (C or N) irrelevant or heterologous amino acid sequences. In the context of such fusion proteins, the term "heterologous amino acid sequences" refers to an amino acid sequence other than (a). A heterologous sequence can be, for example a sequence used for purification of the recombinant protein (e.g., FLAG, polyhistidine (e.g., hexahistidine (SEQ ID NO: 7; corresponding nucleic acid sequence set forth in SEQ ID NO: 8)), hemagluttanin (HA), glutathione-S-transferase (GST), or maltosebinding protein (MBP)). Heterologous sequences also can be proteins useful as diagnostic or detectable markers, for example, luciferase, green fluorescent protein (GFP), or chloramphenicol acetyl transferase (CAT). In some embodiments, the fusion protein contains a signal sequence or leader sequence from another protein. In certain host cells (e.g., yeast host cells), expression and/or secretion of the target protein can be increased through use of a heterologous signal sequence. For example, the signal (leader) sequence may be the Lip2pre sequence. In some embodiments, the fusion protein can contain a carrier (e.g., keyhole limpet hemocyanin (KLH)) useful, e.g., in eliciting an immune response for antibody generation) or ER or Golgi apparatus retention signals. Soluble proteins that reside in the lumen of the ER are known to have at their C terminus, *inter alia*, the tetrapeptides KDEL (SEQ ID NO: 3) or HDEL (SEQ ID NO: 1; corresponding nucleic acid sequence set forth in SEQ ID NO: 2). These tetrapeptides, and others such as DDEL (SEQ ID NO:4) and RDEL (SEQ ID NO: 33), function as retrieval motifs essential for the precise sorting of these proteins along the secretory pathway. Their presence on the terminal end of a luminal protein signals trafficking to the ER. Additional retention signals that may be used in a fusion protein include transmembrane anchors such the transmembrane anchors of yeast ER/Golgi residing proteins (e.g., *S. cervisiae* or *Y. lipolytica* MNS 1 or WBP1). The amino acid sequence of the transmembrane anchor polypeptide of *Yarrowia lipolytica* MNS1 is designated SEQ ID NO: 26 (the corresponding nucleic acid sequence is set forth in SEQ ID NO: 27), and the amino acid sequence of the transmembrane anchor polypeptide of *Yarrowia lipolytica* WBP1 is designated SEQ ID NO: 28 (the corresponding nucleic acid sequence set is forth in SEQ ID NO: 29). Heterologous sequences can be of varying length and in some cases can be a longer sequences than the full-length target proteins to which the heterologous sequences are attached.

As used herein, the term "wild-type" as applied to a nucleic acid or polypeptide refers to a nucleic acid or a polypeptide that occurs in, or is produced by, respectively, a biological organism as that biological organism exists in nature.

The term "exogenous" as used herein with reference to a nucleic acid (or a protein) and a host cell refers to (a) a nucleic acid that does not occur in (and cannot be obtained from) a cell of that particular type as found in nature or (b) a protein encoded by such a nucleic acid. Thus, a non-naturally-occurring nucleic acid is considered to be exogenous to a host cell once in the host cell. It is important to note that non-naturally-occurring nucleic acids can contain nucleic acid subsequences or fragments of nucleic acid sequences that are found in nature provided that the nucleic acid as a whole does not exist in nature. For example, a nucleic acid molecule containing a genomic DNA sequence within an expression vector is nonnaturally-occurring nucleic acid, and thus is exogenous to a host cell once introduced into the host cell, since that nucleic acid molecule as a whole (genomic DNA plus vector DNA) does not exist in nature. Thus, any vector, autonomously replicating plasmid, or virus (e.g., retrovirus, adenovirus, or herpes virus) that as a whole does not exist in nature is considered to be non-naturally-occurring nucleic acid. It follows that genomic DNA fragments produced by PCR or restriction endonuclease treatment as well as cDNAs are considered to be non-naturally-occurring nucleic acid since they exist as separate molecules not found in nature. It also follows that any nucleic acid containing a promoter sequence and polypeptide-encoding sequence (e.g., cDNA or genomic DNA) in an arrangement not found in nature is non-naturally-occurring nucleic acid. A nucleic acid that is naturally-occurring can be exogenous to a particular host cell. For example, an entire chromosome isolated from a cell of yeast x is an exogenous nucleic acid with respect to a cell of yeast y once that chromosome is introduced into a cell of yeast y.

In contrast, "endogenous" as used herein with reference to a nucleic acid (e.g., a gene) (or a protein) and a host cell refers to any nucleic acid (or protein) that does occur in (and can be obtained from) that particular cell as it is found in nature. Moreover, a cell "endogenously expressing" a nucleic acid (or a protein) expresses that nucleic acid (or protein) as does a host cell of the same particular type as it is found in nature. Moreover, a host "endogenously producing" or that "endogenously produces" a nucleic acid, protein, or other compound produces that nucleic acid, protein, or other compound as does a host cell of the same particular type as it is found in nature.

The term "exogenous" as used herein with respect to a promoter that drives expression of a protein coding sequence means that the promoter does not drive expression of that protein coding sequence as the protein coding sequence occurs in nature. On the other hand, the term "endogenous" as used herein with respect to a promoter that drives expression of a protein coding sequence means that the promoter does drive expression of that protein coding sequence as the protein coding sequence occurs in nature.

The term "exogenous" as used herein with respect to a leader or signal sequence that is covalently bound, directly or indirectly, to a mature protein means that the leader or signal sequence is not covalently bound, directly or indirectly, to that mature protein as the corresponding immature protein occurs in nature. On the other hand, the term "endogenous" as used herein with respect to a leader or signal sequence that is covalently bound, directly or indirectly, to a mature protein means that the leader or signal sequence is covalently bound, directly or indirectly, to that mature protein as the corresponding immature protein occurs in nature. Described herein are uses of nucleic acids encoding type I sulfatases, including iduronate sulfatase and sulfamidase, and functional fragments of them. Also featured are type I sulfatases of different origins and functional fragments of these. The use of additional nucleic acid sequences encoding proteins including FGEs of different origins (*i.e*., human, *Streptomyces coelicolor* (bacterium), *Hemicentrotus pulcherrimus* (sea urchin) *Bos taurus* (bovine), *Mycobacterium tuberculosis* (bacterium), *Tupaia chinensis* (tree shrew), *Monodelphis domestica* (opposum), *Gallus gallus* (red junglefowl), *Dendroctonus ponderosa* (mountain pine beetle) or *Columba livia* (rock dove)), various FGEs (i.e., SCO7548, Rv0712, sulfatase modifying factor 1 and C alpha formylglycine generating enzyme), trafficking proteins (i.e. PDIs, Erp44, and SUMF2), ER targeting polypeptides (e.g., those of *Y. lipolytica* MNS1 or WBP1), post-translational modifying enzymes (i.e., mannosidases and polypeptides that effect mannosyl phosphorylation), and functional fragments of all of these is also included. A nucleic acid encoding a polypeptide of interest (e.g., a type I sulfatase, or a functional fragment thereof), an FGE, a trafficking polypeptide, an ER targeting polypeptide (i.e. *Y. lipolytica* MNS1 and *Y. lipolytica* WBP1), a mannosidase, a polypeptide that effects mannosyl phosphorylation or a functional fragment of any of these, can be or contain, a nucleotide sequence, having at least 70% sequence identity (e.g., at least 80%, 85%, 90%, 95%, 97%, 98%, 99%, or 100% sequence identity) to the nucleotide sequences encoding the corresponding wild-type polypeptides or functional fragments. In some embodiments, nucleic acids described herein are, or can contain, a nucleotide sequence that is at least 70% (e.g., at least 75, 80, 85, 90, 93, 95, 99, or 100 percent) identical to the naturally occurring sequences and corresponding functional fragment-encoding sequences. In addition, the nucleic acids can be, or contain, nucleotide sequences, encoding the polypeptides or functional fragments of them that have at least 70% (e.g., at least 75, 80, 85, 90, 95, 99, or 100 percent) identity to the naturally occurring polypeptide amino acid sequences (e.g., those set forth in SEQ ID NO: 9, 11, 13, 15, 17, 19, 21, 43, 45, 47, 49, 51 and whose nucleic acid sequences are set forth in SEQ ID NO: 10, 12, 14, 16, 18, 20, 22, 44, 46, 48, 50, 52) or functional fragments of the naturally occurring polypeptide amino acid sequences. For example, a nucleic acid can encode a type I sulfatase having at least 90% (e.g., at least 95 or 98%) identity to the amino acid sequence set forth in SEQ ID NO: 19 (whose nucleic acid sequence is set forth in SEQ ID NO: 20) or a portion thereof.

The percent identity between a particular amino acid sequence and the amino acid sequence set forth for a protein can be determined as follows. First, the amino acid sequences are aligned using the BLAST 2 Sequences (B12seq) program from the stand-alone version of BLASTZ containing BLASTP version 2.0.14. This stand-alone version of BLASTZ can be obtained from Fish & Richardson's web site (e.g., www.fr.comJblast/) or the U.S. government's National Center for Biotechnology Information web site (www.ncbi.nlm.nih.gov). Instructions explaining how to use the B12seq program can be found in the readme file accompanying BLASTZ. B12seq performs a comparison between two amino acid sequences using the BLASTP algorithm. To compare two amino acid sequences, the options of B12seq are set as follows: -i is set to a file containing the first amino acid sequence to be compared (e.g., C:\seq 1.txt); -j is set to a file containing the second amino acid sequence to be compared (e.g., C:\seq2.txt); -p is set to blastp; -0 is set to any desired file name (e.g., C:\output.txt); and all other options are left at their default setting.

For example, the following command can be used to generate an output file containing a comparison between two amino acid sequences: C:\B12seq -i c:\seq 1.txt-jc:\seq2.txt-pblastp-0 c:\output.txt. If the two compared sequences share homology, then the designated output file will present those regions of homology as aligned sequences. If the two compared sequences do not share homology, then the designated output file will not present aligned sequences. Similar procedures can be following for nucleic acid sequences except that blastn is used. Once aligned, the number of matches is determined by counting the number of positions where an identical amino acid residue is presented in both sequences. The percent identity is determined by dividing the number of matches by the length of the full-length polypeptide amino acid sequence followed by multiplying the resulting value by 100.

It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer. It will be appreciated that a number of nucleic acids can encode a polypeptide having a particular amino acid sequence. The degeneracy of the genetic code is well known to the art; i.e., for many amino acids, there is more than one nucleotide triplet that serves as the codon for the amino acid. For example, codons in the coding sequence for a given polypeptide can be modified such that optimal expression in a particular species (e.g., bacteria or fungus) is obtained, using appropriate codon bias tables for that species. Hybridization also can be used to assess homology between two nucleic acid sequences. A nucleic acid sequence described herein, or a fragment or variant thereof, can be used as a hybridization probe according to standard hybridization techniques. The hybridization of a probe of interest to DNA or RNA from a test source is an indication of the presence of DNA or RNA corresponding to the probe in the test source. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1 % SDS at 50°C. Highly stringent conditions are defined as equivalent to hybridization in 6X sodium chloride/sodium citrate (SSC) at 45°C, followed by a wash in 0.2 X SSC, 0.1 % SDS at 65°C.

In addition to nucleic acids encoding the above-described wild-type and variant polypeptides and polypeptide fragments, this document also describes all the wild-type and variant polypeptides and polypeptide fragments *per se.*

### Enzymes and Other Polypeptides

### Type I sulfatases

This document describes the use of isolated nucleic acids encoding type I sulfatases that can hydrolyze sulfate esters as well as the type I sulfatases themselves and functional fragments thereof. Substrates of type I sulfatases include small cytosolic steroids, such as estrogen sulfate, complex cell-surface carbohydrates, such as the glycosaminoglycans, and glycolipids. Type I sulfatases function in the degradation of sulfated glycosaminoglycans and glycolipids in the lysosome, and in remodeling sulfated glycosaminoglycans in the extracellular space. Type I sulfatases include, without limitation, cerebroside-sulfatase, steroid sulfatase, arylsulfatase A, arylsulfatase B, arylsulfatase C, arylsulfatase E, iduronate 2-sulfatase, N-acetylgalactosamine-6-sulfatase, N-sulfoglucosamine sulfohydrolase, glucosamine-6-sulfatase, N-sulfoglucosamine sulfohydrolase. Sources of type I sulfatases useful for the invention include those from prokaryotes (e.g., bacteria) and eukaryotes (e.g., fungi (including yeasts), plants, insects, molluscs, and vertebrates such as mammals, fish, birds, and reptiles. A mammal can be, for example, a human or a nonhuman primate (e.g., chimpanzee, baboon, or monkey), a mouse, a rat, a rabbit, a guinea pig, a gerbil, a hamster, a horse, a type of livestock (e.g., cow, pig, sheep, or goat), a dog, a cat, or a whale. Fungi can be any of those listed herein as sources of cells for performing the methods of the document. Exemplary sources include, for example, sea urchins and green algae.

Type I sulfatases, or functional fragments thereof, undergo co- or post-translational modification for their activity in hydrolyzing sulfate esters. An active site cysteine residue is oxidized to the aldehyde-containing C_{α}-formylglycine residue by FGE, or funcational ragments thereof, described below. In mediating its catalytic activity, the formylglycine (FGly) residue positioned within the active site of type I sulfatases is believed to undergo hydration to a *gem-*diol, after which one of the hydroxyl groups acts as a catalytic nucleophile to initiate sulfate ester cleavage. The FGly residue is located within a ∼12-residue consensus sequence termed the type I sulfatase motif that defines this family of enzymes and is highly conserved throughout all domains of nature. Sources of FGE can be those listed above for sulfatases. Iduronate sulfatase has, for example, the 12 amino acid conserved sequence CAPSRVSFLTGR (SEQ ID NO: 34) (the cysteine residue that is converted to FGly is underlined (Dierks et al (1999) The EMBO Journal, 18(8), 2084-2091)).

### Formylglycine-Generating Enzymes

This document describes the use of isolated nucleic acids encoding formylglycine-generating enzymes (FGEs), or functional fragments thereof, that can oxidize a cysteine residue in the active site of type I sulfatase to the aldehyde-containing C_{α}-formylglycine residue as well as the FGEs and fragments *per se.* For example, FGE may be the protein product of the human gene sulfatase modifying factors 1 (SUMF1). The functional fragment of an FGE protein generally contains the active site of the FGE enzyme. The functional fragment has the ability to activate a type I sulfatase, or a functional fragment thereof. Candidate functional fragments of type I sulfatases can therefore be produced by one skilled in the art using well established methods. Their activity can be confirmed by well-established methods such as those described in the working examples disclosed here.

Sources of FGEs can be eukaryotic (e.g., bacterial) or eukaryotic (e.g., fungal (including yeast), vertebrate (e.g., mammalian), invertebrate (e.g., insect or mollusc), or plant. Thus, they can be from humans (*Homo sapiens*), *Streptomyces coelicolor, Mycobacterim tuberculosis*, *Hemicentrotus pulcherrimus, Bos taurus*, *Mus musculus, Danio rerio, Drosophila melanogaster, Tupaia chinensis, Monodelphis domestica, Gallus gallus, Dendroctonus ponderosa,* or *Columba livia* and the like. FGE proteins from different species are listed at this website: http://www.ebi.ac.uk/interpro/entry/IPR005532/taxonomy;jsessionid=A50B4C8B868FB85867E 9D179F3959BED.

### Trafficking and Chaperone proteins

Enzymes catalyzing proper protein folding are coupled to the function of protein trafficking and translocation. Certain such chaperone enzymes also aid in the transport of the proteins to different locations within a cell. By acting as a chaperone, these enzymes aid proteins to reach a correctly folded state. This document describes the use of isolated nucleic acids encoding such trafficking proteins and functional fragments thereof, as well as the proteins and functional fragments themselves. These include, for example, PDI (protein disulfide isomerase) that can (i) catalyze the formation and breakage of disulfide bonds between cysteine residues within proteins as they fold (ii) act as a chaperone protein (aid its correct folding of proteins) (iii) act as an isomerase to catalyze a reduction of mispaired thiol residues of a particular substrate (iv) catalyze the posttranslational modification disulfide exchange, and (iv) load antigenic peptides into MHC class I molecules. Genes that code for members of the PDI family include without limitation, AGR2, AGR3, CASQ1, CASQ2, DNAJC10, ERP27, ERP29, ERP44, P4HB, PDIA2, PDIA3, PDIA4, PDIA5, PDIA6, PDIALT, TMX1, TMX2, TMX3, TMX4, TXNDC5, or TXNDC12 (http://www.ncbi.nlm.nih.gov/pubmed/20796029). Also described herein is the use of isolated nucleic acids encoding the trafficking protein ERp44 and functional fragments thereof, as well as ERp44 *per se* and functional fragments of it. ERp44 forms mixed disulfides with both Ero1-Lα and -Eβ (hEROs) and cargo folding intermediates. ERp44 is believed to have a role in the control of oxidative protein folding in the ER and is required to retain certain proteins in the ER.

### Mannosidases

As described herein,type I sulfatases, or functional fragments thereof, containing N-glycans can be demannosylated, and type I sulfatases containing a phosphorylated N-glycan containing a terminal mannose-1-phospho-6-mannose linkage or moiety can be uncapped and demannosylated by contacting the glycoprotein with a mannosidase capable of (i) hydrolyzing a mannose-1- phospho-6-mannose linkage or moiety to mannose-6-phosphate and (ii) hydrolyzing a terminal alpha-1,2-mannose, alpha-1,3-mannose and/or alpha-1,6-mannose linkage or moiety. Non-limiting examples of such mannosidases include a *Canavalia ensiformis* (Jack bean) mannosidase and a *Yarrowia lipolytica* mannosidase (e.g., AMS1). Both the Jack bean and AMSI mannosidase are family 38 glycoside hydrolases. This document describes nucleic acids encoding such mannosidases and functional fragments of them, as well as the mannosidases *per se* and functional fragments thereof.

In an N-glycan bound to a type I sulfatase, or a functional fragment thereof, containing a terminal mannose-1-phospho-6-mannose moiety, there may be an additional mannose residue bound via an alpha 1,2 linkage to the mannose that is bound via its 6-position to the phosphate of the moiety. The mannose that is bound via its 6-position to the phosphate of the moiety is sometimes referred to herein as the underlying mannose residue. Upon contacting an isolated activated type I sulfatase with the purified mannosidases and/or cell lysate, the mannose-1-phospho-6-mannose linkage or moiety can be hydrolyzed to phospho-6-mannose and the terminal alpha-1 ,2 mannose, alpha-1 ,3 mannose and/or alpha-1 ,6 mannose linkage or moiety of such a phosphate containing glycan can be hydrolyzed to produce an uncapped and demannosylated target molecule. In some embodiments, one mannosidase is used that catalyzes both the uncapping and demannosylating steps. In some embodiments, one mannosidase is used to catalyze the uncapping step and a different mannosidase is used to catalyze the demannosylating step. The methods described in PCT/IB2011/002770 or U.S. Application Publication No. U.S. Application Publication No. US2013/0267473-A1 can be used to determine if the type I sulfatase has been uncapped and demannosylated.

This document also describes nucleic acids encoding proteins, as well as the proteins *per se*, with the activities of all the polypeptides described above, as well as the use of the nucleic and the proteins in the methods described herein. These polypeptides include, without limitation, any of the described type I sulfatases, FGEs, trafficking and chaperone molecules, and mannosidases. It is understood that the proteins having these activities include the full-length wild type mature (and immature as appropriate) polypeptides and functional fragments of the full-length wild type mature polypeptides, as well all of the variants of both as described herein. Examples of variants include, without limitation, those specified in terms of percent (%) identity to a reference amino acid or nucleic acid sequence, degrees of hybridization of coding nucleic acids to target nucleic acids, substitutions (e.g., conservative amino acid substitutions), additions (amino acids or nucleotides), and deletions (amino acids or nucleotides).

### Genetically Engineered Cells and Methods of Using the Same

The genetically engineered cells of the present document can contain one or more nucleic acids encoding one or more of a FGE, a type I sulfatase a trafficking protein (i.e., PDI, Erp44), one or more mannosidases, a polypeptide that effects phosphorylation of a mannose residue and functional fragments of these proteins. The nucleic acids may encode one or more copies of either FGE, type 1 sulphatase, or both. Cells suitable for *in vivo* production of activated type I sulfatases or for recombinant production of any of the polypeptides described herein can be of fungal origin, including yeasts such as *Yarrowia lipolytica,* and *Arxula adeninivorans* or other related species of dimorphic yeasts, *Saccharomyces cerevisiae,* methylotrophic yeasts (such as methylotrophic yeasts of the genus *Candida, Hansenula, Ogataea, Pichia* or *Torulopsis*) or filamentous fungi of the genus *Aspergillus, Trichoderma, Neurospora, Fusarium,* or *Chrysosporium.* Exemplary yeast species include, without limitation, *Pichia anomala, Pichia bovis, Pichia canadensis, Pichia carson ii, Pichia farinose, Pichia fermentans, Pichia fluxuum, Pichia membranaefaciens, Pichia membranaefaciens, Candida valida, Candida albicans, Candida ascalaphidarum, Candida amphixiae, Candida Antarctica, Candida atlantica, Candida atmosphaerica, Candida blattae, Candida carpophila, Candida cerambycidarum, Candida chauliodes, Candida corydalis, Candida dosseyi, Candida dubliniensis, Candida ergatensis, Candidafructus, Candida glabra ta, Candida fermentati, Candida guilliermondii, Candida haemulonii, Candida insectamens, Candida insectorum, Candida intermedia, Candida jeffresii, Candida kefYr, Candida krusei, Candida lusitaniae, Candida lyxosophila, Candida maltosa, Candida membranifaciens, Candida milleri, Candida oleophila, Candida oregonensis, Candida parapsilosis, Candida quercitrusa, Candida shehatea, Candida temnochilae, Candida tenuis, Candida tropicalis, Candida tsuchiyae, Candida sinolaborantium, Candida sojae, Candida viswanathii, Candida utilis, Ogataea minuta, Pichia membranaefaciens, Pichia silvestris, Pichia membranaefaciens, Pichia chodati, Pichia membranaefaciens, Pichia menbranaefaciens, Pichia minuscule, Pichia pastoris, Pichia pseudopolymorpha, Pichia quercuum, Pichia robertsii, Pichia saitoi, Pichia silvestrisi, Pichia strasburgensis, Pichia terricola, Pichia vanriji, Pseudozyma Antarctica, Rhodosporidium toruloides, Rhodotorula glutinis, Saccharomyces bayanus, Saccharomyces bayanus, Saccharomyces momdshuricus, Saccharomyces uvarum, Saccharomyces bayanus, Saccharomyces cerevisiae, Saccharomyces bisporus, Saccharomyces chevalieri, Saccharomycesdelbrueckii, Saccharomyces exiguous, Saccharomyces fermentati, Saccharomyces fragilis, Saccharomyces marxianus, Saccharomyces mellis, Saccharomyces rosei, Saccharomyces rouxii, Saccharomyces uvarum, Saccharomyces willianus, Saccharomycodes ludwigii, Saccharomycopsis capsularis, Saccharomycopsis fibuligera, Saccharomycopsis fibuligera, Endomyces hordei, Endomycopsis fobuligera. Saturnispora saitoi, Schizosaccharomyces octosporus, Schizosaccharomyces pombe, Schwanniomyces occidentalis, Torulaspora delbrueckii, Torulaspora delbrueckii, Saccharomyces dairensis, Torulaspora delbrueckii, Torulaspora fermentati, Saccharomyces fermentati, Torulaspora delbrueckii, Torulaspora rosei, Saccharomyces rosei, Torulaspora delbrueckii, Saccharomyces rosei, Torulaspora delbrueckii, Saccharomyces delbrueckii, Torulaspora delbrueckii, Saccharomyces delbrueckii, Zygosaccharomyces mongolicus, Dorulaspora globosa, Debaryomyces globosus, Torulopsis globosa, Trichosporon cutaneum, Trigonopsis variabilis, Williopsis californica, Williopsis saturnus, Zygosaccharomyces bisporus, Zygosaccharomyces bisporus, Debaryomyces disporua. Saccharomyces bisporas, Zygosaccharomyces bisporus, Saccharomyces bisporus, Zygosaccharomyces mellis, Zygosaccharomyces priorianus, Zygosaccharomyces rouxiim, Zygosaccharomyces rouxii, Zygosaccharomyces barkeri, Saccharomyces rouxii, Zygosaccharomyces rouxii, Zygosaccharomyces major, Saccharomyces rousii, Pichia anomala, Pichia bovis, Pichia Canadensis, Pichia carson ii, Pichiafarinose, Pichiafermentans, Pichiafluxuum, Pichia membranaefaciens, Pichia pseudopolymorpha, Pichia quercuum, Pichia robertsii, Pseudozyma Antarctica, Rhodosporidium toruloides, Rhodosporidium toruloides,Rhodotorula glutinis, Saccharomyces bayanus, Saccharomyces bayanus, Saccharomyces bisporus, Saccharomyces cerevisiae, Saccharomyces chevalieri, Saccharomyces delbrueckii, Saccharomyces fermentati, Saccharomyces fragilis, Saccharomycodes ludwigii, Schizosaccharomyces pombe, Schwanniomyces occidentalis, Torulaspora delbrueckii, Torulaspora globosa, Trigonopsis variabilis, Williopsis californica, Williopsis saturnus, Zygosaccharomyces bisporus, Zygosaccharomyces mellis, or Zygosaccharomyces rouxii.* Exemplary filamentous fungi include various species of *Aspergillus* including, but not limited to, *Aspergillus caesiellus, Aspergillus candidus, Aspergillus carneus, Aspergillus clavatus, Aspergillus deflectus, Aspergillus flavus, Aspergillus fum iga tus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terre us, Aspergillus ustus, Aspergillus versicolor, Trichoderma reesei,* or *Neurospora crassa.* Such cells, prior to the genetic engineering as specified herein, can be obtained from a variety of commercial sources and research resource facilities, such as, for example, the American Type Culture Collection (Rockville, MD).

Genetic engineering of a cell can include, in addition to transformation with one or more nucleic acids (e.g., expression vectors) encoding one or more of an FGE, a type I sulfatase, or multiple copies thereof, a trafficking polypeptide, and one or more mannosidases (and functional fragments of these proteins or fusion proteins thereof), further genetic modifications such as: (i) deletion of an endogenous gene encoding an Outer CHain elongation (OCH) protein 1; (ii) introduction of a recombinant nucleic acid encoding a polypeptide capable of effecting mannosyl phosphorylation (e.g, a MNN4 polypeptide from *Yarrowia lipolytica, S. cerevisiae, Ogataea minuta, Pichia pastoris, or C. albicans,* or PNO1 polypeptide from *P. pastoris*) to increase phosphorylation of mannose residues; (iii) introduction or expression of an RNA molecule that interferes with the functional expression of an OCH1 protein; (iv) introduction of a recombinant nucleic acid encoding a wild-type (e.g., endogenous or exogenous) protein having a N-glycosylation activity (i.e., expressing a protein having an N-glycosylation activity); or (v) altering the promoter or enhancer elements of one or more endogenous genes encoding proteins having N-glycosylation activity to thus alter the expression of their encoded proteins. RNA molecules include, e.g., small-interfering RNA (siRNA), short hairpin RNA (shRNA), anti-sense RNA, or micro RNA (miRNA). Further genetic engineering also includes altering an endogenous gene encoding a protein having an N-glycosylation activity to produce a protein having additions (e.g., a heterologous sequence), deletions, or substitutions (e.g., mutations such as point mutations; conservative or non-conservative mutations). Mutations can be introduced specifically (e.g., by site-directed mutagenesis or homologous recombination) or can be introduced randomly (for example, cells can be chemically mutagenized as described in, e.g., Newman and Ferro-Novick (1987) J Cell Biol. 105(4):1587. It is noted the cells can contain one or more (e.g., two of more, three or more, four or more, five or more, six or more, seven or more, eight of more, nine or more, or ten or more) of these further genetic modifications. See, e.g., US Patent 8,026,083 for further details on genetic engineering strategies for use in fungi such as *Yarrowia lipolytica.*

Genetic modifications described herein can result in one or more of (i) an increase in one or more activities in the genetically modified cell, (ii) a decrease in one or more activities in the genetically modified cell, or (iii) a change in the localization or intracellular distribution of one or more activities in the genetically modified cell. It is understood that an increase in the amount of a particular activity (e.g., promoting mannosyl phosphorylation or activating a type I sulfatase) can be due to overexpressing one or more proteins capable of promoting an activity of interest, an increase in copy number of an endogenous gene (e.g., gene duplication), or an alteration in the promoter or enhancer of an endogenous gene that stimulates an increase in expression of the protein encoded by the gene. A decrease in one or more particular activities can be due to overexpression of a mutant form (e.g., a dominant negative form), introduction or expression of one or more interfering RNA molecules that reduce the expression of one or more proteins having a particular activity, or deletion of one or more endogenous genes that encode a protein having the particular activity.

To disrupt a gene by homologous recombination, a "gene replacement" vector can be constructed in such a way to include a selectable marker gene. The selectable marker gene can be operably linked, at both 5' and 3' end, to portions of the gene of sufficient length to mediate homologous recombination. The selectable marker can be one of any number of genes which either complement host cell auxotrophy or provide antibiotic resistance, including URA3, LEU2 and HIS3 genes. Other suitable selectable markers include the CAT gene, which confers chloramphenicol resistance to yeast cells, or the lacZ gene, which results in blue colonies due to the expression of β-galactosidase. Linearized DNA fragments of the gene replacement vector are then introduced into the cells using methods well known in the art (see below). Integration of the linear fragments into the genome and the disruption of the gene can be determined based on the selection marker and can be verified by, for example, Southern blot analysis. A selectable marker can be removed from the genome of the host cell by, e.g., Cre-loxP systems (see below). Alternatively, a gene replacement vector can be constructed in such a way as to include a portion of the gene to be disrupted, which portion is devoid of any endogenous gene promoter sequence and encodes none or an inactive fragment of the coding sequence of the gene. An "inactive fragment" is a fragment of the gene that encodes a protein having, e.g., less than about 5% (e.g., less than about 4%, less than about 3%, less than about 2%, less than about 1 %, or 0%) of the activity of the protein produced from the full-length coding sequence of the gene. Such a portion of the gene is inserted in a vector in such a way that no known promoter sequence is operably linked to the gene sequence, but that a stop codon and a transcription termination sequence are operably linked to the portion of the gene sequence. This vector can be subsequently linearized in the portion of the gene sequence and transformed into a cell. By way of single homologous recombination, this linearized vector is then integrated in the endogenous counterpart of the gene.

Overexpressing a protein in a cell (e.g., a fungal cell) can be acheived using an expression vector. Expression vectors can be autonomous or integrative. A recombinant nucleic acid (e.g., one encoding a type I sulfatase family member, an FGE, a trafficking polypeptide, a polypeptide that effects mannosyl phosphorylation, a mannosidase, or a functional fragment of any of these) can be in introduced into the cell in the form of an expression vector such as a plasmid, phage, transposon, cosmid or virus particle. The recombinant nucleic acid can be maintained extra chromosomally or it can be integrated into the yeast cell chromosomal DNA. Expression vectors can contain selection marker genes encoding proteins required for cell viability under selected conditions (e.g., URA3, which encodes an enzyme necessary for uracil biosynthesis or TRP 1, which encodes an enzyme required for tryptophan biosynthesis) to permit detection and/or selection of those cells transformed with the desired nucleic acids (see, e.g., U.S.. Pat. No. 4,704,362). Expression vectors can also include an autonomous replication sequence (ARS). For example, U.S. Pat. No. 4,837,148 describes autonomous replication sequences which provide a suitable means for maintaining plasmids in *Pichia pastoris.*

Integrative vectors are disclosed, e.g., in U.S. Pat. No. 4,882,279. Integrative vectors generally include a serially arranged sequence of at least a first insertable DNA fragment, a selectable marker gene, and a second insertable DNA fragment. The first and second insertable DNA fragments are each about 200 (e.g., about 250, about 300, about 350, about 400, about 450, about 500, or about 1000 or more) nucleotides in length and have nucleotide sequences which are homologous to portions of the genomic DNA of the species to be transformed. A nucleotide sequence containing a coding sequence of interest (e.g., a coding sequence encoding an FGE or a functional fragment of an FGE) for expression is inserted in this vector between the first and second insertable DNA fragments whether before or after the marker gene. Integrative vectors can be linearized prior to yeast transformation to facilitate the integration of the nucleotide sequence of interest into the host cell genome. An expression vector can feature a recombinant nucleic acid under the control of a yeast (e.g., *Yarrowia lipolytica, Arxula adeninivorans, P. pastoris*, or other suitable fungal species) promoter, which enables them to be expressed in fungal cells. As used herein, a "promoter" refers to a DNA sequence that enables a gene to be transcribed. The promoter is recognized by RNA polymerase, which then initiates transcription. Thus, a promoter contains a DNA sequence that is either bound directly by, or is involved in the recruitment, of RNA polymerase. In addition to a promoter sequence, a nucleic acid such an expression vector can include "enhancer regions," which are one or more regions of DNA that can be bound with proteins (namely, the trans-acting factors, much like a set of transcription factors) to enhance transcription levels of genes (hence the name) in a gene-cluster. The enhancer, while typically at the 5' end of a coding region, can also be separate from a promoter sequence and can be, e.g., within an intronic region of a gene or 3' to the coding region of the gene.

As used herein, "operably linked" means incorporated into a genetic construct (e.g., vector) so that expression control sequences (e.g., promoters and/or enhancers) effectively control expression of a coding sequence of interest. Expression vectors can be introduced into host cells (e.g., by transformation or transfection) for expression of the encoded polypeptide, which then can be purified.

Suitable yeast promoters include, e.g., ADC1, TPI1, ADH2, hp4d, TEF1, POX, and GallO (see, e.g., Guarente et al. (1982) Proc. Natl. Acad. Sci. USA 79(23):7410) promoters. Additional suitable promoters are described in, e.g., Zhu and Zhang (1999) Bioinformatics 15(7- 8):608-611 and U.S. Patent No. 6,265,185.

A promoter can be constitutive or inducible (conditional). A constitutive promoter is understood to be a promoter whose expression is constant under the standard culturing conditions. Inducible promoters are promoters that are responsive to one or more induction cues. For example, an inducible promoter can be chemically regulated (e.g., a promoter whose transcriptional activity is regulated by the presence or absence of a chemical inducing agent such as an alcohol, tetracycline, a steroid, a metal, or other small molecule) or physically regulated (e.g., a promoter whose transcriptional activity is regulated by the presence or absence of a physical inducer such as light or high or low temperatures). An inducible promoter can also be indirectly regulated by one or more transcription factors that are themselves directly regulated by chemical or physical cues. It is understood that other genetically engineered modifications can also be conditional. For example, a gene can be conditionally deleted using, e.g., a site-specific DNA recombinase such as the Cre-loxP system (see, e.g., Gossen et al. (2002) Ann. Rev. Genetics 36: 153-173 and US. Application Publication No. US2006/0014264). While use of a constitutive promoter system such as TEF and quasi-constitutive hp4d do not require extraneous induction in order to induce enzyme production, inducible promoter systems may also be used and form an embodiment of this invention. Such an inducible promoter would include POX2 promoter.

A recombinant nucleic acid can be introduced into a cell described herein using a variety of methods such as the spheroplast technique or the whole-cell lithium chloride yeast transformation method. Other methods useful for transformation of plasmids or linear nucleic acid vectors into cells are described in, for example, U.S. Patent No. 4,929,555; Hinnen et al. (1978) Proc. Nat. Acad. Sci. USA 75:1929; Ito et al. (1983) J Bacterial. 153:163; U.S. Patent No. 4,879,231; and Sreekrishna et al. (1987) Gene 59: 115. Electroporation and PEG 1 000 whole cell transformation procedures may also be used, as described by Cregg and Russel, Methods in Molecular Biology: Pichia Protocols, Chapter 3, Humana Press, Totowa, N.J., pp. 27-39 (1998).

Transformed fungal cells can be selected for by using appropriate techniques including, but not limited to, culturing auxotrophic cells after transformation in the absence of the biochemical product required (due to the cell's auxotrophy), selection for and detection of a new phenotype, or culturing in the presence of an antibiotic which is toxic to the yeast in the absence of a resistance gene contained in the transformants. Transformants can also be selected and/or verified by integration of the expression cassette into the genome, which can be assessed by, e.g., Southern blot or PCR analysis. Prior to introducing the vectors into a target cell of interest, the vectors can be grown (e.g., amplified) in bacterial cells such as *Escherichia coli* (*E. coli*) as described above. The vector DNA can be isolated from bacterial cells by any of the methods known in the art which result in the purification of vector DNA from the bacterial milieu. The purified vector DNA can be extracted extensively with phenol, chloroform, and ether, to ensure that no E. coli proteins are present in the plasmid DNA preparation, since these proteins can be toxic to mammalian cells.

PCR can be used to amplify specific sequences from DNA as well as RNA, including sequences from total genomic DNA or total cellular RNA. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid. Isolated nucleic acids also can be chemically synthesized, either as a single nucleic acid molecule (e.g., using automated DNA synthesis in the 3' to 5' direction using phosphoramidite technology) or as a series of oligonucleotides. For example, one or more pairs of long oligonucleotides (e.g., > 1 00 nucleotides) can be synthesized that contain the desired sequence, with each pair containing a short segment of complementarity (e.g., about 15 nucleotides) such that a duplex is formed when the oligonucleotide pair is annealed. DNA polymerase is used to extend the oligonucleotides, resulting in a single, double-stranded nucleic acid molecule per oligonucleotide pair, which then can be ligated into a vector. Isolated nucleic acids also can be obtained by mutagenesis of, e.g., a naturally occurring DNA.

Expression systems that can be used for small or large scale production of polypeptides include, without limitation, microorganisms such as bacteria (e.g., *E. coli*) transformed with recombinant bacteriophage DNA, plasmid DNA, or cosmid DNA expression vectors containing the nucleic acid molecules, and fungal (e.g., *S. cerevisiae, Yarrowia lipolytica, Arxula adeninivorans, Pichia pastoris, Hansenula polymorpha,* or *Aspergillus*) transformed with recombinant fungal expression vectors containing the nucleic acid molecules. Useful expression systems also include insect cell systems infected with recombinant virus expression vectors (e.g., baculovirus) containing the nucleic acid molecules, and plant cell systems infected with recombinant virus expression vectors (e.g., tobacco mosaic virus) or transformed with recombinant plasmid expression vectors (e.g., Ti plasmid) containing the nucleic acid molecules. Polypeptides also can be produced using mammalian expression systems, which include cells (e.g., immortalized cell lines such as COS cells, Chinese hamster ovary cells, HeLa cells, human embryonic kidney 293 cells, and 3T3 LI cells) harboring recombinant expression constructs containing promoters derived from the genome of mammalian cells (e.g., the metallothionein promoter) or from mammalian viruses (e.g., the adenovirus late promoter and the cytomegalovirus promoter). Typically, recombinant mannosidase polypeptides are tagged with a heterologous amino acid sequence such FLAG, polyhistidine (e.g., hexahistidine), hemagluttanin (HA), glutathione-S-transferase (GST), or maltose-binding protein (MBP) to aid in purifying the protein. Other methods for purifying proteins include chromatographic techniques such as ion exchange, hydrophobic and reverse phase, size exclusion, affinity, hydrophobic charge-induction chromatography, and the like (see, e.g., Scopes, Protein Purification: Principles and Practice, third edition, Springer-Verlag, New York (1993); Burton and Harding, J Chromatogr. A 814:71-81 (1998)). To isolate proteins specifically from the cell culture media, the protein can be concentrated by precipitation, ultrafiltration, batch adsorption or partition in aqueous phase system. Furthermore, the isolated protein can subsequently be enriched by chromatographic techniques as mentioned as well as partition. In addition, high resolution purification of the protein can be achieved by immune-adsorption. The protein can then be subject to pyrogen removal, sterilization and formulation.

In general, for *in vivo* production of the activated type I sulfatases, or the functional fragments of these proteins, by fungal (e.g., *Y. lipolytica*) recombinant cells, the cells can be cultured in an aqueous nutrient medium comprising sources of assimilatable nitrogen and carbon, typically under submerged aerobic conditions (shaking culture, submerged culture, etc.). The aqueous medium can be maintained at a pH of 4.0 - 8.0 (e.g., 4.5, 5.0, 5.5, 6.0, or 7.5), using protein components in the medium, buffers incorporated into the medium or by external addition of acid or base as required. Suitable sources of carbon in the nutrient medium can include, for example, carbohydrates, lipids and organic acids such as glucose, sucrose, fructose, glycerol, starch, vegetable oils, petrochemical derived oils, succinate, formate and the like. Suitable sources of nitrogen can include, for example, yeast extract, Corn Steep Liquor, meat extract, peptone, vegetable meals, distillers solubles, dried yeast, and the like as well as inorganic nitrogen sources such as ammonium sulphate, ammonium phosphate, nitrate salts, urea, amino acids and the like.

Carbon and nitrogen sources, advantageously used in combination, need not be used in pure form because less pure materials, which contain traces of growth factors and considerable quantities of mineral nutrients, are also suitable for use. Desired mineral salts such as sodium or potassium phosphate, sodium or potassium chloride, magnesium salts, copper salts and the like can be added to the medium. An antifoam agent such as liquid paraffin or vegetable oils may be added in trace quantities as required but is not typically required.

Cultivation of recombinant cells (e.g., *Y. lipolytica* cells) expressing a type I sulfatase polypeptide, or functional fragment thereof, can be performed under conditions that promote optimal biomass and/or enzyme titer yields. Such conditions include, for example, batch, fed-batch or continuous culture. Further, changes to the parameters of the conditions can also promote optimal biomass and/or enzyme titer yields of the active form of type I sulfatase, or functional fragment thereof. Such conditions include, for example, glycerol concentration in the culture media, high pO₂ (see below) and the temperature selected for cultivation. For production of high amounts of biomass, submerged aerobic culture methods can be used, while smaller quantities can be cultured in shake flasks. For production in large tanks, a number of smaller inoculum tanks can be used to build the inoculum to a level high enough to minimize the lag time in the production vessel. The medium for production of the biocatalyst is generally sterilized (e.g., by autoclaving) prior to inoculation with the cells. Aeration and agitation of the culture can be achieved by mechanical means simultaneous addition of sterile air or by addition of air alone in a bubble reactor. A higher pO₂ (dissolved oxygen) can be used during cultivation in, for example, a bioreactor to promote optimal biomass. It can also be used to promote optimal active protein expression in the biomass culture. Implementation of such fermentation parameters, including a higher partial oxygen pressure and stepwise glycerol depletion, can result in an increased FGly residue conversion, indicative of active type I sulfatase. pO₂ can be 5% - 40% (e.g., 10%, 15%, 20%, 25%, 30%, or 35%).

The temperature for cultivation may be from 15°C to 32°C (e.g., 16°C, 17°C, 18°C, 19°C, 20°C, 21°C, 22°C, 23°C, 24°C, 25°C, 26°C, 27°C, 28°C, 29°C, 30°C or 31°C).

Provided herein is the use of an expression system in *Y. lipolytica* and a customized fermentation protocol involving higher partial oxygen pressure and stepwise glycerol depletion to produce activated type I sulfatase, or a functional fragment thereof. The presence of FGly residue conversion of a formylglycine-modified peptide, indicative of an active type I sulfatase or functional fragment thereof, was determined using protocols discussed in this document. The conversion of the FGly residue, as a measure of the activation of type I sulfatase or functional fragment thereof, was in a number of instances calculated to be 100%. It is to be understood that an activation of 100% is detected at a detection limit of 0.5% and therefore includes values from 99.5% to 100%.

Active type I sulfatase polypeptides or functional fragments of them are usually secreted by the cells into the relevant culture medium and are not generally retained within the cells of the recombinant fungal cell (e.g., *Yarrowia* cell) and thus do not need to be extracted from the cells. However, should they be retained in the cells, they can be extracted and, if desired, purified by methods known in the art. Where the produced polypeptides are secreted from the recombinant fungal cells, they can be isolated and, as required, purified to a desired level by methods familiar to those in the art (see above).

Where any of the genetic modifications of the genetically engineered cells are inducible or conditional in the presence of an inducing cue (e.g., a chemical or physical cue), the genetically engineered cells can, optionally, be cultured in the presence of an inducing agent before, during, or subsequent to the introduction of one or more nucleic acids. For example, following introduction of the nucleic acid encoding an FGE and a type I sulfatase, and functional fragments of these proteins, the cells can be exposed to a chemical inducing agent that is capable of promoting the expression of the FGE and/or activated type I sulfatase. In such a case, relevant gene(s) can be engineered with an inducible promoter system. This document provides examples of such an inducible promoter system, in particular, POX2. Such a promoter is induced in the presence of oleic acid that is presented to the cell culture as an oleic acid feed. Where multiple inducing cues induce conditional expression of one or more proteins, the fungal cells can be contacted with multiple inducing agents. As indicated above, the activated type I sulfatase, or functional fragment thereof, is secreted into the culture medium via a mechanism provided by a coding sequence (either native to the exogenous nucleic acid or engineered into the expression vector), which directs secretion of the molecule from the cell.

The presence of an activated type I sulfatase molecule in, for example, cells (e.g., fungal cells), cell lysates or culture medium can be verified by a variety of standard protocols for detecting the presence of the activated type I sulfatase. For example, such protocols can include, but are not limited to, immunoblotting or radioimmunoprecipitation with an antibody specific for the activated type I sulfatase or for a tag (e.g., hexa-histidine) fused to the activated type I sulfatase, binding of a ligand specific for the altered, activated type I sulfatase, and/or testing for a type I sulfatase activity. Levels of activated type I sulfatase molecules can also be quantitated using a variety of protocols including nano-ultra high pressure liquid chromatography together with high resolution tandem mass spectrometry. Described herein is the use of such a protocol to measure the presence of formylglycine modified peptide (FGly residue conversion), which is indicative of an activated type I sulfatase.

The proportion of type I sulfatase molecules in a preparation produced by the methods of the present document in which the cysteine to FGly conversion has occurred is greater than 10% (e.g., greater than: 20%; 30%; 40%; 50%; 60%; 70%; 80%; 85%; 90%; 92%; 95%; 97%; 98%; 99;%; or is even 100%).

In some embodiments, following isolation, the activated type I sulfatase, or functional fragment thereof, can be attached to a heterologous moiety, e.g., using enzymatic or chemical means. A "heterologous moiety" refers to any constituent that is joined (e.g., covalently or non-covalently) to the activated type I sulfatase, or functional fragment thereof, which constituent is different from a constituent originally linked to the type I sulfatase molecule, or functional fragment thereof. Heterologous moieties include, e.g., polymers, carriers, adjuvants, immunotoxins, or detectable (e.g., fluorescent, luminescent, or radioactive) moieties. In some embodiments, an additional N-glycan can be added to the altered target molecule.

Methods for detecting glycosylation of a molecule include DNA sequencer assisted (DSA), fluorophore-assisted carbohydrate electrophoresis (FACE) or surface enhanced laser desorption/ionization time-of-flight mass spectrometry (SELDI-TOF MS). For example, an analysis can utilize DSA-FACE in which, for example, glycoproteins are denatured followed by immobilization on, e.g., a membrane. The glycoproteins can then be reduced with a suitable reducing agent such as dithiothreitol (DTT) or β-mercaptoethanol. The sulfhydryl groups of the proteins can be carboxylated using an acid such as iodoacetic acid. Next, the N-glycans can be released from the protein using an enzyme such as N-glycosidase F. N-glycans, optionally, can be reconstituted and derivatized by reductive amination. The derivatized N-glycans can then be concentrated. Instrumentation suitable for N-glycan analysis includes, e.g., the ABI PRISMS 377 DNA sequencer (Applied Biosystems). Data analysis can be performed using, e.g., GENESCAN® 3.1 software (Applied Biosystems). Isolated mannoproteins can be further treated with one or more enzymes such as calf intestine phosphatase to confirm their N-glycan status. Additional methods of N-glycan analysis include, e.g., mass spectrometry (e.g., MALDI -TOF - MS), high-pressure liquid chromatography (HPLC) on normal phase, reversed phase and ion exchange chromatography (e.g., with pulsed amperometric detection when glycans are not labeled and with UV absorbance or fluorescence if glycans are appropriately labeled). See also Callewaert et al. (2001) Glycobiology 11(4):275-281 and Freire et al. (2006) Bioconjug. Chem. 17(2):559-564.

### Cultures of Engineered Cells

This document also describes a substantially pure culture of any of the genetically engineered cells described herein. As used herein, a "substantially pure culture" of a genetically engineered cell is a culture of that cell in which less than about 40% (i.e., less than about: 35%; 30%; 25%; 20%; 15%; 10%; 5%; 2%; 1%; 0.5%; 0.25%; 0.1%; 0.01%; 0.001 %; 0.0001 %; or even less) of the total number of viable cells in the culture are viable cells other than the genetically engineered cell, e.g., bacterial, fungal (including yeast), mycoplasmal, or protozoan cells. The term "about" in this context means that the relevant percentage can be 15% percent of the specified percentage above or below the specified percentage. Thus, for example, about 20% can be 17% to 23%. Such a culture of genetically engineered cells includes the cells and a growth, storage, or transport medium. Media can be liquid, semi-solid (e.g., gelatinous media), or frozen. The culture includes the cells growing in the liquid or in/on the semi-solid medium or being stored or transported in a storage or transport medium, including a frozen storage or transport medium. The cultures are in a culture vessel or storage vessel or substrate (e.g., a culture dish, flask, or tube or a storage vial or tube).

The genetically engineered cells described herein can be stored, for example, as frozen cell suspensions, e.g., in buffer containing a cryoprotectant such as glycerol or sucrose, as lyophilized cells. Alternatively, they can be stored, for example, as dried cell preparations obtained, e.g., by fluidized bed drying or spray drying, or any other suitable drying method.

Additional descriptions of glycosylation engineering, mannosidases, uncapping of mannose-1-phosphate-6-mannose linkages and demannosylation of phosphorylated N-glycans and additional methods of facilitating mammalian cellular uptake of glycoproteins can be found in multiple references. These references include PCT application PCT/IB2011/002770, US Patent 8,026,083, US Patent application 61/611,485, US Patent application 13/499,061, US Patent application 13/510,527, and PCT application PCT/IB2011/002780.

### Disorders treatable with an activated type I sulfatase and functional fragments thereof

Activated type I sulfatases and functional fragments thereof, optionally with any N-glycans uncapped and demannosylated as described herein, can be used to treat a variety of metabolic disorders. A metabolic disorder is one that affects the production of energy within individual human (or animal) cells. Most metabolic disorders are genetic, though some can be "acquired" as a result of diet, toxins, infections, etc. Genetic metabolic disorders are also known as inborn errors of metabolism. In general, the genetic metabolic disorders are caused by genetic defects that result in missing or improperly constructed enzymes (e.g., type I sulfatases or FGEs, or functional fragments of these proteins,) necessary for some step in the metabolic process of the cell. The largest classes of metabolic disorders are disorders of carbohydrate metabolism, disorders of amino acid metabolism, disorders of organic acid metabolism (organic acidurias), disorders of fatty acid oxidation and mitochondrial metabolism, disorders of porphyrin metabolism, disorders of purine or pyrimidine metabolism, disorders of steroid metabolism disorders of mitochondrial function, disorders of peroxisomal function, and lysosomal storage disorders (LSDs).

Examples of disorders that can be treated through the administration of one or more activated type I sulfatases molecules, or functional fragment thereof, optionally uncapped and demannosylated as described herein, (or pharmaceutical compositions of the same) can include metachromatic leukodystrophy, Hunter disease, Sanfilippo disease A & D, Morquio disease A, Maroteaux-Lamy disease, X-linked ichthyosis, Chondroplasia Punctata 1, and MSD.

Symptoms of disorders treatable with activated type I sulfatase, or a functional fragment thereof, are numerous and diverse and can include one or more of e.g., anemia, fatigue, bruising easily, low blood platelets, liver enlargement, spleen enlargement, skeletal weakening, lung impairment, infections (e.g., chest infections or pneumonias), kidney impairment, progressive brain damage, seizures, extra thick meconium, coughing, wheezing, excess saliva or mucous production, shortness of breath, abdominal pain, occluded bowel or gut, fertility problems, polyps in the nose, clubbing of the finger/toe nails and skin, pain in the hands or feet, angiokeratoma, decreased perspiration, corneal and lenticular opacities, cataracts, mitral valve prolapse and/or regurgitation, cardiomegaly, temperature intolerance, difficulty walking, difficulty swallowing, progressive vision loss, progressive hearing loss, hypotonia, macroglossia, areflexia, lower back pain, sleep apnea, orthopnea, somnolence, lordosis, or scoliosis. It is understood that due to the diverse nature of the defective or absent proteins and the resulting disease phenotypes (e.g., symptomatic presentation of a metabolic disorder), a given disorder will generally present only symptoms characteristic to that particular disorder.

In addition to the administration of one or more of the active type I sulfatases, or functional fragments thereof, described herein, an appropriate disorder can also be treated by proper nutrition and vitamins (e.g., cofactor therapy), physical therapy, and pain medications. Depending on the specific nature of a given disorder, a patient can present these symptoms at any age. In many cases, symptoms can present in childhood or in early adulthood.

As used herein, a subject "at risk of developing a disorder treatable with an activated type I sulfatase, or a functional fragment thereof," is a subject that has a predisposition to develop a disorder, i.e., a genetic predisposition to develop such a disorder as a result of a mutation in one or more genes encoding any of the type I sulfatases and FGEs disclosed herein.

A subject "suspected of having a disorder treatable with an activated type I sulfatase, or a functional fragment thereof," is one having one or more symptoms of such a disorder.

Clearly, neither subjects "at risk of developing a disorder treatable with an activated type I sulfatase, or a functional fragment thereof," nor those "suspected of having a disorder treatable with an activated type I sulfatase, or a functional fragment thereof' are all the subjects within a species of interest.

### Pharmaceutical Compositions and Methods of Treatment

One or more activated type I sulfatases, or functional fragments thereof, made by one or more of the methods disclosed herein can be incorporated into a pharmaceutical composition containing a therapeutically effective amount of the one or more activated type I sulfatases, or functional fragments thereof, and one or more adjuvants, excipients, carriers, and/or diluents and used in therapeutic regimens. Acceptable diluents, carriers and excipients typically do not adversely affect a recipient's homeostasis (e.g., electrolyte balance). Acceptable carriers include biocompatible, inert or bioabsorbable salts, buffering agents, oligo- or polysaccharides, polymers, viscosity improving agents, preservatives and the like. One exemplary carrier is physiologic saline (0.15 M NaCI, pH 7.0 to 7.4). Another exemplary carrier is 50 mM sodium phosphate, 100 mM sodium chloride. Further details on techniques for formulation and administration of pharmaceutical compositions can be found in, e.g., Remington's Pharmaceutical Sciences (Maack Publishing Co., Easton, Pa.). Supplementary active compounds can also be incorporated into the compositions.

Administration of a pharmaceutical composition as disclosed herein can be systemic or local. Pharmaceutical compositions can be formulated such that they are suitable for parenteral and/or non-parenteral administration. Specific administration modalities include subcutaneous, intravenous, intramuscular, intraperitoneal, transdermal, intrathecal, oral, rectal, buccal, topical, nasal, ophthalmic, intra-articular, intra-arterial, sub-arachnoid, bronchial, lymphatic, vaginal, and intra-uterine administration.

Administration can be by periodic injections of a bolus of the pharmaceutical composition or can be uninterrupted or continuous by intravenous or intraperitoneal administration from a reservoir which is external (e.g., an IV bag) or internal (e.g., a bio-erodable implant, a bio-artificial organ, or a colony of implanted altered N-glycosylation molecule production cells). See, e.g., U.S. Pat. Nos. 4,407,957, 5,798,113, and 5,800,828. Administration of a pharmaceutical composition can be achieved using suitable delivery means such as: a pump (see, e.g., Annals of Pharmacotherapy, 27:912 (1993); Cancer, 41: 1270 (1993); Cancer Research, 44: 1698 (1984); microencapsulation (see, e.g., U.S. Pat. Nos. 4,352,883; 4,353,888; and 5,084,350); continuous release polymer implants (see, e.g., Sabel, U.S. Pat. No. 4,883,666); macro encapsulation (see, e.g., U.S. Pat. Nos. 5,284,761, 5,158,881, 4,976,859 and 4,968,733 and published PCT patent applications WO92119195, WO 95/05452); injection, either subcutaneously, intravenously, intra-arterially, intramuscularly, or to other suitable site; or oral administration, in capsule, liquid, tablet, pill, or prolonged release formulation. Examples of parenteral delivery systems include ethylene-vinyl acetate copolymer particles, osmotic pumps, implantable infusion systems, pump delivery, encapsulated cell delivery, liposomal delivery, needle-delivered injection, needle-less injection, nebulizer, aerosolizer, electroporation, and trans dermal patch.

Formulations suitable for parenteral administration conveniently contain a sterile aqueous preparation of the activated type I sulfatase, or the functional fragment thereof, which preferably is isotonic with the blood of the recipient (e.g., physiological saline solution). Formulations can be presented in unit-dose or multi-dose form.

Formulations suitable for oral administration can be presented as discrete units such as capsules, cachets, tablets, or lozenges, each containing a predetermined amount of the activated type I sulfatase; or a suspension in an aqueous liquor or anon-aqueous liquid, such as a syrup, an elixir, an emulsion, or a draught.

An activated type I sulfatase, or functional fragment thereof, made by a method disclosed herein and suitable for topical administration can be administered to a mammal (e.g., a human patient) as, e.g., a cream, a spray, a foam, a gel, an ointment, a salve, or a dry rub. A dry rub can be rehydrated at the site of administration. The activated type I sulfatase molecules, or functional fragments thereof, can also be infused directly into (e.g., soaked into and dried) a bandage, gauze, or patch, which can then be applied topically. The activated type I sulfatase,or functional fragment thereof, can also be maintained in a semi-liquid, gelled, or fully-liquid state in a bandage, gauze, or patch for topical administration (see, e.g., U.S. Patent No. 4,307,717).

Therapeutically effective amounts of a pharmaceutical composition can be administered to a subject in need thereof in a dosage regimen ascertainable by one of skill in the art. For example, a composition can be administered to the subject, e.g., systemically at a dosage of activated type I sulfatase from 0.01 µg/kg to 10,000 µg/kg body weight of the subject, per dose. In another example, the dosage is from 1 µg/kg to 100 µg/kg body weight of the subject, per dose. In another example, the dosage is from 1 µg/kg to 30 µg/kg body weight of the subject, per dose, e.g., from 3 µg/kg to 10 µg/kg body weight of the subject, per dose.

In order to optimize therapeutic efficacy, an activated type I sulfatase, or functional fragment thereof, can be first administered at different dosing regimens. The unit dose and regimen depend on factors that include, e.g., the species of mammal, its immune status, the body weight of the mammal. Typically, levels of a such a molecule in a tissue can be monitored using appropriate screening assays as part of a clinical testing procedure, e.g., to determine the efficacy of a given treatment regimen.

The frequency of dosing for an activated type I sulfatase, or functional fragment thereof, is within the skills and clinical judgment of medical practitioners (e.g., doctors or nurses). Typically, the administration regime is established by clinical trials which may establish optimal administration parameters. However, the practitioner may vary such administration regimes according to the subject's age, health, weight, sex and medical status. The frequency of dosing can be varied depending on whether the treatment is prophylactic or therapeutic.

Toxicity and therapeutic efficacy of activated type I sulfatases (or functional fragments thereof) or pharmaceutical compositions thereof can be determined by known pharmaceutical procedures in, for example, cell cultures or experimental animals. These procedures can be used, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Pharmaceutical compositions that exhibit high therapeutic indices are preferred. While pharmaceutical compositions that exhibit toxic side effects can be used, care should be taken to design a delivery system that targets such compounds to the site of affected tissue in order to minimize potential damage to normal cells (e.g., non-target cells) and, thereby, reduce side effects.

The data obtained from the cell culture assays and animal studies can be used in formulating a range of dosages of an activated type I sulfatase, or functional fragment thereof, for use in appropriate subjects (e.g., human patients). The dosage of activated type I sulfatase, or functional fragment thereof, in such pharmaceutical compositions lies generally within a range of circulating concentrations that include the ED₅₀ with little or no toxicity. The dosage may vary within this range depending upon the dosage form employed and the route of administration utilized. For a pharmaceutical composition used as described herein the therapeutically effective dose can be estimated initially from cell culture assays. A dose can be formulated in animal models to achieve a circulating plasma concentration range that includes the IC₅₀ (i.e., the concentration of the pharmaceutical composition which achieves a half-maximal inhibition of symptoms) as determined in cell culture. Such information can be used to more accurately determine useful doses in humans. Levels in plasma can be measured, for example, by high performance liquid chromatography.

As defined herein, a "therapeutically effective amount" of an activated type I sulfatase, or functional fragment thereof, is an amount of activated type I sulfatase, or functional fragment thereof, that is capable of producing a medically desirable result (e.g., amelioration of one or more symptoms of the relevant disorder) in a treated subject. A therapeutically effective amount (i.e., an effective dosage) can includes milligram or microgram amounts of the compound per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram).

The subject can be any mammal, e.g., a human (e.g., a human patient) or a nonhuman primate (e.g., chimpanzee, baboon, or monkey), a mouse, a rat, a rabbit, a guinea pig, a gerbil, a hamster, a horse, a type of livestock (e.g., cow, pig, sheep, or goat), a dog, a cat, or a whale.

An activated type I sulfatase (or functional fragment thereof) or pharmaceutical composition thereof described herein can be administered to a subject as a combination therapy with another treatment, e.g., a treatment for a metabolic disorder (e.g., a lysosomal storage disorder). For example, the combination therapy can include administering to the subject (e.g., a human patient) one or more additional agents that provide a therapeutic benefit to the subject who has, or is at risk of developing, (or suspected of having) the relevant disorder (e.g., a disorder due to the absence of an active type I sulfatase). Thus, the activated type I sulfatase (or functional fragment thereof) or pharmaceutical composition thereof and the one or more additional agents can be administered at the same time. Alternatively, the activated type I sulfatase, or functional fragment thereof, can be administered first and the one or more additional agents administered second, or *vice versa.*

It will be appreciated that in instances where a previous therapy is particularly toxic (e.g., a treatment with significant side-effect profiles), administration of an activated type I sulfatase, or functional fragment thereof, described herein can be used to offset and/or lessen the amount of the previously therapy to a level sufficient to give the same or improved therapeutic benefit, but without the toxicity.

Any of the pharmaceutical compositions described herein can be included in a container, pack, or dispenser together with instructions for administration.

### EXAMPLES

The methods and materials of the disclosure are further described in the following examples, which do not limit the scope of the invention described in the claims.

### EXAMPLE 1

### Expression of human Iduronate-sulfatase (IDS) in Yarrowia lipolytica

The 525 amino acid human IDS precursor (SEQ ID NO 19; corresponding encoding nucleic acid sequence set forth in SEQ ID NO: 20) was synthesized and codon-optimized for expression in *Y. lipolytica.* The synthetic open reading frame (ORF) of human IDS (hIDS) was fused in frame to the N- terminal region of the *Y. lipolytica* signal sequence Lip2pre of the extracellular lipase gene. This coding sequence was followed by two XXX-Ala cleavage sites and flanked by *BamHI* and *AvrII* restriction sites for cloning into the expression vector in which the coding sequence was under the control of the inducible POX2 promoter.

The recombinant *Y. lipolytica* strain carrying one stably integrated copy of POX2 driven hIDS was generated according to established protocols. The *Y lypolytica* strain used in all the following examples contained the following modifications: Δochl, URA3 ::POX2-MNN4; OCH1 ::Hp4d-MNN4; POX2-Lip2pre-hIDS::zeta. The labeling reference of the engineered strain nomenclature is as follows: deletion/insertion of a gene, locus in which the expression cassette is integrated:: identification of the expression cassette integrated. In order to select high recombinant human IDS (rhIDS) expressing clones, several clones were selected at random and were grown in 24-well plates under oleic acid inducing conditions according to a standard protocol. In each case, the culture supernatant was collected 72 hours post-induction and subsequently screened using SDS-PAGE gel and standard Western blot.

### EXAMPLE 2

### Co-expression of recombinant FGE (rFGE) in a Yarrowia lipolytica stains expressing rhIDS

To achieve high levels of cysteine conversion to FGly in type I sulfatases produced in *Y. lipolytica* strains co-expressing type I sulfatase with FGE proteins were derived. The FGE proteins were from different origins, including prokaryotic origin (*Mycobacterium tuberculosis* FGE (MtFGE) and *Streptomyces coelicolor* FGE (ScFGE)) and eukaryotic origin (Human FGE (hFGE), *Bos taurus* FGE (BtFGE), and *Hemicentrotus pulcherrimus* (HpFGE)). The FGEs and their GenBank accession numbers are shown in Table 1.

**Table 1: FGEs from Different Sources**

| **FGE selected for co-expression** | **Accession** |
|---|---|
| protein SCO7548 [*Streptomyces coelicolor* A3(2)] | NP_631591.1 |
| hypothetical protein Rv0712 [*Mycobacterium tuberculosis* H37Rv] | NP_215226.1 |
| sulfatase modifying factor 1 [*Hemicentrotus pulcherrimus*] | BAJ83907 |
| C-alpha-formyglycine-generating enzyme [*Homo sapiens*] | AA034683 |
| Sulfatase-modifying factor 1 precursor [*Bos taurus*] | NP_001069544 |

Genome mining with the human FGE sequence as a template resulted in the identification Genome mining with the human FGE sequence as a template resulted in the identification of putative FGE orthologs in *M*. *tuberculosis* and *Streptomyces coelicolor* (Carlson et al (2008), The Journal of Biological Chemistry, 283, 20117-20125). Co-expression with *M*. *tuberculosis* FGE was used to modify proteins at specific sites using an *E. coli* expression system; this resulted in a FGly formation with an efficiency of 85% (Rabuka et al (2012), Nature Protocols, 7, 1052-1067). *Hemicentrotus pulcherrimus* FGE (HpSumfl gene product) has been shown to be involved in the activation of type I sulfatases responsible for the regulation of skeletogenesis during sea urchin development (Sakuma et al (2011), Development Genes and Evolution, 221, 157-166). Two cysteine residues, Cys₃₃₆ and Cys₃₄₁ (residue numbering based on sequence of mature hFGE) are localized in the substrate binding groove and are essential for catalytic activity of human Sumf1.

HpSumfl also has a conserved potential N-glycosylation site at the corresponding position to human Sumf1 and a long N-terminal extension. Moreover, *H. pulcherrimus* FGE has been shown to be able to activate mammalian ArsA when overexpressed in HEK293T cells (Sakuma et al (2011), Development Genes and Evolution, 221, 157-166).

To target the different FGE enzymes to the ER, the *Y. lipolytica* LIP2 pre leader sequence (SEQ ID NO: 5; corresponding nucleic acid sequence set forth in SEQ ID NO: 6) was fused to the N-terminus of the mature sequences of the FGEs. The mature sequence of FGE does not contain the hFGE leader sequence (signal peptide) (SEQ ID NO: 23) which effects secretory pathway targeting. To target all the FGEs to the ER, a C-terminal HDEL tetrapeptide (SEQ ID NO: 1; corresponding nucleic acid sequences set forth in SEQ ID NOS: 2) was added as is depicted in FIG.1 to FGEs. Upstream of the HDEL sequence a hexahistidine (6HIS) tag (SEQ ID NO: 7; corresponding nucleic acid sequence set forth in SEQ ID NOS: 8) was included to allow immunological detection. A graphical illustration of the method of construction of the FGE constructs is provided in FIG. 1A.

The amino acid sequences of the rFGE proteins that were co-expressed in a *Y. lipolytica* strain expressing human type I sulfatase are those of SEQ ID NOs: 9, 11, 13, 15, 17 (corresponding nucleic acid sequences set forth in SEQ ID NOS: 10, 12, 14, 16, 18, respectively).

All FGE coding sequences were synthesized and codon-optimized for expression within *Y. lipolytica* and were flanked by *Bam*HI and *Avr*II restriction sites for cloning of the segment into an expression vector under the control of the inducible POX2 or Hp4d promoter. A summary of the co-expression strains is shown in Table 2. Each strain carried one copy of the rhIDS coding sequence co-expressed with two copies of either human (h), *Bos taurus* (Bt), *Streptomyces coelicolor* (Sc), *Hemicentrotus pulcherrimus* (Hp) or *Mycobacterium tuberculosis* (Mt) rFGE coding sequence. In each strain, one rFGE was expressed under the hp4d promoter and the other was expressed under the POX2 promoter.

In order to select high rhIDS expressing clones, several clones were selected at random and were grown in 24-well plates under oleic acid inducing-conditions according to a standard protocol. In each case, the culture supernatant was collected 72 hours post-induction and screened by SDS-PAGE. FIG.2 shows SDS-PAGE detection of human rIDS (SEQ ID NO 22; corresponding amino acid sequence set forth in SEQ ID NO: 21) produced in *Y. lipolytica* at 28°C, 24 deep well plate induction conditions. Samples were treated with Peptide -N-Glycosidase F (PNGaseF) to remove N-glycans. Lanes 1 to 6 are T146 (OXYY1828; BtFGE) clones A to F, respectively (FIG. 2A); lanes 7 to 13 are T147 (OXYY1831; ScFGE) clones A to F, respectively (FIG. 2A AND 2B); lanes 15 to 20 are T148 (OXYYl801; HpFGE) clones A to F, respectively (FIG. 2B); lanes 21 to 24 are T126 (OXYY1827; hFGE) clones A to D, respectively (FIG 2C); lane 25 is empty (FIG 2C); lane 27 contains commercial ELAPRASE® (FIG. 2C); lanes 10, 14, 26 contain protein molecular weight markers (BioRad; Hercules, CA) (FIG 2A-C). The molecular weights of the markers shown in Figures 2B and 2C can be deduced from the labelled ones in FIG.2A in which the same combination of molecular weight markers were used.

**Table 2: Y. lipolytica Strains Co-Expressing Human rIDS and rFGE from Different Sources**

| **rhIDS strains** | **rhFGE coexpression** | **Strain genotype** |
|---|---|---|
| OXYY1827 (T126) | HumanFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-hFGE:Leu2Ex::zeta, POX2-Lip2pre-hFGE:Ade2Ex::zeta |
| OXYY1828 (T146) | BtFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-BtFGE:Leu2Ex::zeta, POX2-Lip2pre-BtFGE:Ade2Ex::zeta |
| OXYY1831 (T147) | ScFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-ScFGE:Leu2Ex::zeta, POX2-Lip2pre-ScFGE:Ad e2 Ex: :zeta |
| OXYY1801 (T148) | HpFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-HpFGE:Leu2Ex::zeta, POX2-Lip2pre-HpFGE:Ade2Ex: :zeta |
| OXYY182 (T153) | MtFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-MtFGE:Leu2Ex::zeta, POX2-Lip2pre-MtFGE:Ade2Ex::zeta |

Recombinant hIDS was expressed in the presence of FGE from different sources in *Y*. *lipolytica* strains. Co-expression with FGE from *Bos Taurus* and *Hemicentrotus pulcherrimus* resulted in expression of IDS. Co-expression with FGE from *Streptomyces coelicolor,* however resulted in suppressed levels of IDS expression relative to that of the other strains.

### EXAMPLE 3

### Detection of Intracellular FGE expression

*Y. lipolytica* cells were harvested 96 hours following the oleic acid induction phase. Yeast cell lysates containing 6HIS-tagged rFGE were prepared according to standard procedures. The expression level of each of the different rFGE proteins was evaluated utilizing Western blot analysis with anti-HIS antibody (Geneart THEtm). The results are shown in FIG.3 and FIG.4. The expected molecular weights of the expressed proteins are as follows: 40.3kDa for *Bos taurus* FGE, 36.7kDa for *Streptomyces coelicolor,* 47,5kDa for *H. pulcherrimus;* 36.1kDa for *M*. *tuberculosis*; and 40,6kDa for *Homo sapiens.*

FIG.3 presents Western blot detection of rFGE utilizing an anti-His6 antibody (Geneart THEtm; 1:5000). Recombinant FGE is indicated by arrows. Lanes 1 to 4 are T146 (OXYY1828; BtFGE) clones A and B grown at 28°C (lanes 1 and 2) and 20°C (lanes 3 and 4), respectively (FIG 3A); lanes 5-8 are T147 (OXYY1831; ScFGE) clones A and B grown at 28°C (lanes 5 and 6) and 20°C (lanes 7 and 8), respectively (FIG 3B); lanes 9 and 19 are T126 (OXYYl827; hFGE) grown at 28°C and 20°C, respectively (FIG 3A AND 3B); lanes 11-14 are T148 (OXYYl801; HpFGE) clones A and B grown at 28°C (lanes 11 and 12) and 20°C (lanes 13 and 14), respectively (FIG 3B); lanes 15-18 are T153 (OXYY1802; MtFGE) clones A and B grown at 28°C (lanes 15 and 16) and 20°C (lanes 17 and 18), respectively (FIG 3B). Lane 21 is a clone of T148 (OXYYl801; HpFGE) grown at 28°C (FIG 3C); lane 22 is a clone of T153 (OXYYl802; MtFGE) grown at 28°C(FIG 3C); lane 23 is a clone of T148 (OXYYl801; HpFGE) grown at 20°C (FIG 3C); lane 24 is a clone of T153 (OXYYl802; MtFGE) grown at 20°C (FIG 3C); lane 25 is a clone of T161 (OXYY1798; BtFGE) grown at 28°C (FIG 3C); Lane 26 is a clone of T156 (OXYY1803; BtFGE and hPDI) grown at 28°C (FIG 3C); Lane 27 is a clone of T146 (OXYY1828; BtFGE) grown at 28°C (FIG 3C). Lanes 10, 20, and 28 contain protein molecular weight markers (BioRad; Hercules, CA) (FIG 3A-C). T126 (OXYYl827) expresses human FGE without a hexahistidine (His6) tag and is the negative control for His6 tagged detection.

Human recombinant FGE detection by Western blot utilizing commercial anti-human SUMF1 polyclonal goat antibody is shown in FIG.4. Lanes 1-4 are T126 (OXYYl827; hFGE) clones A and B grown at 28°C and 20°C (reducing conditions), respectively; and lanes 6-9 are OXYY1827 clones A and B grown at 28°C and 20°C (non-reducing conditions), respectively. Lane 5 contains protein molecular weight markers (BioRad; Hercules, CA).

Different levels of expression were observed for FGE from different sources in *Y. lipolytica* strains. *Bos taurus* FGE presented the strongest expression of the differently-sourced FGEs analyzed. *Hemicentrotus pulcherrimus and Mycobacterium tuberculosis* FGE expressed similar levels of FGE but at levels less than those *of Bos taurus* and *Streptomyces coelicolor* derived FGE. FGE from *Streptomyces coelicolor* expressed at levels less than those *of Bos taurus* but more than those of *Hemicentrotus pulcherrimus* and *Mycobacterium tuberculosis.*

### EXAMPLE 4

### Fermentation of a Yarrowia lipolytica strain expressing rhIDS and co-expressing FGE

For the production of rhIDS in *Yarrowia lipolytica,* a culture was established via the following two-phase method comprising:
1) Growth of the culture on glucose for biomass formation: Strains were grown under standard conditions of pH 6.8, 1vvm air, 28°C, DO=20% with stirring cascade in 500 mL MSI+5g/L glycerol.
2) Feed phase I for biomass generation was started following glycerol depletion (DO-spike); 60% glycerol + MSA linear feed (0.27^{∗}t+1.08)/1.12 for 24 hours.
3) Feed phase II began following feed phase II (4 hours): 60% glycerol + MSA exponential feed 0.4011^{∗}exp(0.007^{∗}t)/1.12 + 20% OA exponential feed 0.8022^{∗}exp (0.007^{∗}t)/0.978 until the end of the fermentation process.

**Table 3: Overview of Fermentation Protocol of Yarrowia lipolytica**

| **Culture medium** | **Feed phase I** | **Feed phase II** |
|---|---|---|
| 500 mL MSI+5g/L glycerol | (0.27xt(h)+1.08)/1.12 | 20% Oleic Acid: 0.72exp(0.007xt(h)/0.978 + 60% Glycerol + MSA: 0.39exp(0.007xt(h)/1.12 |
| | 60 % glycerol + MSA → 24h | |

Table 3 presents the fermentation process for the production the bacteria in the bioreactor. The resulting 50 mL culture was centrifuged for 40 minutes at 7000 rpm. The supernatant was retrieved and stored at -20°C. Ten (10) µl aliquots of the supernatant were analyzed on SDS-PAGE and Western blot as shown in FIG.5.

FIG.5 shows expression analysis of IDS from strains co-expressing rIDS and rFGE grown under fed-batch fermentation by SDS-PAGE (FIG. 5A) and Western blot (FIG. 5B). The supernatant of each culture was analyzed for four FGE strains at four timepoints (11.1 hours, 58.8 hours, 131.6 hours, and 154.9 hours from the start of induction): T146 (OXYY1828; BtFGE co-expression), T147 (OXYY1831; ScFGE co-expression), T148 (OXYYl801; HpFGE co-expression), T153 (OXYYl802; MtFGE co-expression). rhIDS was detected with a rabbit anti-hIDS antiserum. *Y*. *lipolytica* produced rhIDS is visible at an approximate MW of 76 kDa. The four timepoints refer to the samplings in feed phase II. Each timepoint had a 24 hour space in between.

Strains of *Y. lipolytica* co-expressing rhIDS and recombinant FGE (rFGE) were successfully cultivated in the bioreactor. Expression levels of rhIDS however were dependent on the source of the FGE. When co-expressed with FGE derived from *Bos taurus,* rhIDS was expressed at the highest levels observed among the other FGE sources. Co-expression with FGE derived from *Hemicentrotus pulcherrimus and Mycobacterium tuberculosis* demonstrated lower expression levels of IDS. Low IDS expression was noted in cultivations co-expressing *Streptomyces coelicolor* derived FGE.

### EXAMPLE 5

### Analysis of the activity of Y. lipolytica-expressed recombinant human (rhIDS) derived from a strain co-expressing FGE from different origins

To compare and evaluate the level of production and secretion of human IDS among different recombinant *Y. lipolytica* strains, a fluorogenic activity assay using 4-methylumbelliferyl-alpha-L-iduronide-2-sulphate (4MU) and an ELISA quantification were employed. The activity of lysosomal iduronate 2-sulfatase was assayed using fluorogenic 4MU glycoside derivatives as a substrate, as described previously (Voznyi et al (2001), Journal of Inherited Metabolic Disease, 24, 675-680). The results are summarized in Table 4 and FIG.6. Production of human IDS under oleic acid induction conditions at 28°C and 20°C was evaluated in 24 deep-well cultivation.

As shown in Table 4, several clones for each rFGE were tested. Percentage functional rhIDS was calculated as a ratio between the active rhIDS as determined in fluorogenic assay versus the total secreted human IDS as determined in sandwich ELISA. In both tests, the standard curves were generated using commercial elaprase. ELISA was performed on non-buffer exchanged samples, whereas activity was measured on buffer-exchanged samples.

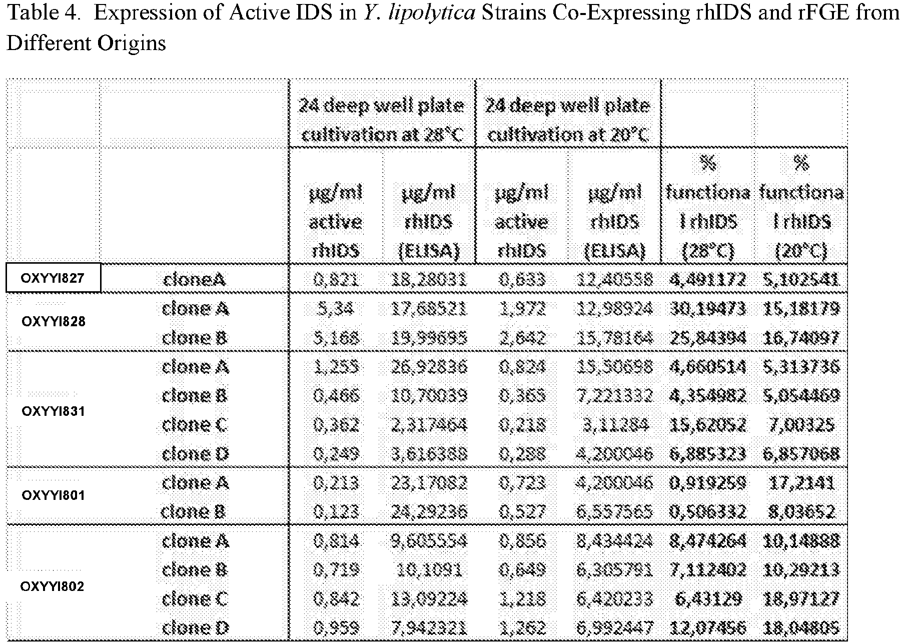

All *Y. lipolytica* strains co-expressing rFGE with rhIDS resulted in the expression of active rhIDS. Strains co-expressing *Bos taurus* (OXYY1828) demonstrated the strongest activity of rhIDS. This was particularly noted in stains cultivated at 28°C. In strains co-expressing *Hemicentrotuspulcherrimus* derived FGE (OXYY1801) a drastic increase in IDS -activity was seen when strains were grown at 20°C instead of 28°C.

### EXAMPLE 6

### Coexpression of hPDI in a strain expressing rhIDS and FGE

The present inventors considered that PDI co-expression in yeast could yield higher levels of active, secreted type I sulfatases in *Y. lipolytica.*

The LIP2 pre leader sequence was fused to the mature hPDI sequence (accession number NP 000909). A HDEL tetrapeptide was fused at the C-terminus to allow targeting to the ER. The complete protein sequence of the engineered protein is given below (SEQ ID NO 21; corresponding nucleic acid sequence set forth in SEQ ID NO: 22).

The PDI gene was synthesized and codon-optimized for *Y. lipolytica* expression and flanked by BamHI and AvrII for cloning into the expression vector under the control of the inducible POX2 promoter. The PDI-expressing plasmid was transformed into the rhIDS-FGE coexpressing strains using random integration and a dominant hygromycin marker.
The strain construction overview of rhIDS expressing *Y. lipolytica* strains, co-expressing hPDI and FGE from different origin is shown in Table 5.

**Table 5: Y. lipolytica Strains Co-Expressing Human rIDS, rPDI and rFGE from Different Sources**

| **rhIDS strains** | **rhFGE coexpression** | **Strain genotype** |
|---|---|---|
| OXYY1827 | humanFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d-Lip2pre-hFGE:Leu2Ex::zeta, POX2- Lip2pre-hFGE:Ade2Ex::zeta, POX2- Lip2pre-hPDI:HygEx::zeta |
| (T126) | | |
| OXYY1803 | BtFGE | MATA, leu2-958, ura3-302, xpe2-322, ade2-844, ΔSc suc2, Δ0ch1, URA3 ::POX2-MNN4, OCH1 ::Hp4d-MNN4, POX2-Lip2pre-hIDS:URA3Ex::zeta, Hp4d -Lip2pre-BtFGE:Leu2Ex::zeta, POX2-Lip2pre-BtFGE:Ade2Ex::zeta, POX2- Lip2pre-hPDI:HygEx::zeta |
| (T156) | | |
| OXYY1844 | ScFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d -Lip2pre-ScFGE:Leu2Ex::zeta, POX2-Lip2pre-ScFGE:Ade2Ex::zeta, POX2- Lip2pre-hPDI:HygEx::zeta |
| (T157) | | |
| OXYY1846 | HpFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d -Lip2pre-HpFGE:Leu2Ex::zeta, POX2-Lip2pre-HpFGE:Ade2Ex::zeta, POX2- Lip2pre-hPDI:HygEx::zeta |
| (T158) | | |
| OXYY1848 | MtFGE | MATA, leu2-958, ura3-302, xpe2-322, ade2-844, ΔSc suc2, Δoch1, URA3 ::POX2-MNN4, OCH1 ::Hp4d-MNN4, POX2-Lip2pre-hIDS:URA3Ex::zeta, Hp4d -Lip2pre-MtFGE:Leu2Ex::zeta, POX2-Lip2pre-MtFGE:Ade2Ex::zeta, POX2- Lip2pre-hPDI:HygEx::zeta |
| (T159) | | |

Each rhIDS strain had one rhIDS coding sequence copy co-expressed with 2 copies of either human, *Bos taurus* (Bt), *Streptomyces coelicolor* (Sc), *Hemicentrotus pulcherrimus* (Hp) *or Mycobacterium tuberculosis* (Mt) rFGE coding sequences. One rFGE was expressed under hp4d promoter while the other was expressed under POX2 promoter. Additionally, one POX2 driven hPDI coding sequence was expressed in each strain.

### EXAMPLE 7

### Determination of FGly conversion using nano-LC MS

Recombinant human IDS (rhIDS) produced in *Y. lipolytica* was treated with PNGaseF to remove N-glycans and separated on a SDS-PAGE gel. Proteins in excised gel slices were digested overnight with trypsin and followed by reduction with dithiothreitol and alkylation with iodoacetamide. The latter adds a carbamidomethyl group to the free cysteine residues and prevents the reformation of disulfide bridges. Trypsin cleaves the protein C-terminally of arginine and lysine residues. The resulting peptides were subsequently extracted from the gel and subject to nano-ultra-high pressure liquid chromatography (nano-UHPLC) connected to high-resolution tandem mass spectrometry (hybrid quadrupole time-of-flight - Q-TOF). A ThermoScientific/Dionex UHPLC system and an Agilent Technologies 6540 Q-TOF mass spectrometer were used. Separation was performed on a nano-column with an internal diameter of 75 µm and a length of 15 cm packed with sub 2 µm C18 particles. Injected peptides were eluted from the column at a flow rate of 300 nl/min using a 0.1% formic acid/acetonitrile gradient. Separated peptides were converted to gas-phase ions using a coated nanospray needle with an 8 µm tip maintained at 2000 V. Quadrupole time-of-flight measurement subsequently allowed the derivation of the m/z values of the intact peptides and the fragments thereof at high mass accuracy (<10 ppm). The formylglycine modified peptide derived from the peptide with the amino acid sequence SPNIDQLASHSLLFQNAFAQQAVCAPSR (cysteine residue that is subject to formylglycine conversion is underlined) could be quantified relative to the non-modified alkylated peptide by extracting, respectively, the triply charged ions at 999,1728 and 1024,1775 at an extraction window of 20 ppm and by determining the peak area following peak smoothing and integration. Identity was confirmed by obtaining the m/z values of the fragments generated by collision induced dissociation.

The results are shown in Table 6. Production of rhIDS under oleic acid inducting condition for 72h was performed at 28°C (except *Hemicentrotus pulcherrimus* -derived clone OXYYl801) in 24 deep-well cultivation unless stated differently in Table 6. Some strains were grown in duplicate (§). Strains co-expressing *Hemicentrotus pulcherrimus* -derived FGE (OXYY1801) demonstrated a drastic increase in IDS-activity when grown at 20°C as compared to 28 °C. Unless otherwise indicated, all strains were grown at 28 °C.

**Table 6: Conversion of the Formylglycine Residue in IDS Expressed in Y. lipolytica Strains**

| **rhIDS production strain (all samples are fermentation samples)** | **%FGly conversion** |
|---|---|
| T146 (OXYY1828; BtFGE) | 89.85 |
| T148 (OXYY1801; HpFGE) | 8.1 |
| T148 (OXYY1801; HpFGE) § | 3.72 |
| T148 (OXYY1801; HpFGE) (20°C) | 68.14 |
| T146 (OXYY1828; BtFGE) § | 92.69 |

FGE derived from different organisms was concluded to be active when recombinantly expressed in *Y. lipolytica* strains. The derived FGEs analyzed were shown to convert the cysteine residues in the active site of the IDS protein to formylglycine. Recombinant FGE sourced from *Bos taurus* (T146; OXYY1828) was observed to be the most active of the FGEs from the other organisms that were analyzed. It was further concluded that the conversion rate to formylglycine was higher when the strains were cultivated in a fermenter. This is likely attributed to the higher partial oxygen pressure in a bioreactor as compared to alternative growth conditions which utilize a shake flask or a 24-well cultivation plate.

It was recently shown that formylglycine is easily hydrated with the formation of a geminal diol (Rabuka et al. (2012), Nat Protoc 7(6), 1052-1067) and that the aldehyde group in formylglycine can interact with the N-terminus of the peptide with the formation of a Schiff base resulting in a water loss (Grove et al. (2008) Biochemistry, 47(28), 7523-7538). Therefore, the the data shown in Table 6 were re-computed taking this geminal diol formation and water loss into account. The same bioreactor samples from OXYY1828 and OXYY1801 strains were re-analyzed.

**Table 7: Re-analysis of samples shown in Table 6**

| **Bioreactor sample** | **rhIDS production strain*** | **% FGly conversion (from Table 6)** | **% FGly conversion - re-analyzed samples** |
|---|---|---|---|
| DG29U5#7 | OXYY1828; BtFGE | 89.85 | 95.8 |
| DG29U7#7 | OXYY1801; HpFGE | 8.1 | 19.2 |
| DG33U1#6 | OXYY1801; HpFGE | 3.72 | 8.3 |
| DG33U3#6 | OXYY1801; HpFGE (20°C) | 68.14 | 84.5 |
| DG33U8#6 | OXYY1828; BtFGE | 92.69 | 97.1 |
| DG33U6#6 | OXYY1803; BtFGE and hPDI | 79.48 | 90.3 |

Re-evaluation of the data confirmed that some of the formylglycine is indeed hydrated to the geminal diol. No Schiff base formation could be detected. The high rhIDS FGly conversion levels obtained by FGE that was sourced from *Bos taurus* (OXYY1828) was confirmed, as well as the low (<20%) FGly conversion levels obtained by FGE sourced from *Hemicentrotus pulcherrimus* (OXYY1801) when the *Y. lipolytica* strain was grown at 28°C. At 20°C HpFGE enabled high FGly conversion.

The accuracy of the above-described nano-LC-MS method was further improved by incorporating a cation exchange chromatography purification step of the rhIDS. The results that were previously obtained (with rhIDS derived from gel slices) on *Y. lipolytica* coexpression of rhIDs and BtFGE were confirmed using this improved method. Such an experiment showed complete conversion (100%, with a detection limit of ∼ 0.5%) of Cys->FGly in rhIDS when BtFGE was coexpressed as a single POX2 driven copy. It was also confirmed that carboxymethylation of free cysteine residues occurred and that the 100% formylglycine incorporation detection was not sample preparation related.

### EXAMPLE 8

### Determination of FGly conversion in Sulfamidase produced in Y. lipolytica using nano-LC MS

A *Y*. *lipolytica* strain was constructed that expressed recombinant human sulfamidase (rSGSH) (SEQ ID NO: 24; corresponding nucleic acid sequence set forth in SEQ ID NO: 25) and co-expressed BtFGE (1 copy, POX2 driven) and hPDI (1 copy, POX2 driven). A strain expressing rSGSH alone and a strain expressing rSGSH in combination with BtFGE without hPDI were also constructed.

These strains were grown in 24-well plates as described in Example 5. The supernatant was analyzed on SDS-PAGE and a gel slice containing SGSH was isolated for MS analysis. The results of the analysis are shown in Table 8.

**Table 8. Conversion of the Formylglycine Residue in rSGSH Expressed in Y. lipolytica strains**

| **Sample No.** | **SGSH production strain (all samples are fermentation samples)** | **%FGly conversion** |
|---|---|---|
| 1 | SGSH (POX) + BtFGE (POX) + hPDI (POX) | 95.4 |
| 2 | SGSH (POX) + BtFGE (POX) + hPDI (POX) | 80.4 |
| 3 | SGSH (POX) + BtFGE (POX) + hPDI (POX) § | 93.4 |
| 4 | SGSH (POX) + BtFGE (POX) + hPDI (POX) § | 86.1 |
| 5 | SGSH (POX) + BtFGE (POX & Hp4d) | 94.4 |
| 6 | SGSH (POX) alone | 4.9 |

The first four samples shown in Table 8 are derived from the same strain run four times independantly in the bioreactor. The fifth sample was derived from a strain having two copies of BtFGE, one under the control of the POX2 inducible promoter and the other under the control of the Hp4d semi-constitutive promoter. Some strains were grown in duplicate (§). All strains were grown at 28 °C. In the absence of an activating factor, 4.9% conversion to FGly was observed. This suggests the presence of a *Y. lipolytica* specific activation mechanism. It was concluded that FGEs from the different sources tested could convert cysteine to formylglycine in SGSH and thereby activated the enzyme. It was further concluded that the conversion rate to formylglycine was higher when the strains were cultivated in a fermentor.

### EXAMPLE 9

### Use of Hemicentrotus vulcherrimus FGE (HpFGE) for rhIDS activation in Y. lipolytica

The Cys->FGly conversion levels of a rhIDS expessing strain (OXYY1801) co-expressing HpFGE (*Hemicentrotus pulcherrimus* -derived FGE) at different growth temperatures was assessed. Strains co-expressing HpFGE (OXYY1801) demonstrated a drastic increase in IDS-activity when grown at 20 °C as compared to 28 °C. Additionally, use of the *Yarrowia* MNS1 anchorage domain as a fusion with HpFGE in an attempt to improve ER retention of HpFGE was assessed. For the latter, fusion of the HpFGE to the transmembrane anchor of *Yarrowia* MNS1 (Accession: XP_502939.1) was performed to obtain correct localisation of HpFGE into the endoplasmic reticulum. Specifically, amino acids 1-163 of YlMNS1 were fused N-terminally to the mature form of HpFGE. At the C-terminal end a 6HIS tag was added (SEQ ID NO: 35, corresponding coding sequence set out as SEQ ID NO: 36). The strain tested was designated FGE6.1.

The MNS1HpFGE coding sequence was synthesized and codon-optimized for expression within *Y. lipolytica* and was flanked by *Bam*HI and *Avr*II restriction sites for cloning of the segment into an expression vector under the control of the inducible POX2 promoter or Hp4d promoter. The relevant constructs are designated OXYP3438 and OXYP3439, respectively. The plasmids were transformed into a *Y. lipolytica* strain expressing rhIDs (T116.22).

**Table 9. Conversion of the Formylglycine Residue in strains of Y. lipolytica co-expressing rIDS and rFGE cultivated in the bioreactor**

| **Strain ID** | **Strain description** | ***%FGly** |
|---|---|---|
| OXYY1801 (20°C) | 1c rhIDS, 2c HpFGE (POX/PHp4d) | 100 |
| OXYY1801 (22°C) | 1c rhIDS, 2c HpFGE (POX/PHp4d)-> | ND (FAILED) |
| OXYY1801 (24°C) | 1c rhIDS, 2c HpFGE (POX/PHp4d) | 100 |
| OXYY1801 (26°C) | 1c rhIDS, 2c HpFGE (POX/PHp4d) | 70.44 |
| FGE6.1 | 1c rhIDS, 2c MNS1-HpFGE | 0 |

| | | |
|---|---|---|
| *FGly conversion of cation exchange chromatography purified samples LC-MS | | |

The data obtained with these strains are shown in Table 9. As was previously observed, full conversion was detected when a *Y. lipolytica* strain co-expressing HpFGE was grown at 20°C. Also at 24°C, conversion was complete. When grown at higher temperature (26°C) the conversion decreased to 70%. At 28°C the conversion was less than 20%. It therefore seems likely the catalytic temperature optimum of HpFGE differs from that of the other tested FGEs.

For the MNS1-HpFGE strain (FGE6.1) conversion of Cys->FGly as determined by LC-MS was shown to be 0% at 28°C. This could be due to low expression or strongly reduced catalytic activity at 28°C as was observed for the HDEL fusion protein.

### EXAMPLE 10

### Localization of mature rFGE to the endoplasmic reticulum (ER) by fusion with the anchorage domain of Yarrowia lipolytica MNS1 mannosidase

Fusions of rFGEs to the transmembrane anchorage domain of *Yarrowia lipolytica* MNS1 (Accession: XP_502939.1) were used to obtain localization of the rFGEs into the endoplasmic reticulum and reduce FGE secretion as was observed for HDEL tagged BtFGE. In order to do this, an expression vector containing a coding nucleotide sequence encoding a fusion polypeptide consisting of, N-terminus to C-terminus, amino acids 1-163 of MNS1 (SEQ ID NO: 26), a mature FGE (e.g., BtFGE), and a hexahistidine (6HIS) (FIG. 7A) was generated (SEQ ID NO: 37, corresponding coding sequence set out as SEQ ID NO: 38). It is expected that when this fusion polypeptide is expressed in *Yarrowia lipolytica* cells, it is localized to the ER of the cells.

The MNS1-BtFGE coding sequence, which was synthesized and codon-optimized for expression within *Y. lipolytica*, are flanked by *Bam*HI and AvrII restriction sites for cloning of the segment into an expression vector under the control of the inducible POX2 promoter or Hp4d promoter. The relevant constructs were designated OXYP3418 and OXYP3424, respectively.

In addition, an expression vector containing a coding nucleotide sequence encoding a fusion polypeptide consisting of, N-terminus to C-terminus, amino acids 1-163 of MNS1 (SEQ ID NO: 26), a novel mature ClFGE from *Columba livia* (Rock dove), and a c-myc tag, was generated (SEQ ID NO: 67, corresponding coding sequence set out as SEQ ID NO: 68). It is expected that when this fusion polypeptide is expressed in *Yarrowia lipolytica* cells, it is localized to the ER of the cells.

The MNS1-ClFGE coding sequence, which was synthesized and codon-optimized for expression within *Y. lipolytica,* are flanked by *Bam*HI and *Avr*II restriction sites for cloning of the segment into an expression vector under the control of the inducible POX2 promoter or Hp4d promoter.

### EXAMPLE 11

### Localization of mature rFGE to the endoplasmic reticulum (ER) by fusion with the anchorage domain of Yarrowia lipolytica WBP1

Fusions of rFGEs to the transmembrane anchorage domain of *Yarrowia lipolytica* WBP1 (Accession: XP 502492.1) (Accession: XP 502939.1) to obtain localization of the rFGEs into the endoplasmic reticulum were generated. In order to do this, an expression vector containing a coding nucleotide sequence encoding a fusion polypeptide consisting of, N-terminus to C-terminus, the Lip2 signal sequence, a hexahistidine (6HIS) tag, a mature FGE (e.g., BtFGE), and the C-terminal 118 amino acids (amino acids 400-505 of XP_502492.1) of *Yarrowia lipolytica* WBP1 (SEQ ID NO: 28) (FIG. 7B) was generated. It is expected that when this fusion polypeptide is expressed in *Yarrowia lipolytica* cells, it is localized to the ER of the cells.

The WBP1-BtFGE coding sequence, which was synthesized and codon-optimized for expression within *Y. lipolytica,* are flanked by *Bam*HI and *Avr*II restriction sites for cloning of the segment into an expression vector under the control of the inducible POX2 promoter or Hp4d promoter. Relevant constructs are designated OXYP3422 and OXYP3428, respectively.

### EXAMPLE 12

### Production of a construct encoding chimeric protein consisting of the N-terminal end of BtFGE fused to the C-terminal end of HpFGE

A construct encoding a chimeric protein consisting of of the N-terminal end of BtFGE (amino acids 32-104 of NP_001069544, fused to the C-terminal end of HpFGE (amino acids 144-423 of BAJ83907) was generated. The Lip2 leader was fused to the N-terminal end of the chimeric coding sequence. At the C-terminus a 6HIS tag was added, followed by the HDEL tetrapeptide. A schematic representation of the protein is given in FIG 7C.

The entire coding sequence, which was synthesized and codon-optimized for expression within *Y. lipolytica,* is flanked by *Bam*HI and AvrII restriction sites for cloning of the segment into an expression vector under the control of the inducible POX2 promoter or Hp4d promoter. Relevant constructs are designated OXYP3420 and OXYP3426, respectively.

### EXAMPLE 13

### Bioreactor fermentation expression analysis of fusion proteins of mature rFGE designed to localize to the endoplasmic reticulum (ER)

The strains of *Y*. *lipolytica* co-expressing rIDS and rFGE successfully cultivated in a bioreactor (Dasgip 37) are described in Table 10 below.

**Table 10. Strains of Y. lipolytica co-expressing rIDS and rFGE successfully cultivated in a bioreactor**

| **Unit** | **Strain ID** | **strain description** |
|---|---|---|
| | | |
| 1 | OXYY1818* | 1 copy rhIDS, 2 copies ChFGE (POX/Hp4d) - 20°C |
| 2 | OXYY1818 | 1 copy rhIDS, 2 copies ChFGE (POX/Hp4d) -28°C |
| 3 | Y3035+* | 2 copy SGSH-5, 2 copies HpFGE (POX/Hp4d) - 20°C |
| 4 | Y3035+ | 2 copy SGSH-5, 2 copies HpFGE (POX/Hp4d) - 28°C |
| 5 | OXYY1822 | 1 copy rhIDS, 2 copies BtFGE-WBPI (POX/Hp4d) |
| 6 | OXYY1826 | 1 copy rhIDS, 2 copies BtFGE-MNS1 (POX/Hp4d) |
| 7 | OXYY1798+hPDI | 1 copy rhIDS, 1 copy BtFGE (POX), 1 copy hPDI (POX) |
| 8 | OXYY1798 | 1 copy rhIDS, 1 copy BtFGE (POX) |

FIG. 8A shows the expression analysis (by Western blot with a rabbit anti-human IDS antiserum) of rhIDS from strains co-expressing rhIDS (1 copy, POX2 driven) and rFGE (1 copy POX2 driven and 1 copy Hp4d driven) grown under fed-batch fermentation. The *Y. lipolytica-*produced IDS is visible at an approximate MW of 76 kDa. The supernatant was analyzed for six rIDS expressing strains at the endpoint of the fermentation. Lane 1 is the MW Marker; lane 2 is ChFGE (the chimeric protein described in Example 12) co-expressed at 20°C; lane 3 is ChFGE co-expressed at 28°C; lane 6 is BtFGE-WBP1 co-expression; lane 7 is BtFGE-MNS1 co-expression; and lanes 8-9 are the control strains co-expressing BtFGE-HDEL (1 copy, POX2 driven). Varying levels of rhIDS were detected, with the highest levels obtained for the MNS1-BtFGE coexpression strain (lane 7). Degradation is present mostly in the WBPI-BtFGE and MNS1-BtFGE coexpression strains (lane 6 and lane 7 respectively).

FIG. 8B shows expression analysis of rFGE by Western blot using anti-his antibody (A00186-100, Genscript). The contents in each lane correspond to those in FIG. 8A. Small amounts of BtFGE were shown to leak into the media for Units 7 and 8 (1 copy POX-driven expression of BtFGE) (lanes 8 and 9). However, in the case of the chimeric protein-expression constructs (lanes 2 and 3) no FGE leaked into the medium. For the WBP1 and MNS1-fusions only very low amounts of FGE leaked into the medium (lanes 6 and 7 respectively).

To compare and evaluate the level of production and secretion of human IDS among different recombinant *Y. lipolytica* strains, a fluorogenic activity assay using 4-methylumbelliferyl-alpha-L-iduronide-2-sulphate (4MU) and an ELISA quantification were employed. The activity of lysosomal iduronate 2-sulfatase was assayed using fluorogenic 4MU glycoside derivatives as a substrate, as described previously (Voznyi et al.(2001) J Inherit Metab Dis, 24(6), 675-680). Percentage functional rhIDS was calculated as a ratio between the active rhIDS as determined in fluorogenic assay versus the total secreted human IDS as determined in sandwich ELISA. In both tests, the standard curves were generated using commercial ELAPRASE®. Results are shown in Table 11.

**Table 11. Conversion of the Formylglycine Residue in strains of Y. lipolytica endoplasmic reticulum (ER) fusion constructs cultivated in the bioreactor**

| **Sample** | **rhIDS concentration (ng/ml)** | **% active** | **%FGly (LC-MS)** |
|---|---|---|---|
| 1 copy rhIDS, 2 copy ChFGE (POX/Hp4d) - 20°C* | 6065 | 0 | ND |
| 1 copy rhIDS, 2 copy ChFGE (POX/Hp4d) -28°C | 9268 | 0 | ND |
| 1 copy rhIDS, 2 copy BtFGE-WBPI (POX/Hp4d) | 13078 | 124 | 89.15 |
| 1 copy rhIDS, 2 copy BtFGE-MNS1 (POX/Hp4d) | 27542 | 98 | 99.5 |
| 1 copy rhIDS, 1 copy BtFGE (POX), 1 copy hPDI (POX) | 14620 | 121 | 100 |
| 1 copy rhIDS, 1 copy BtFGE (POX) | 12534 | 129 | 100 |

In conclusion, a high level of activity and almost full Cys->FGly conversion was obtained when mature BtFGE protein was fused to MNS1 or WBP1 anchorage domains. Reduced leakage of the rFGE into the supernatant was observed when BtFGE was fused to MNS1 or WBP1 anchorage domains. Co-expression of BtFGE-MNS1 appeared to result in an increased rhIDS secetory level. Co-expression of WBP1- and MNS1-BtFGE resulted in increased proteolysis.

In a follow-up analysis carried out under the same conditions described above, two strains containing (i) two copies of rhIDS and one copy of BtFGE (POX2 driven) and (ii) one copy of rhIDS and 2 copies of BtFGE-MNS1 (one driven by POX2 and the other by Hp4d), gave 101% activity (with 100% FGly conversion at a detection limit of ~0.5%) and 81.5% activity (with 100% FGly conversion), respectively.

### EXAMPLE 14

### FGEs from additional species for co-expression in Y. lipolytica

A number of additional human FGE homologues were identified and and tested for their ability to activate rhIDs in *Yarrowia lipolytica* cells. A summary of the FGEs and their accession numbers is shown in Table 12.

**Table 12. Overview of additional FGEs**

| **FGE origin** | **Accession No.** |
|---|---|
| Gray short-tailed opossum (*Monodelphis domestica*) | GI:126336367 |
| Rock Dove (*Columba livia*) | GI:543740918 |
| Chinese tree shrew (*Tupaia chinensis*) | GI:444707484 |
| Red junglefow (*Gallus gallus*) | GI:363738801 |
| Mountain pine beetle (*Dendroctonus ponderosa*) | GI:478257082 |

Mature sequences of the FGE's were fused at the N-terminus to the Lip2pre as a leader sequence (MKLSTILFTACATLAAA) (SEQ ID NO: 5). To the C-terminal end a 6His (HHHHHH) (SEQ ID NO: 7), followed by a HDEL tetrapeptide was fused (HDEL) (SEQ ID NO: 1). The amino acid sequences of the rFGE fusion proteins that were coexpressed in a *Y. lipolytica* strain expressing rhIDS are set out as SEQ ID NOs: 53, 55, 57, 59 and 61 (corresponding nucleic acid sequences SEQ ID NOs: 54, 56, 58, 60 and 62 respectively). The amino acid sequences of the corresponding mature FGEs are set out as SEQ ID NOs: 43, 45, 47, 49 and 51 (corresponding nucleic acid sequences SEQ ID NOs: 44, 46, 48, 50 and 52 respectively).

All FGE fusion coding sequences were synthesized and codon-optimized for expression within *Y. lipolytica* and were flanked by *Bam*HI and *Avr*II restriction sites for cloning of the segment into an expression vector under the control of the inducible POX2 promoter or Hp4d promoter. A summary of these FGE co-expression strains is shown in Table 13. Each strain carries one copy of the rhIDS coding sequence co-expressed with two copies of either *Tupaia chinensis* (Tup), *Monodelphis domestica* (Md), *Gallus gallus* (Gg), *Dendroctonus ponderosa* (Dp) or *Columba livia* (C1) rFGE coding sequence. In each strain, the two FGE copies are expressed under the POX2 promoter.

**Table 13. Summary of the additional FGE co-expression strains**

| **Strain ID** | **rFGE expressed** | **Strain genotype** |
|---|---|---|
| OXYY3084 | TupFGE | MATA, leu2-958, ura3-302, xpe2-322, ade2-844, ΔSc suc2, Δoch1,, URA3 ::POX2-MNN4, OCH1 ::Hp4d-MNN4, POX2-Lip2pre-hIDS:URA3Ex::zeta, POX2-Lip2pre-TupFGE:Leu2Ex::zeta, POX2-Lip2pre-TupFGE:Ade2Ex: :zeta |
| OXYY3085 | MdFGE | MATA, leu2-958, ura3-302, xpe2-322, ade2-844, ΔSc suc2, Δochl, , URA3 ::POX2-MNN4, OCH1 ::Hp4d-MNN4, POX2-Lip2pre-hIDS:URA3Ex::zeta, POX2-Lip2pre-MdFGE:Leu2Ex::zeta, POX2-Lip2pre-MdFGE:Ade2Ex: :zeta |
| OXYY3086 | GgFGE | MATA, leu2-958, ura3-302, xpe2-322, ade2-844, ΔSc suc2, Δochl, , URA3 ::POX2-MNN4, OCH1 ::Hp4d-MNN4, POX2-Lip2pre-hIDS:URA3Ex::zeta, POX2-Lip2pre-GgFGE:Leu2Ex::zeta, POX2-Lip2pre-GgFGE:Ade2Ex: :zeta |
| OXYY3087 | DpFGE | MATA, leu2-958, ura3-302, xpe2-322, ade2-844, ΔSc suc2, Δoch1,, URA3 ::POX2-MNN4, OCH1 ::Hp4d-MNN4, POX2-Lip2pre-hIDS:URA3Ex::zeta, POX2-Lip2pre-DpFGE:Leu2Ex::zeta, POX2-Lip2pre-DpFGE:Ade2Ex: : zeta |
| OXYY3088 | ClFGE | MATA, leu2-958, ura3-302, xpe2-322, ade2-844, ΔSc suc2, Δochl, , URA3 ::POX2-MNN4, OCH1 ::Hp4d-MNN4, POX2-Lip2pre-hIDS:URA3Ex::zeta, POX2-Lip2pre-ClFGE:Leu2Ex::zeta, POX2-Lip2pre-ClFGE: Ade2Ex: :zeta |

Clonal selection was based on 24-well cultivation. Strains of *Y. lipolytica* co-expressing rIDS and rFGE were successfully cultivated in a bioreactor (Dasgip 43) as set out in Table 14.

**Table 14. Summary of the novel FGE co-expression strains cultivated in the bioreactor**

| **Unit** | **Srain** | **Description** |
|---|---|---|
| 1 | OXYY3086 | 1c rhIDS (POX), 2c GgFGE (POX/POX) |
| 2 | OXYY3087 | 1c rhIDS (POX), 2c DpFGE (POX/POX) |
| 3 | OXYY3088 | 1c rhIDS (POX), 2c ClFGE (POX/POX) |
| 4 | OXYY3085 | 1c rhIDS (POX), 2c MdFGE (POX/POX) |
| 5 | OXYY3084 | 1c rhIDS (POX), 2c TupFGE (POX/POX) |
| 6 | OXYY3089 | 1c rhIDS (POX), 2c MNS1-HpFGE (POX/POX) |

Fairly constant expression levels of rhIDs were observed with the different strain backgrounds. Unit 4 (MdFGE; *Monodelphis domestica*) showed incresed levels of rhIDS, however increased levels of rhIDs degradation were also visible. A variable degree of FGE can be observed in the supernatant with strong leakage of FGE to the medium in MdFGE strain. This can be explained by saturation of the HDEL receptor leading to significant leakage of the FGE into the supernatant.

As shown in Table 15, 100 % FGly conversion for rhIDS was obtained for co-expression with MdFGE (*Monodelphis domestica*), ClFGE (*Columba livia*) and TupFGE (*Tupaia chinensis*). GgFGE (*Gallus gallus*) and DpFGE (*Dendroctonus ponderosa*) co-expression resulted in incomplete Cys to FGly conversion. The activity data show the same trend, with high specific activity for TupFGE, ClFGE and MdFGE, intermediate activity for GgFGE and low activity for DpFGE.

**Table 15. Overview of % activity and FGly conversion as determined by LC-MS for the additional strains.**

| **rFGE** | **% activity** | **%FGly (LC-MS)** |
|---|---|---|
| GgFGE | 33 | 78 |
| DpFGE | 5 | 25 |
| ClGFE | 61 | 100 |
| MdFGE | 58 | 100 |
| TupFGE | 51 | 99 |

In summary, co-expression of three rFGE's, MdFGE, ClFGE and TupFGE resulted in complete or essentially complete conversion of FGly in rhIDS.

### EXAMPLE 15

### Analysis of the activity of rhIDS obtained from a recombinant strain of Yarrowa lipolytica not co-expressing an rFGE

A recombinant *Yarrowia lipolytica* strain (T135) was constructed containing two POX driven copies of a rhIDS coding sequence with the following genotype: Δoch1, URA3 ::POX2-MNN4, OCH1 ::Hp4d-MNN4, POX2-Lip2pre-hIDS::zeta, POX2-Lip2pre-hIDS::zeta. This strain contained no rFGE expressing nucleotide sequence. Production of rhIDS under oleic acid inducting condition was performed in a fermentor using standard protocol.
To compare and evaluate the level of production and secretion of rhIDS, a fluorogenic activity assay using 4-methylumbelliferyl-alpha-L-iduronide-2-sulphate (4MU) was employed. The activity of rhIDS in supernatant recovered from the culture was assayed as previously described Voznyi et al (2001), Journal of Inherited Metabolic Disease, 24, 675-680). The assay does not detect sulfamidase activity. Absorbances are summarized in Table 16. A control *Yarrowia lipolytica* strain was constructed that did not express rhIDS but expressed human sulfamidase (hSGSH) and co-expressed BtfGE (1 copy, POX2 driven) and hPDI (1 copy, POX2 driven). Clearly, elevated sulfatase activity could be observed in the supernatant of the rhIDS expressing strain, corresponding to 30 ng/ml of active rhIDS. Results therefore show from the low IDS activity in the control strain that expression of FGE is required for IDS activity.

**Table 16. IDS activity (in absorbance units) secreted by a recombinant strain of Yarrowia lipolytica producing rhIDS versus a control strain expressing hSGSH.**

| **Supernatant dilution factor** | **Strain T135 (expressing rhIDS)** | **Control Strain (not expressing rhIDS)** |
|---|---|---|
| **10** | **2717** | **44** |
| **50** | **606** | **21** |
| **100** | **353** | **37** |

### EXAMPLE 16

### Construction of Yarrowia lipolytica strains co-expressing human endoplasmic reticulum resident protein 44 (hERP44) and rFGE

*Yarrowia lipolytica* strains are constructed in which rFGEs (e.g., BtFGE) without a C-terminal HDEL signal sequence are co-expressed with hERp44. In order to do this, two expression vectors are made. The first contains a coding nucleotide sequence encoding a fusion polypeptide consisting of, N-terminus to C-terminus, the Lip2 signal sequence (SEQ ID NO: 6), and the mature form of hERp44 (SEQ ID NO: 30; Accession: CAC87611.1) with the C-terminal RDEL sequence replaced by a HDEL tetrapeptide (SEQ ID NO: 1). The second vector contains a coding nucleotide sequence encoding a fusion polypeptide consisting of, N-terminus to C-terminus, the Lip2 signal sequence (SEQ ID NO: 6) and the mature form of an rFGE (e.g., BtFGE). It is expected that co-expression of the two expression vectors in *Yarrowia lipolytica* cells results in the localization of rFGE fusion polypeptide to the ER of the cells.

### SEQUENCES REFERRED TO IN THE APPLICATION

SEQ ID 1: HDEL tag
   HDEL
SEQ ID 2: HDEL tag coding sequence
   CACGACGAGCTG
SEQ ID 3: KDEL tag
   KDEL
SEQ ID 4: DDEL tag
   DDEL
SEQ ID NO 5: LIP2 leader sequence
   MKLSTILFTACA TLAAA
SEQ ID NO 6: LIP2 leader sequence; coding sequence
   ATGAAGCTGTCTACTATTCTCTTTACTGCCTGCGCTACTCTCGCCGCTGCT
SEQ ID NO 7: Six Histidine (HIS) tag
   HHHHHH
SEQ ID NO 8: Six Histidine (HIS) tag
   CACCACCACCACCACCAC
SEQ ID NO 9; Human FGE mature protein
SEQ ID NO 10; Human FGE coding sequence of the mature protein
SEQ ID NO 11; *Streptomyces coelicolor* FGE mature protein
SEQ ID NO 12; *Streptomyces coelicolor* FGE coding sequence of the mature protein
SEQ ID NO 13; *Hemicentrotus pulcherrimus* FGE mature protein
SEQ ID NO 14; *Hemicentrotus pulcherrimus* FGE coding sequence of the mature protein
SEQ ID NO 15; *Bos taurus* FGE coding sequence mature sequence
SEQ ID NO 16; *Bos taurus* FGE coding sequence of the mature protein
SEQ ID NO 17; *Mycobacterium tuberculosis* FGE mature sequence
SEQ ID NO 18; *Mycobacterium tuberculosis* FGE coding sequence of the mature protein
SEQ ID NO 19; human iduronate sulfatase mature sequence
SEQ ID NO 20; human iduronate sulfatase coding sequence of the mature protein
SEQ ID NO 21; human PDI mature sequence
SEQ ID NO 22; human PDI coding sequence of the mature protein
SEQ ID NO 23: hFGE leader sequence
   MAAPALGLVCGRCPELGLVLLLLLLSLLCGAAG
SEQ ID NO 24; human sulfamidase mature sequence
SEQ ID NO25: coding sequence of mature sulfamidase (SGSH)
   >SGSH-1 Genscript (62bp - 1501bp, direct) 1440bp
SEQ ID NO 26: MNS1 anchorage domain (AA 1-163 of XP 502939.1)
SEQ ID NO 27: Coding sequence for the MNS1 anchorage domain (AA 1-163 of XP_502939.1)
SEQ ID NO 28: WBP1 anchorage domain (AA 400-505 of XP_502492.1)
SEQ ID NO 29: Coding sequence for the WBP1 anchorage domain (AA 400-505 of XP_502492. 1)
SEQ ID NO 30: ERp44 mature protein
SEQ ID NO 31: Coding sequence for the ERp44 mature protein
SEQ ID NO 32: Fusion construct: LIP2-BtFGE-6xHis-HDEL
SEQ ID NO 33: RDEL
   RDEL
SEQ ID NO 34: Conserved sequence of Iduronate Sulfatase
   CAPSRVSFL TGR
SEQ ID NO 35: MNS1-HpFGE-6xHis fusion construct
SEQ ID NO 36: Coding sequence for MNS1-HpFGE-6xHis fusion construct
SEQ ID NO 37: MNS1-BtFGE-6xHis fusion construct
SEQ ID NO 38: Coding sequence for the MNS1-BtFGE-6xHis fusion construct
SEQ ID NO 39: Lip2pre-6xHis-BtFGE-WBPlfusion construct
SEQ ID NO 40: Coding sequence for the Lip2pre-6xHis-BtFGE-WBP1fusion construct
SEQ ID NO 41: Chimeric Lip2pre-BtFGE-HpFGE-6xHis-HDEL fusion construct
SEQ ID NO 42: Coding sequence for the Chimeric Lip2pre-BtFGE-HpFGE-6xHis-HDEL fusion construct
SEQ ID NO 43: *Tupaia chinensis* FGE
SEQ ID NO 44: Coding sequence for the *Tupaia chinensis* FGE
SEQ ID NO 45: *Monodelphis domestica* FGE
SEQ ID NO 46: Coding sequence for the *Monodelphis domestica* FGE
SEQ ID NO 47: *Gallus gallus* FGE
SEQ ID NO 48: Coding sequence for the *Gallus gallus* FGE
SEQ ID NO 49: *Dendroctonus ponderosa* FGE
SEQ ID NO 50: Coding sequence for the *Dendroctonus ponderosa* FGE
SEQ ID NO 51: *Columba livia* FGE
SEQ ID NO 52: Coding sequence for the *Columba livia* FGE
SEQ ID NO 53: *Tupaia chinensis* Lip2-TupFGE-His6-HDEL fusion construct
SEQ ID NO 54: Coding sequence for the Lip2-TupFGE-His6-HDEL fusion protein
SEQ ID NO 55: *Monodelphis domestica* Lip2-MdFGE-His6-HDEL fusion construct
SEQ ID NO 56: Coding sequence for the Lip2-MdFGE-His6-HDEL fusion protein
SEQ ID NO 57: *Gallus gallus* Lip2-GgFGE-His6-HDEL fusion construct
SEQ ID NO 58: Coding sequence for the Lip2-GgFGE-His6-HDEL fusion protein
SEQ ID NO 59: *Dendroctonus ponderosa* Lip2-DpFGE-His6-HDEL fusion construct
SEQ ID NO 60: Coding sequence for the Lip2-DpFGE-His6-HDEL fusion protein
SEQ ID NO 61: *Columba livia* Lip2-C1FGE-His6-HDEL fusion construct
SEQ ID NO 62: Coding sequence for the Lip2-C1FGE-His6-HDEL fusion protein
SEQ ID NO 63: MNS1-C1FGE fusion construct
SEQ ID NO 64: Coding sequence for the MNS1-C1FGE fusion protein
SEQ ID NO 65: c-myc protein tag
   EQKLISEEDL
SEQ ID NO 66: Coding sequence for the c-myc protein tag
   GAACAAAAACTCATCTCAGAAGAGGATCTGTAA
SEQ ID NO 67: MNS1-C1FGE-c-myc fusion construct
SEQ ID NO 68: Coding sequence for the MNS1-C1FGE-c-myc fusion protein

## Claims

1. A method for making a type I sulfatase in an active form,
(I) the method comprising:
a) providing a fungal cell genetically engineered to produce a protein with the type I sulfatase activating activity of a Formylglycine Generating Enzyme (FGE); and
b) introducing into the cell a nucleic acid encoding a type I sulfatase, wherein the encoded type I sulfatase, without an activation step, is in an inactive form,
wherein, after the introduction, the cell produces, or produces at an increased level, the type I sulfatase in an active form, and wherein, after step (b), the cell, or the progeny thereof, is cultivated at a pO₂ of 5% - 40%; or
(II) the method comprising:
a) providing a fungal cell genetically engineered to produce a type I sulfatase, wherein the type I sulfatase, without an activation step, is in an inactive form; and
b) introducing into the cell a nucleic acid encoding a protein with the type I sulfatase activating activity of a Formylglycine Generating Enzyme (FGE),
wherein, after the introduction, the cell produces, or produces at an increased level, the type I sulfatase in an active form, and wherein, after step (b), the cell, or the progeny thereof, is cultivated at a pO₂ of 5% - 40%.

2. The method of claim 1, wherein the fungal cell is a *Yarrowia lipolytica* cell.

3. The method of claim 1, wherein:
(AA) the protein with the type I sulfatase activating activity of a FGE comprises:
(a) a mature wild type FGE polypeptide;
(b) a functional fragment of a mature wild type FGE polypeptide comprising at least 50 consecutive amino acids of the mature wild type FGE;
(c) a polypeptide with at least 80% identity to (a);
(d) a polypeptide with at least 90% identity to (b);
(e) (a) but with no more than 10 conservative substitutions; or
(f) (b) but with no more than 5 conservative substitutions; or
(AB) the method is as defined in (AA) and the mature wild type FGE polypeptide is mature wild type protein SCO7548; or
(AC) the method is as defined in (AA) and the mature wild type FGE polypeptide is mature wild type protein Rv0712; or
(AD) the method is as defined in (AA) and the mature wild type FGE polypeptide is mature wild type sulfatase modifying factor 1; or
(AE) the method is as defined in (AA) and the mature wild type FGE polypeptide is mature wild type C-alpha-formylglycine-generating enzyme; or
(AF) the method is as defined in (AA) and the mature wild type FGE polypeptide is mature wild type sulfatase-modifying factor 1; or
(AG) the method is as defined in any of claims 1 or 2 or in (AA) and the protein with the type I sulfatase activating activity of a FGE is a prokaryotic protein with the type I sulfatase activating activity of a FGE; or
(AH) the method is as defined in (AG) and the prokaryote is *Mycobacterium tuberculosis* or *Streptomyces coelicolor*; or
(AI) the method is as defined in any of claims 1 or 2 or in (AA) and the protein with the type I sulfatase activating activity of a FGE is a protein with the type I sulfatase activating activity of a eukaryotic FGE; or
(AJ) the method is as defined in (AI) and the eukaryote is *Homo sapiens, Bos taurus, Hemicentrotus pulcherrimus, Tupaia chinensis, Monodelphis domestica, Gallus gallus, Dendroctonus ponderosa,* or *Columba livia*; or
(AK) the method is as defined in any of claims 1 or 2 or in any of (AA) to (AJ) and the protein with the type I sulfatase activating activity of a FGE further comprises an ER targeting motif; or
(AL) the method is as defined in (AK) and the ER targeting motif is fused to the C-terminus of the protein with the type I sulfatase activating activity of a FGE polypeptide; or
(AM) the method is as defined in (AK) and the ER targeting motif is fused to the N-terminus of the protein with the type I sulfatase activating activity of a FGE polypeptide; or
(AN) the method is as defined in any one of (AK) to (AM) and the ER targeting motif comprises HDEL (SEQ ID NO: 1); or
(AO) the method is as defined in any one of (AK) to (AM) and the ER targeting motif comprises KDEL (SEQ ID NO: 3); or
(AP) the method is as defined in any one of (AK) to (AM) and the ER targeting motif comprises DDEL (SEQ ID NO: 4) or RDEL (SEQ ID NO: 33); or
(AQ) the method is as defined in any one of (AK) to (AM) and the ER targeting motif comprises a yeast MNS 1 transmembrane anchor polypeptide; or
(AR) the method is as defined in (AQ) and the yeast MNS1 transmembrane anchor polypeptide comprises the *Yarrowia lipolytica* MNS 1 transmembrane anchor polypeptide; or
(AS) the method is as defined in any one of (AK) to (AM) and the ER targeting motif comprises a yeast WBP1 transmembrane anchor polypeptide; or
(AT) the method is as defined in (AS) and the yeast WBP1 transmembrane anchor polypeptide comprises the *Yarrowia lipolytica* WBP1 transmembrane anchor polypeptide; or
(AU) the method is as defined in any one of claims 1 or 2 or in any one of (AA) to (AT) and the type I sulfatase or the protein with the type I sulfatase activating activity of a FGE further comprises a leader or signal sequence; or
(AV) the method is as defined in (AU) and the leader or signal sequence is an exogenous leader or signal sequence; or
(AW) the method is as defined in (AU) and the leader or signal sequence is an endogenous leader or signal sequence; or
(AX) the method is as defined in any one of (AU) to (AW) and the leader or signal sequence is Lip2pre; or
(AY) the method is as defined in any one of claims 1 or 2 or in any one of (AA) to (AX) and the method further comprises introducing into the cell a nucleic acid encoding a polypeptide capable of effecting mannosyl phosphorylation, or a functional fragment thereof; or
(AW) the method is as defined in (AY) and the polypeptide capable of effecting mannosyl phosphorylation is selected from the group consisting of MNN4, PNO1, and MNN6; or
(AZ) the method is as defined in any one of claims 1 or 2 or in any one of (AA) to (AW) and the method further comprises introducing into the cell a nucleic acid encoding a mannosidase, or a functional fragment thereof, capable of hydrolyzing a terminal mannose-1-phospho-6-mannose moiety to a terminal phospho-6-mannose; or
(BA) the method is as defined in (AZ) and the mannosidase is the family 92 glycoside hydrolase CcMan5 from *Cellulosimicrobium cellulans*; or
(BB) the method is as defined in (AZ) and the mannosidase is also capable of removing a mannose residue bound by an alpha 1,2 linkage to the underlying mannose in the terminal mannose-1-phospho-6-mannose moiety; or
(BC) the method is as defined in (BB) and the mannosidase is a family 38 glycoside hydrolase selected from the group consisting of a *Canavalia ensiformis* (Jack Bean) mannosidase and *Yarrowia lipolytica* AMS1 mannosidase; or
(BD) the method is as defined in (AZ) and the method further comprises introducing into the cell a nucleic acid encoding a mannosidase, or a functional fragment thereof, that is capable of removing a mannose residue bound by an alpha 1,2 linkage to the underlying mannose in the terminal mannose-1-phospho-6-mannose moiety; or
(BE) the method is as defined in (BD) and the mannosidase is selected from the group consisting of the family 38 glycoside hydrolase *Canavalia ensiformis* (Jack Bean) mannosidase, the family 38 glycoside hydrolase *Yarrowia lipolytica* AMS1 mannosidase, the family 47 glycoside hydrolase *Aspergillus satoi* As mannosidase, and the family 92 glycoside hydrolase *Cellulosimicrobium cellulans* CcMan4 mannosidase; or
(BF) the method is as defined in any one of claims 1 or 2 or in any one of (AA) to (BE) and the method further comprises introducing into the cell a nucleic acid encoding a trafficking protein, or a functional fragment thereof, wherein the trafficking protein or functional fragment thereof, directs the protein with the type I sulfatase activating activity of a FGE to the endoplasmic reticulum (ER) of the cell; or
(BG) the method is as defined in (BF) and the trafficking protein is Protein Disulfide Isomerase (PDI); or
(BH) the method is as defined in (BF) and the trafficking protein is Endoplasmic Reticulum Protein 44 (Erp44) or human SUMF2; or
(BI) the method is as defined in (BF) and the trafficking protein, or functional fragment thereof, binds to the protein with the type I sulfatase activating activity of a FGE; or
(BJ) the method is as defined in any one of claims 1 or 2 or in any one of (AA) to (BI) and the fungal cell is a yeast cell; or
(BK) the method is as defined in (BJ) and the yeast cell is a cell of a methylotrophic yeast; or
(BL) the method is as defined in (BK) and the methylotrophic yeast is selected from the group comprising *Pichia pastoris, Pichia methanolica, Ogataea minuta,* and *Hansenula polymorpha*; or
(BM) the method is as defined in any one of claims 1 or 2 or in any one of (AA) to (BI) and the fungal cell is a cell of a filamentous fungus; or
(BN) the method is as defined in (BM) and the filamentous fungus is selected from a group consisting of: *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi*, *Aspergillus tamari, Aspergillus terreus, Aspergillus ustus, Aspergillus versicolor, Trichoderma,* and *Neurospora*; or
(BO) the method is as defined in any one of claims 1 or 2 or in any one of (AA) to (BN) and the type I sulfatase is a human type I sulfatase; or
(BP) the method is as defined in any one of claims 1 or 2 or in any one of (AA) to (BN) and the type I sulfatase is iduronate sulfatase; or
(BQ) the method is as defined in any one of claims 1 or 2 or in any one of (AA) to (BN) and the type I sulfatase is sulfamidase; or
(BR) the method is as defined in any one of claims 1 or 2 or in any one of (AA) to (BQ) and the cell comprises a deficiency in OCH1 activity; or
(BS) the method is as defined in (AA) and the protein with the type I sulfatase activating activity of a FGE comprises any one of (a) - (f) and the mature wild type FGE polypeptide is a mature wild type *Columba livia* FGE polypeptide and further comprises a yeast MNS 1 transmembrane anchor polypeptide; or
(BT) the method is as defined in (BS) and the protein with the type I sulfatase activating activity of a FGE comprises the amino acid sequence set forth in SEQ ID NO: 63; or
(BU) the method is as defined in (AA) and the mature wild type FGE polypeptide is:
(i) a mature wild type FGE of *Hemicentrotus pulcherrimus* having the amino acid sequence set forth in SEQ ID NO: 13, a mature wild type FGE of *Gallus gallus* having the amino acid sequence set forth in SEQ ID NO: 47, a mature wild type FGE of *Dendroctonus ponderosa* having the amino acid sequence set forth in SEQ ID NO: 49, or a mature wild type FGE of *Columba livia* having the amino acid sequence set forth in SEQ ID NO: 51; or
(ii) a functional mature FGE having an amino acid sequence that is at least 80% identical to any one of the amino acid sequences of (i).

4. The method of any one of claims 1-3, wherein the method results in the production of a type I sulfatase in which greater than 10%, 20%, 30%, 40%, 50%, 60%, 70%, 80%, or 90% of the molecules of the type I sulfatase comprise a formylglycine residue in the active site.

5. The method of any one of claims 1-3, wherein the method results in the production of a type I sulfatase in which greater than 95% of the molecules of the type I sulfatase comprise a formylglycine residue in the active site.

6. The method of any one of claims 1-3, wherein the method results in the production of a type I sulfatase in which 100% of the molecules of the type I sulfatase comprise a formylglycine residue in the active site.

7. The method of claim 1, wherein the protein with the type I sulfatase activating activity of a FGE is encoded by a nucleotide sequence comprising
(i) the nucleic acid sequence set out in any one of SEQ ID NOs: 14, 48, 50 or 52; or
(ii) a nucleic acid sequence that is at least 80% identical to any one of the nucleic acid sequences of (i) and encodes a mature functional FGE; or
(iii) a nucleic acid sequence that hybridizes to a complement of any one of the nucleic acid sequences of (i) under high stringency and encodes a mature functional FGE.

## Patentansprüche

1. Verfahren zur Herstellung einer Typ-I-Sulfatase in aktiver Form,
(I) wobei das Verfahren umfasst:
a) Bereitstellen einer Pilzzelle, die gentechnisch verändert wurde, um ein Protein mit der Typ-I-Sulfatase-aktivierenden Aktivität eines Formylglycin-generierenden-Enzyms (FGE) herzustellen; und
b) Einbringen einer Nukleinsäure, die eine Typ-I-Sulfatase codiert, in die Zelle, wobei die codierte Typ-I-Sulfatase, ohne einen Aktivierungsschritt, in einer inaktiven Form vorliegt,
wobei nach dem Einbringen die Zelle die Typ-I-Sulfatase in aktiver Form produziert oder in erhöhtem Maße produziert, und wobei nach Schritt (b) die Zelle oder deren Nachkommen bei einem pO₂ von 5% - 40% kultiviert werden; oder
(II) wobei das Verfahren umfasst:
a) Bereitstellen einer Pilzzelle, die gentechnisch verändert wurde, um eine Typ-I-Sulfatase herzustellen, wobei die Typ-I-Sulfatase, ohne einen Aktivierungsschritt, in inaktiver Form vorliegt; und
b) Einbringen einer Nukleinsäure, die ein Protein mit der Typ-I-Sulfatase-aktivierenden Aktivität eines Formylglycin-generierenden-Enzyms (FGE) codiert, in die Zelle,
wobei nach dem Einbringen die Zelle die Typ I-Sulfatase in aktiver Form produziert oder in erhöhtem Maße produziert und wobei nach Schritt (b) die Zelle oder deren Nachkommen bei einem pO₂ von 5% - 40% kultiviert werden.

2. Verfahren nach Anspruch 1, wobei die Pilzzelle eine *Yarrowia-lipolytica-*Zelle ist.

3. Verfahren nach Anspruch 1, wobei:
(AA) das Protein mit der Typ-I-Sulfatase-aktivierenden Aktivität eines FGE umfasst:
(a) ein reifes Wildtyp-FGE-Polypeptid;
(b) ein funktionelles Fragment eines reifen Wildtyp-FGE-Polypeptids, umfassend mindestens 50 aufeinanderfolgende Aminosäuren des reifen Wildtyp-FGE;
(c) ein Polypeptid mit mindestens 80% Identität zu (a);
(d) ein Polypeptid mit mindestens 90% Identität zu (b);
(e) (a) jedoch mit nicht mehr als 10 konservativen Substitutionen; oder
(f) (b) jedoch mit nicht mehr als 5 konservativen Substitutionen; oder
(AB) das Verfahren wie in (AA) definiert ist und das reife Wildtyp-FGE-Polypeptid das reife Wildtyp-Protein SCO7548 ist; oder
(AC) das Verfahren wie in (AA) definiert ist und das reife Wildtyp-FGE-Polypeptid das reife Wildtyp-Protein Rv0712 ist; oder
(AD) das Verfahren wie in (AA) definiert ist und das reife Wildtyp-FGE-Polypeptid reifer Wildtyp-Sulfatase-modifizierender Faktor 1 ist; oder
(AE) das Verfahren wie in (AA) definiert ist und das reife Wildtyp-FGE-Polypeptid reifes Wildtyp-C-alpha-Formylglycin-generierendes-Enzym ist; oder
(AF) das Verfahren wie in (AA) definiert ist und das reife Wildtyp-FGE-Polypeptid reifer Wildtyp-Sulfatase-modifizierender Faktor 1 ist; oder
(AG) das Verfahren wie in einem der Ansprüche 1 oder 2 oder in (AA) definiert ist und das Protein mit der Typ-I-Sulfatase-aktivierenden Aktivität eines FGE ein prokaryotisches Protein mit der Typ-I-Sulfatase-aktivierenden Aktivität eines FGE ist; oder
(AH) das Verfahren wie in (AG) definiert ist und der Prokaryot *Mycobacterium tuberculosis* oder *Streptomyces coelicolor* ist; oder
(AI) das Verfahren wie in einem der Ansprüche 1 oder 2 oder in (AA) definiert ist und das Protein mit der Typ-I-Sulfatase-aktivierenden Aktivität eines FGE ein Protein mit der Typ-I-Sulfatase-aktivierenden Aktivität eines eukaryotischen FGE ist; oder
(AJ) das Verfahren wie in (AI) definiert ist und der Eukaryot *Homo sapiens, Bos taurus, Hemicentrotus pulcherrimus, Tupaia chinensis, Monodelphis domestica, Gallus gallus, Dendroctonus ponderosa* oder *Columba livia* ist; oder
(AK) das Verfahren wie in einem der Ansprüche 1 oder 2 oder in einem von (AA) bis (AJ) definiert ist, und das Protein mit der Typ-I-Sulfatase-aktivierenden Aktivität eines FGE ferner ein ER-Targeting-Motiv umfasst; oder
(AL) das Verfahren wie in (AK) definiert ist und das ER-Targeting-Motiv an den C-Terminus des Proteins mit der Typ-I-Sulfatase-aktivierenden Aktivität eines FGE-Polypeptids fusioniert ist; oder
(AM) das Verfahren wie in (AK) definiert ist und das ER-Targeting-Motiv an den N-Terminus des Proteins mit der Typ-I-Sulfatase-aktivierenden Aktivität eines FGE-Polypeptids fusioniert ist; oder
(AN) das Verfahren wie in einem von (AK) bis (AM) definiert ist und das ER-Targeting-Motiv HDEL (SEQ ID NO: 1) umfasst; oder
(AO) das Verfahren wie in einem von (AK) bis (AM) definiert ist und das ER-Targeting-Motiv KDEL (SEQ ID NO: 3) umfasst; oder
(AP) das Verfahren wie in einem von (AK) bis (AM) definiert ist und das ER-Targeting-Motiv DDEL (SEQ ID NO: 4) oder RDEL (SEQ ID NO: 33) umfasst; oder
(AQ) das Verfahren wie in einem von (AK) bis (AM) definiert ist und das ER-Targeting-Motiv ein Hefe-MNS1-Transmembran-Ankerpolypeptid umfasst; oder
(AR) das Verfahren wie in (AQ) definiert ist und das Hefe-MNSl-Transmembran-Ankerpolypeptid das *Yarrowia-lipolytica*-MNS1-Transmembran-Ankerpolypeptid umfasst; oder
(AS) das Verfahren wie in einem von (AK) bis (AM) definiert ist und das ER-Targeting-Motiv ein Hefe-WBPl-Transmembran-Ankerpolypeptid umfasst; oder
(AT) das Verfahren wie in (AS) definiert ist und das Hefe-WBPl-Transmembran-Ankerpolypeptid das *Yarrowia-lipolytica*-WBP1-Transmembran-Ankerpolypeptid umfasst; oder
(AU) das Verfahren wie in einem der Ansprüche 1 oder 2 oder in einem von (AA) bis (AT) definiert ist, und die Typ-I-Sulfatase oder das Protein mit der Typ-I-Sulfatase-aktivierenden Aktivität eines FGE ferner eine Leader- oder Signalsequenz umfasst; oder
(AV) das Verfahren wie in (AU) definiert ist und die Leader- oder Signalsequenz eine exogene Leader-oder Signalsequenz ist; oder
(AW) das Verfahren wie in (AU) definiert ist und die Leader- oder Signalsequenz eine endogene Leader-oder Signalsequenz ist; oder
(AX) das Verfahren wie in einem von (AU) bis (AW) definiert ist und die Leader- oder Signalsequenz Lip2pre ist; oder
(AY) das Verfahren wie in einem der Ansprüche 1 oder 2 oder in einem von (AA) bis (AX) definiert ist und das Verfahren ferner das Einbringen einer Nukleinsäure, die ein Polypeptid codiert, das zum Bewirken von Mannosylphosphorylierung in der Lage ist, oder eines funktionellen Fragments davon, in die Zelle umfasst; oder
(AW) das Verfahren wie in (AY) definiert ist und das Polypeptid, das zum Bewirken von Mannosylphosphorylierung in der Lage ist, ausgewählt wird aus der Gruppe bestehend aus MNN4, PNO1 und MNN6; oder
(AZ) das Verfahren wie in einem der Ansprüche 1 oder 2 oder in einem von (AA) bis (AW) definiert ist, und das Verfahren ferner das Einbringen einer Nukleinsäure, codierend für eine Mannosidase oder ein funktionelles Fragment davon, welche(s) fähig ist, eine terminale Mannose-1-Phospho-6-mannose-Einheit zu einer terminalen Phospho-6-mannose zu hydrolysieren, in die Zelle umfasst; oder
(BA) das Verfahren wie in (AZ) definiert ist und es sich bei der Mannosidase um die Familie-92-Glycosid-hydrolase CcMan5 aus *Cellulosimicrobium cellulans* handelt; oder
(BB) das Verfahren wie in (AZ) definiert ist und die Mannosidase auch in der Lage ist, einen Mannoserest zu entfernen, der durch eine alpha-1,2-Bindung an die darunterliegende Mannose in der terminalen Mannose-1-Phospho-6-mannose-Einheit gebunden ist; oder
(BC) das Verfahren wie in (BB) definiert ist und die Mannosidase eine Familie-38-Glycosidhydrolase ist, ausgewählt aus der Gruppe bestehend aus einer *Canavalia-ensiformis* (Jackbohne)-Mannosidase und *Yarrowia-lipolytica*-AMS1-Mannosidase; oder
(BD) das Verfahren wie in (AZ) definiert ist und das Verfahren ferner das Einbringen einer Nukleinsäure, codierend eine Mannosidase oder ein funktionelles Fragment davon, welche(s) in der Lage ist, einen Mannoserest zu entfernen, der durch eine alpha-1,2-Bindung an die darunterliegende Mannose in der terminalen Mannose-1-phospho-6-mannose-Einheit gebunden ist, in die Zelle umfasst; oder
(BE) das Verfahren wie in (BD) definiert ist und die Mannosidase ausgewählt ist aus der Gruppe bestehend aus der Familie-38-Glycosidhydrolase *Canavalia-ensiformis* (Jackbohne)-Mannosidase, der Familie-38-Glycosid-hydrolase *Yarrowia-lipolytica*-AMS1-Mannosidase, der Familie-47-Glycosidhydrolase *Aspergillus-satoi-As-Man*nosidase und der Familie-92-Glycosidhydrolase *Cellulosimicrobium-cellulans-CcMan4-Mannosidase;* oder
(BF) das Verfahren wie in einem der Ansprüche 1 oder 2 oder in einem von (AA) bis (BE) definiert ist, und das Verfahren ferner das Einbringen einer Nukleinsäure, die ein Trafficking-Protein oder ein funktionelles Fragment davon codiert, in die Zelle umfasst, wobei das Trafficking-Protein oder das funktionelle Fragment davon das Protein mit der Typ-I-Sulfatase-aktivierenden Aktivität eines FGE zum endoplasmatischen Retikulum (ER) der Zelle lenkt; oder
(BG) das Verfahren wie in (BF) definiert ist und es sich bei dem Trafficking-Protein um Proteindisulfidisomerase (PDI) handelt; oder
(BH) das Verfahren wie in (BF) definiert ist und das Trafficking-Protein Endoplasmic-Reticulum-Protein 44 (Erp44) oder humanes SUMF2 ist; oder
(BI) das Verfahren wie in (BF) definiert ist und das Trafficking-Protein oder das funktionelle Fragment davon an das Protein mit der Typ-I-Sulfatase-aktivierenden Aktivität eines FGE bindet; oder
(BJ) das Verfahren wie in einem der Ansprüche 1 oder 2 oder in einem von (AA) bis (BI) definiert ist und die Pilzzelle eine Hefezelle ist; oder
(BK) das Verfahren wie in (BJ) definiert ist und die Hefezelle eine Zelle einer methylotrophen Hefe ist; oder
(BL) das Verfahren wie in (BK) definiert ist und die methylotrophe Hefe ausgewählt ist aus der Gruppe, umfassend *Pichia pastoris, Pichia methanolica, Ogataea minuta* und *Hansenula polymorpha;* oder
(BM) das Verfahren wie in einem der Ansprüche 1 oder 2 oder in einem von (AA) bis (BI) definiert ist und die Pilzzelle eine Zelle eines Fadenpilzes (filamentösen Pilzes) ist; oder
(BN) das Verfahren wie in (BM) definiert ist und der Fadenpilz ausgewählt ist aus einer Gruppe bestehend aus: *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus ustus, Aspergillus versicolor, Trichoderma* und *Neurospora*; oder
(BO) das Verfahren wie in einem der Ansprüche 1 oder 2 oder in einem von (AA) bis (BN) definiert ist und die Typ-I-Sulfatase eine menschliche Typ-I-Sulfatase ist; oder
(BP) das Verfahren wie in einem der Ansprüche 1 oder 2 oder in einem von (AA) bis (BN) definiert ist und die Typ-I-Sulfatase Iduronatsulfatase ist; oder
(BQ) das Verfahren wie in einem der Ansprüche 1 oder 2 oder in einem von (AA) bis (BN) definiert ist und die Typ-I-Sulfatase Sulfamidase ist; oder
(BR) das Verfahren wie in einem der Ansprüche 1 oder 2 oder in einem von (AA) bis (BQ) definiert ist und die Zelle einen Mangel an OCH1-Aktivität umfasst; oder
(BS) das Verfahren wie in (AA) definiert ist und das Protein mit der Typ-I-Sulfatase-aktivierenden Aktivität eines FGE eines von (a) - (f) umfasst, und das reife Wildtyp-FGE-Polypeptid ein reifes Wildtyp-*Columba-livia-FGE-Polypeptid* ist und ferner ein Hefe-MNS1-Transmembran-Ankerpolypeptid umfasst; oder
(BT) das Verfahren wie in (BS) definiert ist und das Protein mit der Typ-I-Sulfatase-aktivierenden Aktivität eines FGE die in SEQ ID NO: 63 angegebene Aminosäuresequenz umfasst; oder
(BU) das Verfahren wie in (AA) definiert ist und es sich bei dem reifen Wildtyp-FGE-Polypeptid handelt um:
(i) ein reifes Wildtyp-FGE von *Hemicentrotus pulcherrimus* mit der in SEQ ID NO: 13 angegebenen Aminosäuresequenz, ein reifes Wildtyp-FGE von *Gallus gallus* mit der in SEQ ID NO: 47 angegebenen Aminosäuresequenz, ein reifes Wildtyp-FGE von *Dendroctonus ponderosa* mit der in SEQ ID NO: 49 angegebenen Aminosäuresequenz oder ein reifes Wildtyp-FGE von *Columba livia* mit der in SEQ ID NO: 51 angegebenen Aminosäuresequenz; oder
(ii) ein funktionelles reifes FGE mit einer Aminosäuresequenz, die mindestens 80% zu einer der Aminosäuresequenzen von (i) identisch ist.

4. Verfahren nach einem der Ansprüche 1-3, wobei das Verfahren zur Herstellung einer Typ-I-Sulfatase führt, wobei mehr als 10%, 20%, 30%, 40%, 50%, 60%, 70%, 80% oder 90% der Moleküle der Typ-I-Sulfatase einen Formylglycinrest im aktiven Zentrum umfassen.

5. Verfahren nach einem der Ansprüche 1-3, wobei das Verfahren zur Herstellung einer Typ-I-Sulfatase führt, wobei mehr als 95% der Moleküle der Typ-I-Sulfatase einen Formylglycinrest im aktiven Zentrum umfassen.

6. Verfahren nach einem der Ansprüche 1-3, wobei das Verfahren zur Herstellung einer Typ-I-Sulfatase führt, wobei 100% der Moleküle der Typ-I-Sulfatase einen Formylglycinrest im aktiven Zentrum umfassen.

7. Verfahren nach Anspruch 1, wobei das Protein mit der Typ-I-Sulfatase-aktivierenden Aktivität eines FGE durch eine Nukleotidsequenz codiert wird, umfassend
(i) die Nukleinsäuresequenz, die in einer der SEQ ID NOs: 14, 48, 50 oder 52 angegeben ist; oder
(ii) eine Nukleinsäuresequenz, die mindestens 80% zu einer der Nukleinsäuresequenzen von (i) identisch ist und ein reifes funktionelles FGE codiert; oder
(iii) eine Nukleinsäuresequenz, die unter hoher Stringenz an ein Komplement von einer der Nukleinsäuresequenzen von (i) hybridisiert und ein reifes funktionelles FGE codiert.

## Revendications

1. Procédé destiné à élaborer une sulfatase de type I sous une forme active,
(I) le procédé comprenant:
a) fournir une cellule de champignon génétiquement modifiée pour produire une protéine avec l'activité d'activation d'une sulfatase de type I d'une enzyme générant de la formyl-glycine (EGF) ; et
b) introduire dans la cellule un acide nucléique codant pour une sulfatase de type I, dans lequel la sulfatase de type I codée, sans étape d'activation, est sous une forme inactive,
dans lequel, après l'introduction, la cellule produit, ou produit à un taux accru, la sulfatase de type I sous une forme active et dans lequel, après l'étape (b), la cellule ou sa descendance est cultivée à une pO₂ de 5% - 40% ; ou
(II) le procédé comprenant:
a) fournir une cellule de champignon génétiquement modifiée pour produire une sulfatase de type I, dans lequel la sulfatase de type I, sans étape d'activation, est sous une forme inactive ; et
b) introduire dans la cellule un acide nucléique codant pour une protéine avec l'activité d'activation d'une sulfatase de type I d'une enzyme générant de la formyl-glycine (EGF),
dans lequel, après l'introduction, la cellule produit, ou produit à un taux accru, la sulfatase de type I sous une forme active et dans lequel, après l'étape (b), la cellule ou sa descendance est cultivée à une pO₂ de 5% - 40%.

2. Procédé de la revendication 1, dans lequel la cellule de champignon est une cellule de *Yarrowia lipolytica.*

3. Procédé de la revendication 1, dans lequel :
(AA) la protéine avec l'activité d'activation d'une sulfatase de type I d'une EGF comprend :
(a) un polypeptide d'EGF de type sauvage mature ;
(b) un fragment fonctionnel d'un polypeptide d'EGF de type sauvage mature comprenant au moins 50 acides aminés consécutifs de l'EGF de type sauvage mature ;
(c) un polypeptide avec une identité d'au moins 80% avec (a) ;
(d) un polypeptide avec une identité d'au moins 90% avec (b) ;
(e) (a) mais n'ayant pas plus de 10 substitutions conservatives ; ou
(f) (b) mais n'ayant pas plus de 5 substitutions conservatives ; ou
(AB) le procédé est tel que défini dans le paragraphe (AA) et le polypeptide d'EGF de type sauvage mature est la protéine SCO7548 de type sauvage mature ; ou
(AC) le procédé est tel que défini dans le paragraphe (AA) et le polypeptide d'EGF de type sauvage mature est la protéine Rv0712 de type sauvage mature ; ou
(AD) le procédé est tel que défini dans le paragraphe (AA) et le polypeptide d'EGF de type sauvage mature est le facteur 1 modifiant une sulfatase de type sauvage mature ; ou
(AE) le procédé est tel que défini dans le paragraphe (AA) et le polypeptide d'EGF de type sauvage mature est une enzyme générant de la formyl-glycine C-alpha de type sauvage mature ; ou
(AF) le procédé est tel que défini dans le paragraphe (AA) et le polypeptide d'EGF de type sauvage mature est le facteur 1 modifiant une sulfatase de type sauvage mature ; ou
(AG) le procédé est tel que défini dans l'une quelconque des revendications 1 ou 2 ou dans le paragraphe (AA) et la protéine avec l'activité d'activation d'une sulfatase de type I d'une EGF est une protéine procaryote avec l'activité d'activation d'une sulfatase de type I d'une EGF ; ou
(AH) le procédé est tel que défini dans le paragraphe (AG) et le procaryote est *Mycobacterium tuberculosis* ou *Streptomyces coelicolor* ; ou
(AI) le procédé est tel que défini dans l'une quelconque des revendications 1 ou 2 ou dans le paragraphe (AA) et la protéine avec l'activité d'activation d'une sulfatase de type I d'une EGF est une protéine avec l'activité d'activation d'une sulfatase de type I d'une EGF eucaryote ; ou
(AJ) le procédé est tel que défini dans le paragraphe (AI) et l'eucaryote est *Homo sapiens, Bos taurus, Hemicentrotus pulcherrimus, Tupaia chinensis, Monodelphis domestica, Gallus gallus, Dendroctonus ponderosa* ou *Columba livia* ; ou
(AK) le procédé est tel que défini dans l'une quelconque des revendications 1 ou 2 ou dans l'un quelconque des paragraphes (AA) à (AJ) et la protéine avec l'activité d'activation d'une sulfatase de type I d'une EGF comprend en outre un motif de ciblage du RE ; ou
(AL) le procédé est tel que défini dans le paragraphe (AK) et le motif de ciblage du RE est fusionné avec l'extrémité C-terminale de la protéine avec l'activité d'activation d'une sulfatase de type I d'un polypeptide d'EGF ; ou
(AM) le procédé est tel que défini dans le paragraphe (AK) et le motif de ciblage du RE est fusionné avec l'extrémité N-terminale de la protéine avec l'activité d'activation d'une sulfatase de type I d'un polypeptide d'EGF ; ou
(AN) le procédé est tel que défini dans l'un quelconque des paragraphes (AK) à (AM) et le motif de ciblage du RE comprend HDEL (SEQ ID NO: 1) ; ou
(AO) le procédé est tel que défini dans l'un quelconque des paragraphes (AK) à (AM) et le motif de ciblage du RE comprend KDEL (SEQ ID NO: 3) ; ou
(AP) le procédé est tel que défini dans l'un quelconque des paragraphes (AK) à (AM) et le motif de ciblage du RE comprend DDEL (SEQ ID NO: 4) ou RDEL (SEQ ID NO: 33) ; ou
(AQ) le procédé est tel que défini dans l'un quelconque des paragraphes (AK) à (AM) et le motif de ciblage du RE comprend un polypeptide d'ancrage transmembranaire MNS1 de levure ; ou
(AR) le procédé est tel que défini dans le paragraphe (AQ) et le polypeptide d'ancrage transmembranaire MNS1 de levure comprend le polypeptide d'ancrage transmembranaire MNS1 de *Yarrowia lipolytica* ; ou
(AS) le procédé est tel que défini dans l'un quelconque des paragraphes (AK) à (AM) et le motif de ciblage du RE comprend un polypeptide d'ancrage transmembranaire WBP1 de levure ; ou
(AT) le procédé est tel que défini dans le paragraphe (AS) et le polypeptide d'ancrage transmembranaire WBP1 de levure comprend le polypeptide d'ancrage transmembranaire WBP1 de *Yarrowia lipolytica* ; ou
(AU) le procédé est tel que défini dans l'une quelconque des revendications 1 ou 2 ou dans l'un quelconque des paragraphes (AA) à (AT) et la sulfatase de type I ou la protéine avec l'activité d'activation d'une sulfatase de type I d'une EGF comprend en outre une séquence de tête ou signal ; ou
(AV) le procédé est tel que défini dans le paragraphe (AU) et la séquence de tête ou signal est une séquence de tête ou signal exogène ; ou
(AW) le procédé est tel que défini dans le paragraphe (AU) et la séquence de tête ou signal est une séquence de tête ou signal endogène ; ou
(AX) le procédé est tel que défini dans l'un quelconque des paragraphes (AU) à (AW) et la séquence de tête ou signal est Lip2pre ; ou
(AY) le procédé est tel que défini dans l'une quelconque des revendications 1 ou 2 ou dans l'un quelconque des paragraphes (AA) à (AX) et le procédé comprend en outre l'étape d'introduction dans la cellule d'un acide nucléique codant pour un polypeptide capable d'effectuer une phosphorylation du mannosyl ou un fragment fonctionnel de celui-ci ; ou
(AW) le procédé est tel que défini dans le paragraphe (AY) et le polypeptide capable d'effectuer une phosphorylation du mannosyl est choisi dans le groupe constitué par les MNN4, PNO1 et MNN6 ; ou
(AZ) le procédé est tel que défini dans l'une quelconque des revendications 1 ou 2 ou dans l'un quelconque des paragraphes (AA) à (AW) et le procédé comprend en outre l'étape d'introduction dans la cellule d'un acide nucléique codant pour une mannosidase, ou un fragment fonctionnel de celle-ci, capable d'hydrolyser une fraction terminale de mannose-1-phospho-6-mannose en un phospho-6-mannose terminal ; ou
(BA) le procédé est tel que défini dans le paragraphe (AZ) et la mannosidase est la CcMan5 glycoside hydrolase de la famille 92 de *Cellulosimicrobium cellulans* ; ou
(BB) le procédé est tel que défini dans le paragraphe (AZ) et la mannosidase est également capable de retirer un résidu de mannose lié par une liaison alpha 1,2 au mannose sous-jacent dans la fraction terminale de mannose-1-phospho-6-mannose ; ou
(BC) le procédé est tel que défini dans le paragraphe (BB) et la mannosidase est une glycoside hydrolase de la famille 38 choisie dans le groupe constitué par une mannosidase de *Canavalia ensiformis* (Jack Bean) et la mannosidase AMS1 de *Yarrowia lipolytica* ; ou
(BD) le procédé est tel que défini dans le paragraphe (AZ) et le procédé comprend en outre l'étape d'introduction dans la cellule d'un acide nucléique codant pour une mannosidase, ou un fragment fonctionnel de celle-ci, qui capable de retirer un résidu de mannose lié par une liaison alpha 1,2 au mannose sous-jacent dans la fraction terminale de mannose-1-phospho-6-mannose ; ou
(BE) le procédé est tel que défini dans le paragraphe (BD) et la mannosidase est choisie dans le groupe constitué par la mannosidase glycoside hydrolase de la famille 38 de *Canavalia ensiformis* (Jack Bean), la mannosidase AMS1 glycoside hydrolase de la famille 38 de *Yarrowia lipolytica,* la mannosidase As glycoside hydrolase de la famille 47 *d'Aspergillus satoi* et la mannosidase CcMan4 glycoside hydrolase de la famille 92 de *Cellulosimicrobium cellulans* ; ou
(BF) le procédé est tel que défini dans l'une quelconque des revendications 1 ou 2 ou dans l'un quelconque des paragraphes (AA) à (BE) et le procédé comprend en outre l'étape d'introduction dans la cellule d'un acide nucléique codant pour une protéine de trafic, ou un fragment fonctionnel de celle-ci, dans lequel la protéine de trafic, ou un fragment fonctionnel de celle-ci, dirige la protéine avec l'activité d'activation d'une sulfatase de type I d'une EGF vers le réticulum endoplasmique (RE) de la cellule ; ou
(BG) le procédé est tel que défini dans le paragraphe (BF) et la protéine de trafic est une protéine disulfure isomérase (PDI) ; ou
(BH) le procédé est tel que défini dans le paragraphe (BF) et la protéine de trafic est la protéine 44 du réticulum endoplasmique (Erp44) ou la SUMF2 humaine ; ou
(BI) le procédé est tel que défini dans le paragraphe (BF) et la protéine de trafic, ou un fragment fonctionnel de celle-ci, se lie à la protéine avec l'activité d'activation d'une sulfatase de type I d'une EGF ; ou
(BJ) le procédé est tel que défini dans l'une quelconque des revendications 1 ou 2 ou dans l'un quelconque des paragraphes (AA) à (BI) et la cellule de champignon est une cellule de levure ; ou
(BK) le procédé est tel que défini dans le paragraphe (BJ) et la cellule de levure est une cellule d'une levure méthylotrophique ; ou
(BL) le procédé est tel que défini dans le paragraphe (BK) et la levure méthylotrophique est choisie dans le groupe constitué par *Pichia pastoris, Pichia methanolica, Ogataea minuta* et *Hansenula polymorpha* ; ou
(BM) le procédé est tel que défini dans l'une quelconque des revendications 1 ou 2 ou dans l'un quelconque des paragraphes (AA) à (BI) et la cellule de champignon est une cellule d'un champignon filamenteux ; ou
(BN) le procédé est tel que défini dans le paragraphe (BM) et le champignon filamenteux est choisi dans le groupe constitué par : *Aspergillus caesiellus, Aspergillus candidus, Aspergillus carneus, Aspergillus clavatus, Aspergillus deflectus, Aspergillus flavus, Aspergillus fumigatus, Aspergillus glaucus, Aspergillus nidulans, Aspergillus niger, Aspergillus ochraceus, Aspergillus oryzae, Aspergillus parasiticus, Aspergillus penicilloides, Aspergillus restrictus, Aspergillus sojae, Aspergillus sydowi, Aspergillus tamari, Aspergillus terreus, Aspergillus* ustus, *Aspergillus versicolor, Trichoderma* et *Neurospora* ; ou
(BO) le procédé est tel que défini dans l'une quelconque des revendications 1 ou 2 ou dans l'un quelconque des paragraphes (AA) à (BN) et la sulfatase de type I est une sulfatase de type I humaine ; ou
(BP) le procédé est tel que défini dans l'une quelconque des revendications 1 ou 2 ou dans l'un quelconque des paragraphes (AA) à (BN) et la sulfatase de type I est une iduronate sulfatase ; ou
(BQ) le procédé est tel que défini dans l'une quelconque des revendications 1 ou 2 ou dans l'un quelconque des paragraphes (AA) à (BN) et la sulfatase de type I est une sulfamidase ; ou
(BR) le procédé est tel que défini dans l'une quelconque des revendications 1 ou 2 ou dans l'un quelconque des paragraphes (AA) à (BQ) et la cellule comprend une déficience en activité d'OCH1 ; ou
(BS) le procédé est tel que défini dans le paragraphe (AA) et la protéine avec l'activité d'activation d'une sulfatase de type I d'une EGF comprend un quelconque élément de (a) à (f) et le polypeptide d'EGF de type sauvage mature est un polypeptide d'EGF de *Columba livia* de type sauvage mature et comprend en outre un polypeptide d'ancrage transmembranaire MNS1 de levure ; ou
(BT) le procédé est tel que défini dans le paragraphe (BS) et la protéine avec l'activité d'activation d'une sulfatase de type I d'une EGF comprend la séquence d'acides aminés indiquée dans la SEQ ID NO: 63 ; ou
(BU) le procédé est tel que défini dans le paragraphe (AA) et le polypeptide d'EGF de type sauvage mature est :
(i) une EGF de type sauvage mature d'*Hemicentrotus pulcherrimus* ayant la séquence d'acides aminés indiquée dans la SEQ ID NO: 13, une EGF de type sauvage mature de *Gallus gallus* ayant la séquence d'acides aminés indiquée dans la SEQ ID NO: 47, une EGF de type sauvage mature de *Dendroctonus ponderosa* ayant la séquence d'acides aminés indiquée dans la SEQ ID NO: 49 ou une EGF de type sauvage mature de *Columba livia* ayant la séquence d'acides aminés indiquée dans la SEQ ID NO: 51 ; ou
(ii) une EGF mature fonctionnelle ayant une séquence d'acides aminés qui est identique au moins à 80% à l'une quelconque des séquences d'acides aminés du paragraphe (i).

4. Procédé de l'une quelconque des revendications 1 à 3, dans lequel le procédé conduit à la production d'une sulfatase de type I dans laquelle plus de 10%, 20%, 30%, 40%, 50%, 60%, 70%, 80% ou 90% des molécules de la sulfatase de type I comprennent un résidu de formyl-glycine dans le site actif.

5. Procédé de l'une quelconque des revendications 1 à 3, dans lequel le procédé conduit à la production d'une sulfatase de type I dans laquelle plus de 95% des molécules de la sulfatase de type I comprennent un résidu de formyl-glycine dans le site actif.

6. Procédé de l'une quelconque des revendications 1 à 3, dans lequel le procédé conduit à la production d'une sulfatase de type I dans laquelle 100% des molécules de la sulfatase de type I comprennent un résidu de formyl-glycine dans le site actif.

7. Procédé de la revendication 1, dans lequel la protéine avec l'activité d'activation d'une sulfatase de type I d'une EGF est codée par une séquence nucléotidique comprenant
(i) la séquence d'acides nucléiques indiquée dans l'une quelconque des SEQ ID NOs: 14, 48, 50 ou 52 ; ou
(ii) une séquence d'acides nucléiques qui est identique au moins à 80% à l'une quelconque des séquences d'acides nucléiques du paragraphe (i) et qui code pour une EGF mature fonctionnelle ; ou
(iii) une séquence d'acides nucléiques qui s'hybride avec un complément de l'une quelconque des séquences d'acides nucléiques du paragraphe (i) sous une forte stringence et qui code pour une EGF mature fonctionnelle.


### SEQUENCE LISTING

<110> Oxyrane UK Ltd
<120> PRODUCTION OF CATALYTICALLY ACTIVE TYPE I SULFATASE
<130> P1363PC00
<150> 61/773,034
   <151> 2013-03-05
<150> 61/790,530
   <151> 2013-03-15
<160> 68
<170> PatentIn version 3.5
<210> 1
   <211> 4
   <212> PRT
   <213> Artificial Sequence
<220>
   <223> HDEL tag
<400> 1
   His Asp Glu Leu
   1
<210> 2
   <211> 12
   <212> DNA
   <213> Artificial Sequence
<220>
   <223> HDEL tag coding sequence
<400> 2
   cacgacgagc tg 12
<210> 3
   <211> 4
   <212> PRT
   <213> Artificial Sequence
<220>
   <223> KDEL tag
<400> 3
   Lys Asp Glu Leu
   1
<210> 4
   <211> 4
   <212> PRT
   <213> Artificial Sequence
<220>
   <223> DDEL tag
<400> 4
   Asp Asp Glu Leu
   1
<210> 5
   <211> 17
   <212> PRT
   <213> Artificial Sequence
<220>
   <223> Leader/Signal sequence
<400> 5 Ala
<210> 6
   <211> 51
   <212> DNA
   <213> Artificial Sequence
<220>
   <223> Coding sequence for Lip2 Leader/Signal Sequence
<400> 6
   atgaagctgt ctactattct ctttactgcc tgcgctactc tcgccgctgc t 51
<210> 7
   <211> 6
   <212> PRT
   <213> Artificial Sequence
<220>
   <223> His6 tag
<400> 7
   His His His His His His
   1 5
<210> 8
   <211> 18
   <212> DNA
   <213> Artificial Sequence
<220>
   <223> His6 tag coding sequence
<400> 8
   caccaccacc accaccac 18
<210> 9
   <211> 341
   <212> PRT
   <213> Homo sapiens mature FGE protein
<400> 9
   Ser Gln Glu Ala Gly Thr Gly Ala Gly Ala Gly Ser Leu Ala Gly Ser
   1               5                   10                  15
   Cys Gly Cys Gly Thr Pro Gln Arg Pro Gly Ala His Gly Ser Ser Ala
               20                  25                  30
   Ala Ala His Arg Tyr Ser Arg Glu Ala Asn Ala Pro Gly Pro Val Pro
           35                  40                  45
   Gly Glu Arg Gln Leu Ala His Ser Lys Met Val Pro Ile Pro Ala Gly
       50                  55                  60
   Val Phe Thr Met Gly Thr Asp Asp Pro Gln Ile Lys Gln Asp Gly Glu
   65                  70                  75                  80
   Ala Pro Ala Arg Arg Val Thr Ile Asp Ala Phe Tyr Met Asp Ala Tyr
                   85                  90                  95
   Glu Val Ser Asn Thr Glu Phe Glu Lys Phe Val Asn Ser Thr Gly Tyr
               100                 105                 110
   Leu Thr Glu Ala Glu Lys Phe Gly Asp Ser Phe Val Phe Glu Gly Met
           115                 120                 125
   Leu Ser Glu Gln Val Lys Thr Asn Ile Gln Gln Ala Val Ala Ala Ala
       130                 135                 140
   Pro Trp Trp Leu Pro Val Lys Gly Ala Asn Trp Arg His Pro Glu Gly
   145                 150                 155                 160
   Pro Asp Ser Thr Ile Leu His Arg Pro Asp His Pro Val Leu His Val
                   165                 170                 175
   Ser Trp Asn Asp Ala Val Ala Tyr Cys Thr Trp Ala Gly Lys Arg Leu
               180                 185                 190
   Pro Thr Glu Ala Glu Trp Glu Tyr Ser Cys Arg Gly Gly Leu His Asn
           195                 200                 205
   Arg Leu Phe Pro Trp Gly Asn Lys Leu Gln Pro Lys Gly Gln His Tyr
       210                 215                 220
   Ala Asn Ile Trp Gln Gly Glu Phe Pro Val Thr Asn Thr Gly Glu Asp
   225                 230                 235                 240
   Gly Phe Gln Gly Thr Ala Pro Val Asp Ala Phe Pro Pro Asn Gly Tyr
                   245                 250                 255
   Gly Leu Tyr Asn Ile Val Gly Asn Ala Trp Glu Trp Thr Ser Asp Trp
               260                 265                 270
   Trp Thr Val His His Ser Val Glu Glu Thr Leu Asn Pro Lys Gly Pro
           275                 280                 285
   Pro Ser Gly Lys Asp Arg Val Lys Lys Gly Gly Ser Tyr Met Cys His
       290                 295                 300
   Arg Ser Tyr Cys Tyr Arg Tyr Arg Cys Ala Ala Arg Ser Gln Asn Thr
   305                 310                 315                 320
   Pro Asp Ser Ser Ala Ser Asn Leu Gly Phe Arg Cys Ala Ala Asp Arg
                   325                 330                 335
   Leu Pro Thr Met Asp
               340
<210> 10
   <211> 1023
   <212> DNA
   <213> Homo sapiens coding sequence of mature FGE protein
<400> 10
   tcccaggaag ccggcaccgg agctggtgct ggttctctgg ctggatcgtg cggatgtggc      60
   actcctcagc gacctggagc tcatggctcc tctgccgctg cccaccgata ctctcgagag     120
   gctaacgctc ctggtcctgt ccccggagag cgacagctcg cccattctaa gatggtgcct     180
   atccccgctg gagttttcac catgggcact gacgatcctc agatcaagca ggacggagag     240
   gctcctgctc gacgagtgac cattgacgcc ttttacatgg atgcttacga ggtttcgaac     300
   actgagttcg agaagtttgt caactctacc ggatacctga ctgaggccga gaagttcggt     360
   gactcgttcg tgtttgaggg aatgctctcc gagcaggtca agaccaacat ccagcaggct     420
   gtggctgccg ctccttggtg gctgcccgtt aagggagcta actggcgaca ccctgaggga     480
   cctgactcca ccattctgca ccgacctgat catcccgtcc tccacgtgtc ttggaacgac     540
   gccgttgctt actgtacctg ggctggcaag cgactgccta ctgaggctga gtgggagtac     600
   tcctgccgag gcggtctgca taaccgactc ttcccttggg gcaacaagct ccagcccaag     660
   ggtcagcact acgccaacat ctggcagggc gagtttcctg tgaccaacac tggagaggac     720
   ggattccagg gcaccgctcc tgttgatgct tttcccccta acggttacgg actgtacaac      780
   attgtcggta acgcttggga gtggacctct gactggtgga ctgttcacca ttcggtcgag     840
   gagaccctca accccaaggg ccctccctct ggcaaggatc gagtcaagaa gggaggctcc     900
   tacatgtgcc accgatctta ctgttaccga taccgatgcg ccgctcgatc ccagaacacc     960
   cccgactcgt ccgcctctaa cctgggcttc cgatgtgccg ctgaccgact gcctactatg    1020
   gac                                                                  1023
<210> 11
   <211> 314
   <212> PRT
   <213> Streptomyces coelicolor FGE mature protein
<400> 11
   Met Ala Val Ala Ala Pro Ser Pro Ala Ala Ala Ala Glu Pro Gly Pro
   1               5                   10                  15
   Ala Ala Arg Pro Arg Ser Thr Arg Gly Gln Val Arg Leu Pro Gly Gly
               20                  25                  30
   Glu Phe Ala Met Gly Asp Ala Phe Gly Glu Gly Tyr Pro Ala Asp Gly
           35                  40                  45
   Glu Thr Pro Val His Thr Val Arg Leu Arg Pro Phe His Ile Asp Glu
       50                  55                  60
   Thr Ala Val Thr Asn Ala Arg Phe Ala Ala Phe Val Lys Ala Thr Gly
   65                  70                  75                  80
   His Val Thr Asp Ala Glu Arg Phe Gly Ser Ser Ala Val Phe His Leu
                   85                  90                  95
   Val Val Ala Ala Pro Asp Ala Asp Val Leu Gly Ser Ala Ala Gly Ala
               100                 105                 110
   Pro Trp Trp Ile Asn Val Arg Gly Ala His Trp Arg Arg Pro Glu Gly
           115                 120                 125
   Ala Arg Ser Asp Ile Thr Gly Arg Pro Asn His Pro Val Val His Val
       130                 135                 140
   Ser Trp Asn Asp Ala Thr Ala Tyr Ala Arg Trp Ala Gly Lys Arg Leu
   145                 150                 155                 160
   Pro Thr Glu Ala Glu Trp Glu Tyr Ala Ala Arg Gly Gly Leu Ala Gly
                   165                 170                 175
   Arg Arg Tyr Ala Trp Gly Asp Glu Leu Thr Pro Gly Gly Arg Trp Arg
   180 185 190
   Cys Asn Ile Trp Gln Gly Arg Phe Pro His Val Asn Thr Ala Glu Asp
   195 200 205
   Gly His Leu Ser Thr Ala Pro Val Lys Ser Tyr Arg Pro Asn Gly His
   210 215 220
   Gly Leu Trp Asn Thr Ala Gly Asn Val Trp Glu Trp Cys Ser Asp Trp
   225 230 235 240
   Phe Ser Pro Thr Tyr Tyr Ala Glu Ser Pro Thr Val Asp Pro His Gly
   245 250 255
   Pro Gly Thr Gly Ala Ala Arg Val Leu Arg Gly Gly Ser Tyr Leu Cys
   260 265 270
   His Asp Ser Tyr Cys Asn Arg Tyr Arg Val Ala Ala Arg Ser Ser Asn
   275 280 285
   Thr Pro Asp Ser Ser Ser Gly Asn Leu Gly Phe Arg Cys Ala Asn Asp
   290 295 300
   Ala Asp Leu Thr Ser Gly Ser Ala Ala Glu
   305 310
<210> 12
   <211> 942
   <212> DNA
   <213> Streptomyces coelicolor FGE protein coding sequence
<400> 12
   atggctgttg ctgctccctc gcctgctgct gctgccgagc ccggtcctgc tgctcgaccc 60
   cgatctaccc gaggacaggt gcgactgcct ggcggtgagt tcgctatggg cgacgctttt 120
   ggagagggat accctgccga tggagagacc cctgtgcaca ctgttcgact ccgacccttc 180
   catatcgacg agaccgctgt tactaacgcc cgattcgccg cttttgtcaa ggctaccgga 240
   cacgtgactg atgccgagcg attcggctcc tctgctgttt ttcatctggt cgtggccgct 300
   cccgacgctg atgtcctggg ctccgctgct ggagctcctt ggtggatcaa cgttcgaggt 360
   gcccactggc gacgacctga gggagctcga tctgacatta ccggtcgacc caaccaccct 420
   gttgtccatg tctcctggaa cgatgctacc gcttacgctc gatgggctgg aaagcgactg 480
   cctactgagg ctgagtggga gtacgctgct cgaggcggcc tggctggtcg acgatacgct 540
   tggggagacg agctcacccc cggtggacga tggcgatgca acatttggca gggacgattc 600
   cctcacgtca acaccgccga ggacggccat ctgtccactg ctcccgtgaa gtcttaccga 660
   cctaacggtc acggactctg gaacaccgcc ggtaacgtct gggagtggtg ttctgactgg 720
   ttttcgccca cctactacgc cgagtctcct actgtcgacc cccacggacc tggtactgga 780
   gctgctcgag ttctgcgagg cggttcgtac ctctgccatg actcctactg taaccgatac 840
   cgagtggccg ctcgatcgtc caacaccccc gactcttcgt ccggcaacct cggtttccga 900
   tgcgccaacg atgctgacct gacttctgga tctgccgctg ag 942
<210> 13
   <211> 402
   <212> PRT
   <213> Hemicentrotus pulcherrimus FGE mature protein
<400> 13
   Glu Asn Glu Asp Ile Asn Gln Asn Ile Ser Pro Thr Gln Ser His Thr
   1               5                   10                  15
   Thr Ala Thr Thr Glu Glu Glu Leu Ala Glu Ala Arg Gly Glu Glu Ile
               20                  25                  30
   Asp Ser Asp Pro Thr Ser Glu Gly Ser Gly Ala Gly Glu Gly Cys Gly
           35                  40                  45
   Cys Gly Ser Ser Ala Leu Asn Arg Asn His Asp Glu Asp Ala Leu Gly
       50                  55                  60
   Leu Ala Leu Glu Glu Asn Leu His Asp His Val Gln Glu Gly Ala Ala
   65                  70                  75                  80
   Leu Lys Tyr Ser Arg Glu Ala Asn Asp Pro Ile Ser Met Asp His Pro
                   85                  90                  95
   Glu Ala Asn Val Gly Ala Phe Pro Arg Thr Asn Gln Met Asn Phe Ile
               100                 105                 110
   Glu Gly Gly Thr Phe Arg Met Gly Thr Asp Lys Ala Lys Ile Tyr Leu
           115                 120                 125
   Asp Gly Glu Ser Pro Ser Arg Leu Val Thr Leu Asp Pro Tyr Tyr Phe
       130                 135                 140
   Asp Val Tyr Glu Val Ser Asn Ser Glu Phe Glu Leu Phe Val Asn Thr
   145                 150                 155                 160
   Thr Ser Tyr Ile Thr Glu Ala Glu Lys Phe Gly Asp Ser Phe Val Leu
                   165                 170                 175
   Glu Ala Arg Ile Ser Glu Glu Val Lys Lys Asp Ile Ser Gln Val Val
   180 185 190
   Ala Ala Ala Pro Trp Trp Leu Pro Val Lys Gly Ala Glu Trp Arg His
   195 200 205
   Pro Glu Gly Pro Asp Ser Ser Ile Ser Ser Arg Met Asp His Pro Val
   210 215 220
   Thr His Ile Ser Trp Asn Asp Ala Thr Ala Tyr Cys Gln Trp Ala Gly
   225 230 235 240
   Lys Arg Leu Pro Thr Glu Ala Glu Trp Glu Asn Ala Ala Arg Gly Gly
   245 250 255
   Leu Asn Asn Arg Leu Phe Pro Trp Gly Asn Lys Leu Met Pro Lys Asp
   260 265 270
   His His Arg Val Asn Ile Trp Gln Gly Glu Phe Pro Lys Val Asn Thr
   275 280 285
   Ala Glu Asp Gly Tyr Glu Gly Thr Cys Pro Val Thr Ala Phe Glu Pro
   290 295 300
   Asn Gly Tyr Gly Leu Tyr Asn Thr Val Gly Asn Ala Trp Glu Trp Val
   305 310 315 320
   Ala Asp Trp Trp Thr Thr Val His Ser Pro Glu Ser Gln Asn Asn Pro
   325 330 335
   Val Gly Pro Asp Glu Gly Thr Asp Lys Val Lys Lys Gly Gly Ser Tyr
   340 345 350
   Met Cys His Ile Ser Tyr Cys Tyr Arg Tyr Arg Cys Glu Ala Arg Ser
   355 360 365
   Gln Asn Ser Pro Asp Ser Ser Ala Cys Asn Leu Gly Phe Arg Cys Ala
   370 375 380
   Ala Thr Asn Leu Pro Glu Asp Ile Pro Cys Ser Asn Cys Asn Asp Ser
   385 390 395 400
   Thr Pro
<210> 14
   <211> 1206
   <212> DNA
   <213> Hemicentrotus pulcherrimus FGE protein coding sequence
<400> 14
   gagaacgagg acatcaacca gaacatttcg cctacccagt ctcacaccac tgccaccact      60
   gaggaagagc tcgctgaggc ccgaggcgag gagatcgact ccgatcccac ctctgagggc     120
   tctggtgctg gagagggatg cggttgtggc tcctctgccc tgaaccgaaa ccacgacgag     180
   gatgctctgg gtctcgccct ggaggagaac ctccacgacc atgttcagga aggcgccgct     240
   ctgaagtact cgcgagaggc taacgacccc atttctatgg atcatcctga ggctaacgtc     300
   ggtgccttcc cccgaaccaa ccagatgaac ttcatcgagg gcggtacctt tcgaatggga     360
   actgacaagg ccaagatcta cctggatggt gaatctcctt cccgactggt gaccctggac     420
   ccttactact ttgatgttta cgaggtctct aactcggagt tcgagctctt tgttaacacc     480
   acttcttaca tcaccgaggc tgagaagttc ggtgactcct ttgtgctgga ggcccgaatc     540
   tctgaggaag tcaagaagga tatttctcag gtggtggctg ctgctccttg gtggctcccc     600
   gtcaagggtg ctgagtggcg acaccctgag ggtcctgact cgtccatctc ttcgcgaatg     660
   gatcaccccg tgacccatat ttcctggaac gacgctactg cctactgtca gtgggctgga     720
   aagcgactcc ctaccgaggc tgagtgggag aacgctgctc gaggcggcct caacaaccga     780
   ctgttcccct ggggcaacaa gctgatgcct aaggaccacc atcgagttaa catttggcag     840
   ggagagttcc ccaaggtcaa caccgctgag gacggatacg agggcacctg ccccgtgact     900
   gcctttgagc ctaacggcta cggtctgtac aacactgtgg gaaacgcttg ggagtgggtt     960
   gccgactggt ggaccactgt ccactcgccc gagtcccaga acaaccccgt cggtcctgac    1020
   gagggaaccg ataaggtcaa gaagggcggc tcctacatgt gccatatctc ttactgttac    1080
   cgataccgat gcgaggctcg atctcagaac tcgcccgact cctctgcctg taacctcggc    1140
   ttccgatgcg ctgccaccaa cctgcctgag gacattcctt gttctaactg taacgattcc    1200
   actccc                                                                1206
<210> 15
   <211> 351
   <212> PRT
   <213> Bos Taurus FGE coding sequence mature sequence
<400> 15
   Ala Gly Gly Glu Glu Ala Gly Pro Glu Ala Gly Ala Pro Ser Leu Val
   1               5                   10                  15
   Gly Ser Cys Gly Cys Gly Asn Pro Gln Arg Pro Gly Ala Gln Gly Ser
               20                  25                  30
   Ser Ala Ala Ala His Arg Tyr Ser Arg Glu Ala Asn Ala Pro Gly Ser
   35 40 45
   Val Pro Gly Gly Arg Pro Ser Pro Pro Thr Lys Met Val Pro Ile Pro
   50 55 60
   Ala Gly Val Phe Thr Met Gly Thr Asp Asp Pro Gln Ile Lys Gln Asp
   65 70 75 80
   Gly Glu Ala Pro Ala Arg Arg Val Ala Ile Asp Ala Phe Tyr Met Asp
   85 90 95
   Ala Tyr Glu Val Ser Asn Ala Glu Phe Glu Lys Phe Val Asn Ser Thr
   100 105 110
   Gly Tyr Leu Thr Glu Ala Glu Lys Phe Gly Asp Ser Phe Val Phe Glu
   115 120 125
   Gly Met Leu Ser Glu Gln Val Lys Ser Asp Ile Gln Gln Ala Val Ala
   130 135 140
   Ala Ala Pro Trp Trp Leu Pro Val Lys Gly Ala Asn Trp Arg His Pro
   145 150 155 160
   Glu Gly Pro Asp Ser Thr Val Leu His Arg Pro Asp His Pro Val Leu
   165 170 175
   His Val Ser Trp Asn Asp Ala Val Ala Tyr Cys Thr Trp Ala Gly Lys
   180 185 190
   Arg Leu Pro Thr Glu Ala Glu Trp Glu Tyr Ser Cys Arg Gly Gly Leu
   195 200 205
   Gln Asn Arg Leu Phe Pro Trp Gly Asn Lys Leu Gln Pro Lys Gly Gln
   210 215 220
   His Tyr Ala Asn Ile Trp Gln Gly Glu Phe Pro Val Thr Asn Thr Gly
   225 230 235 240
   Glu Asp Gly Phe Arg Gly Thr Ala Pro Val Asp Ala Phe Pro Pro Asn
   245 250 255
   Gly Tyr Gly Leu Tyr Asn Ile Val Gly Asn Ala Trp Glu Trp Thr Ser
   260 265 270
   Asp Trp Trp Thr Val His His Ser Ala Glu Glu Thr Ile Asn Pro Lys
   275 280 285
   Gly Pro Pro Ser Gly Lys Asp Arg Val Lys Lys Gly Gly Ser Tyr Met
       290                 295                 300
   Cys His Lys Ser Tyr Cys Tyr Arg Tyr Arg Cys Ala Ala Arg Ser Gln
   305                 310                 315                 320
   Asn Thr Pro Asp Ser Ser Ala Ser Asn Leu Gly Phe Arg Cys Ala Ala
                   325                 330                 335
   Asp His Leu Pro Thr Thr Gly Ala Asp His Leu Pro Thr Thr Gly
               340                 345                 350
<210> 16
   <211> 1029
   <212> DNA
   <213> Bos Taurus FGE protein coding sequence
<400> 16
   gccggcggcg aggaagccgg acctgaggcc ggcgctccct ctctggttgg atcgtgtgga      60
   tgtggaaacc cccagcgacc tggcgctcag ggttcctctg ccgctgccca ccgatactct     120
   cgagaggcta acgctcctgg ctctgtccct ggaggccgac cctcgccccc taccaagatg     180
   gttcccatcc ctgccggcgt cttcaccatg ggtactgacg atcctcagat caagcaggac     240
   ggagaggctc ctgctcgacg agtggctatt gacgcttttt acatggatgc ctacgaggtc     300
   tctaacgctg agttcgagaa gtttgtgaac tcgaccggat acctgactga ggccgagaag     360
   ttcggagact ccttcgtttt tgagggcatg ctctccgagc aggtgaagtc tgatattcag     420
   caggctgttg ctgccgctcc ttggtggctg cctgtcaagg gagctaactg gcgacatccc     480
   gagggtcctg actccaccgt gctgcaccga cccgatcatc ctgtcctcca cgtgtcttgg     540
   aacgacgccg tcgcttactg tacctgggct ggcaagcgac tgcctactga ggctgagtgg     600
   gagtactctt gccgaggtgg actgcagaac cgactcttcc cttggggtaa caagctccag     660
   cccaagggac agcactacgc caacatctgg cagggagagt ttcctgtgac caacactggt     720
   gaagacggct tccgaggcac cgctcctgtt gatgcttttc cccctaacgg ttacggactc     780
   tacaacatcg ttggcaacgc ctgggagtgg acctccgact ggtggactgt ccaccattct     840
   gctgaggaga ctattaaccc caagggtccc ccttctggaa aggatcgagt gaagaagggc     900
   ggttcgtaca tgtgccacaa gtcctactgt taccgatacc gatgcgccgc tcgatcgcag     960
   aacacccccg actcgtccgc ctccaacctg ggattccgat gtgccgctga ccacctgcct    1020
   actactgga                                                            1029
<210> 17
   <211> 299
   <212> PRT
   <213> Mycobacterium tuberculosis FGE mature sequence
<400> 17
   Met Leu Thr Glu Leu Val Asp Leu Pro Gly Gly Ser Phe Arg Met Gly
   1               5                   10                  15
   Ser Thr Arg Phe Tyr Pro Glu Glu Ala Pro Ile His Thr Val Thr Val
               20                  25                  30
   Arg Ala Phe Ala Val Glu Arg His Pro Val Thr Asn Ala Gln Phe Ala
           35                  40                  45
   Glu Phe Val Ser Ala Thr Gly Tyr Val Thr Val Ala Glu Gln Pro Leu
       50                  55                  60
   Asp Pro Gly Leu Tyr Pro Gly Val Asp Ala Ala Asp Leu Cys Pro Gly
   65                  70                  75                  80
   Ala Met Val Phe Cys Pro Thr Ala Gly Pro Val Asp Leu Arg Asp Trp
                   85                  90                  95
   Arg Gln Trp Trp Asp Trp Val Pro Gly Ala Cys Trp Arg His Pro Phe
               100                 105                 110
   Gly Arg Asp Ser Asp Ile Ala Asp Arg Ala Gly His Pro Val Val Gln
           115                 120                 125
   Val Ala Tyr Pro Asp Ala Val Ala Tyr Ala Arg Trp Ala Gly Arg Arg
       130                 135                 140
   Leu Pro Thr Glu Ala Glu Trp Glu Tyr Ala Ala Arg Gly Gly Thr Thr
   145                 150                 155                 160
   Ala Thr Tyr Ala Trp Gly Asp Gln Glu Lys Pro Gly Gly Met Leu Met
                   165                 170                 175
   Ala Asn Thr Trp Gln Gly Arg Phe Pro Tyr Arg Asn Asp Gly Ala Leu
               180                 185                 190
   Gly Trp Val Gly Thr Ser Pro Val Gly Arg Phe Pro Ala Asn Gly Phe
           195                 200                 205
   Gly Leu Leu Asp Met Ile Gly Asn Val Trp Glu Trp Thr Thr Thr Glu
       210                 215                 220
   Phe Tyr Pro His His Arg Ile Asp Pro Pro Ser Thr Ala Cys Cys Ala
   225                 230                 235                 240
   Pro Val Lys Leu Ala Thr Ala Ala Asp Pro Thr Ile Ser Gln Thr Leu
                   245                 250                 255
   Lys Gly Gly Ser His Leu Cys Ala Pro Glu Tyr Cys His Arg Tyr Arg
               260                 265                 270
   Pro Ala Ala Arg Ser Pro Gln Ser Gln Asp Thr Ala Thr Thr His Ile
           275                 280                 285
   Gly Phe Arg Cys Val Ala Asp Pro Val Ser Gly
       290                 295
<210> 18
   <211> 897
   <212> DNA
   <213> Mycobacterium tuberculosis FGE protein coding sequence
<400> 18
   atgctgactg agctggttga cctccctggt ggttccttcc gaatgggatc tacccgattt      60
   taccccgagg aggcccctat ccacactgtt accgtccgag ccttcgctgt cgagcgacat     120
   cccgtgacca acgctcagtt cgccgagttt gtttcggcta ctggctacgt gaccgttgct     180
   gagcagcctc tggaccctgg actctaccct ggagtcgacg ctgctgatct gtgccctggc     240
   gctatggtct tctgtcctac cgctggtcct gtggacctcc gagattggcg acagtggtgg     300
   gactgggtcc ctggtgcttg ctggcgacac ccttttggac gagactccga tattgctgac     360
   cgagctggac atcctgtcgt gcaggtggct taccctgatg ccgttgctta cgctcgatgg     420
   gctggtcgac gactgcctac tgaggctgag tgggagtacg ctgctcgagg aggtaccact     480
   gctacctacg cttggggtga ccaggagaag cctggaggca tgctgatggc taacacctgg     540
   cagggacgat tcccttaccg aaacgatgga gccctcggct gggttggtac ctcccctgtc     600
   ggacgattcc ctgctaacgg ctttggtctg ctcgacatga tcggcaacgt gtgggagtgg     660
   accactaccg agttttaccc ccaccatcga attgaccccc cttctactgc ttgctgtgct     720
   cctgttaagc tcgctaccgc tgctgatcct actatctcgc agaccctgaa gggtggctcc     780
   cacctctgcg ctcccgagta ctgtcatcga taccgacccg ccgctcgatc ccctcagtct     840
   caggacaccg ccactaccca cattggtttt cgatgtgttg ctgaccctgt ttcgggc        897
<210> 19
   <211> 525
   <212> PRT
   <213> human iduronate sulfatase mature sequence
<400> 19
   Ser Glu Thr Gln Ala Asn Ser Thr Thr Asp Ala Leu Asn Val Leu Leu
   1               5                   10                  15
   Ile Ile Val Asp Asp Leu Arg Pro Ser Leu Gly Cys Tyr Gly Asp Lys
               20                  25                  30
   Leu Val Arg Ser Pro Asn Ile Asp Gln Leu Ala Ser His Ser Leu Leu
           35                  40                  45
   Phe Gln Asn Ala Phe Ala Gln Gln Ala Val Cys Ala Pro Ser Arg Val
       50                  55                  60
   Ser Phe Leu Thr Gly Arg Arg Pro Asp Thr Thr Arg Leu Tyr Asp Phe
   65                  70                  75                  80
   Asn Ser Tyr Trp Arg Val His Ala Gly Asn Phe Ser Thr Ile Pro Gln
                   85                  90                  95
   Tyr Phe Lys Glu Asn Gly Tyr Val Thr Met Ser Val Gly Lys Val Phe
               100                 105                 110
   His Pro Gly Ile Ser Ser Asn His Thr Asp Asp Ser Pro Tyr Ser Trp
           115                 120                 125
   Ser Phe Pro Pro Tyr His Pro Ser Ser Glu Lys Tyr Glu Asn Thr Lys
       130                 135                 140
   Thr Cys Arg Gly Pro Asp Gly Glu Leu His Ala Asn Leu Leu Cys Pro
   145                 150                 155                 160
   Val Asp Val Leu Asp Val Pro Glu Gly Thr Leu Pro Asp Lys Gln Ser
                   165                 170                 175
   Thr Glu Gln Ala Ile Gln Leu Leu Glu Lys Met Lys Thr Ser Ala Ser
               180                 185                 190
   Pro Phe Phe Leu Ala Val Gly Tyr His Lys Pro His Ile Pro Phe Arg
           195                 200                 205
   Tyr Pro Lys Glu Phe Gln Lys Leu Tyr Pro Leu Glu Asn Ile Thr Leu
       210                 215                 220
   Ala Pro Asp Pro Glu Val Pro Asp Gly Leu Pro Pro Val Ala Tyr Asn
   225                 230                 235                 240
   Pro Trp Met Asp Ile Arg Gln Arg Glu Asp Val Gln Ala Leu Asn Ile
                   245                 250                 255
   Ser Val Pro Tyr Gly Pro Ile Pro Val Asp Phe Gln Arg Lys Ile Arg
   260 265 270
   Gln Ser Tyr Phe Ala Ser Val Ser Tyr Leu Asp Thr Gln Val Gly Arg
   275 280 285
   Leu Leu Ser Ala Leu Asp Asp Leu Gln Leu Ala Asn Ser Thr Ile Ile
   290 295 300
   Ala Phe Thr Ser Asp His Gly Trp Ala Leu Gly Glu His Gly Glu Trp
   305 310 315 320
   Ala Lys Tyr Ser Asn Phe Asp Val Ala Thr His Val Pro Leu Ile Phe
   325 330 335
   Tyr Val Pro Gly Arg Thr Ala Ser Leu Pro Glu Ala Gly Glu Lys Leu
   340 345 350
   Phe Pro Tyr Leu Asp Pro Phe Asp Ser Ala Ser Gln Leu Met Glu Pro
   355 360 365
   Gly Arg Gln Ser Met Asp Leu Val Glu Leu Val Ser Leu Phe Pro Thr
   370 375 380
   Leu Ala Gly Leu Ala Gly Leu Gln Val Pro Pro Arg Cys Pro Val Pro
   385 390 395 400
   Ser Phe His Val Glu Leu Cys Arg Glu Gly Lys Asn Leu Leu Lys His
   405 410 415
   Phe Arg Phe Arg Asp Leu Glu Glu Asp Pro Tyr Leu Pro Gly Asn Pro
   420 425 430
   Arg Glu Leu Ile Ala Tyr Ser Gln Tyr Pro Arg Pro Ser Asp Ile Pro
   435 440 445
   Gln Trp Asn Ser Asp Lys Pro Ser Leu Lys Asp Ile Lys Ile Met Gly
   450 455 460
   Tyr Ser Ile Arg Thr Ile Asp Tyr Arg Tyr Thr Val Trp Val Gly Phe
   465 470 475 480
   Asn Pro Asp Glu Phe Leu Ala Asn Phe Ser Asp Ile His Ala Gly Glu
   485 490 495
   Leu Tyr Phe Val Asp Ser Asp Pro Leu Gln Asp His Asn Met Tyr Asn
   500 505 510
   Asp Ser Gln Gly Gly Asp Leu Phe Gln Leu Leu Met Pro
   515 520 525
<210> 20
   <211> 1575
   <212> DNA
   <213> human iduronate sulfatase protein coding sequence
<400> 20
   tctgagaccc aggctaactc gactactgac gctctgaacg tgctcctgat tattgttgac      60
   gacctgcgac cctccctcgg ttgctacggt gacaagctgg tgcgatctcc caacatcgac     120
   cagctcgctt ctcactcgct gctcttccag aacgcctttg ctcagcaggc cgtctgcgct     180
   ccttcgcgag tgtccttcct gaccggacga cgacccgaca ccactcgact ctacgatttt     240
   aactcctact ggcgagtcca cgccggtaac ttctctacca tccctcagta ctttaaggag     300
   aacggatacg tgactatgtc cgtgggcaag gttttccacc ccggtatttc ctctaaccat     360
   accgacgatt ctccttactc ctggtctttt cccccttacc acccctcgtc cgagaagtac     420
   gagaacacca agacttgccg aggccctgac ggagagctgc atgctaacct gctctgtccc     480
   gtcgacgtgc tggatgttcc tgagggaacc ctccccgata agcagtccac tgagcaggcc     540
   attcagctgc tcgagaagat gaagacctcg gcctccccct tctttctggc tgtcggctac     600
   cacaagcccc atatcccttt ccgataccct aaggagtttc agaagctgta ccccctcgag     660
   aacattaccc tggctcccga ccctgaggtt cctgatggtc tgcctcccgt ggcttacaac     720
   ccttggatgg acatccgaca gcgagaggat gtgcaggccc tgaacatctc cgttccctac     780
   ggtcccattc ctgtcgactt ccagcgaaag attcgacagt cttactttgc ttctgtgtcg     840
   tacctggaca cccaggttgg tcgactgctc tccgccctcg acgatctgca gctcgccaac     900
   tcgaccatca ttgctttcac ttccgaccac ggatgggccc tgggagagca tggcgagtgg     960
   gctaagtact ctaacttcga cgttgccacc cacgtccctc tgatctttta cgttcctgga    1020
   cgaactgcct ccctccctga ggctggtgaa aagctgttcc cttacctcga cccctttgat    1080
   tccgcttctc agctgatgga gcctggccga cagtctatgg acctggtcga gctcgtgtcg    1140
   ctgttcccca ccctggctgg tctggctggc ctgcaggtcc ctccccgatg ccccgtgcct    1200
   tctttccacg ttgagctctg tcgagaggga aagaacctgc tcaagcattt ccgatttcga    1260
   gacctggagg aagaccccta cctccctggc aacccccgag agctgatcgc ctactcccag    1320
   tacccccgac cttctgacat tcctcagtgg aactctgaca agccctcgct caaggatatc    1380
   aagattatgg gctactccat ccgaaccatt gactaccgat acactgtttg ggtcggtttc    1440
   aaccccgacg agttcctggc caacttttcg gatattcacg ctggagagct gtacttcgtc    1500
   gactctgatc cctccaggac ccataacatg tacaacgact cgcagggcgg tgacctcttc    1560
   cagctcctga tgcct                                                    1575
<210> 21
   <211> 487
   <212> PRT
   <213> human PDI mature sequence
<400> 21
   Asp Ala Pro Glu Glu Glu Asp His Val Leu Val Leu Arg Lys Ser Asn
   1               5                   10                  15
   Phe Ala Glu Ala Leu Ala Ala His Lys Tyr Leu Leu Val Glu Phe Tyr
               20                  25                  30
   Ala Pro Trp Cys Gly His Cys Lys Ala Leu Ala Pro Glu Tyr Ala Lys
           35                  40                  45
   Ala Ala Gly Lys Leu Lys Ala Glu Gly Ser Glu Ile Arg Leu Ala Lys
       50                  55                  60
   Val Asp Ala Thr Glu Glu Ser Asp Leu Ala Gln Gln Tyr Gly Val Arg
   65                  70                  75                  80
   Gly Tyr Pro Thr Ile Lys Phe Phe Arg Asn Gly Asp Thr Ala Ser Pro
                   85                  90                  95
   Lys Glu Tyr Thr Ala Gly Arg Glu Ala Asp Asp Ile Val Asn Trp Leu
               100                 105                 110
   Lys Lys Arg Thr Gly Pro Ala Ala Thr Thr Leu Pro Asp Gly Ala Ala
           115                 120                 125
   Ala Glu Ser Leu Val Glu Ser Ser Glu Val Ala Val Ile Gly Phe Phe
       130                 135                 140
   Lys Asp Val Glu Ser Asp Ser Ala Lys Gln Phe Leu Gln Ala Ala Glu
   145                 150                 155                 160
   Ala Ile Asp Asp Ile Pro Phe Gly Ile Thr Ser Asn Ser Asp Val Phe
                   165                 170                 175
   Ser Lys Tyr Gln Leu Asp Lys Asp Gly Val Val Leu Phe Lys Lys Phe
               180                 185                 190
   Asp Glu Gly Arg Asn Asn Phe Glu Gly Glu Val Thr Lys Glu Asn Leu
           195                 200                 205
   Leu Asp Phe Ile Lys His Asn Gln Leu Pro Leu Val Ile Glu Phe Thr
       210                 215                 220
   Glu Gln Thr Ala Pro Lys Ile Phe Gly Gly Glu Ile Lys Thr His Ile
   225                 230                 235                 240
   Leu Leu Phe Leu Pro Lys Ser Val Ser Asp Tyr Asp Gly Lys Leu Ser
                   245                 250                 255
   Asn Phe Lys Thr Ala Ala Glu Ser Phe Lys Gly Lys Ile Leu Phe Ile
               260                 265                 270
   Phe Ile Asp Ser Asp His Thr Asp Asn Gln Arg Ile Leu Glu Phe Phe
           275                 280                 285
   Gly Leu Lys Lys Glu Glu Cys Pro Ala Val Arg Leu Ile Thr Leu Glu
       290                 295                 300
   Glu Glu Met Thr Lys Tyr Lys Pro Glu Ser Glu Glu Leu Thr Ala Glu
   305                 310                 315                 320
   Arg Ile Thr Glu Phe Cys His Arg Phe Leu Glu Gly Lys Ile Lys Pro
                   325                 330                 335
   His Leu Met Ser Gln Glu Leu Pro Glu Asp Trp Asp Lys Gln Pro Val
               340                 345                 350
   Lys Val Leu Val Gly Lys Asn Phe Glu Asp Val Ala Phe Asp Glu Lys
           355                 360                 365
   Lys Asn Val Phe Val Glu Phe Tyr Ala Pro Trp Cys Gly His Cys Lys
       370                 375                 380
   Gln Leu Ala Pro Ile Trp Asp Lys Leu Gly Glu Thr Tyr Lys Asp His
   385                 390                 395                 400
   Glu Asn Ile Val Ile Ala Lys Met Asp Ser Thr Ala Asn Glu Val Glu
                   405                 410                 415
   Ala Val Lys Val His Ser Phe Pro Thr Leu Lys Phe Phe Pro Ala Ser
               420                 425                 430
   Ala Asp Arg Thr Val Ile Asp Tyr Asn Gly Glu Arg Thr Leu Asp Gly
           435                 440                 445
   Phe Lys Lys Phe Leu Glu Ser Gly Gly Gln Asp Gly Ala Gly Asp Asp
       450                 455                 460
   Asp Asp Leu Glu Asp Leu Glu Glu Ala Glu Glu Pro Asp Met Glu Glu
   465 470 475 480
   Asp Asp Asp Gln Lys Ala Val
   485
<210> 22
   <211> 1461
   <212> DNA
   <213> human PDI protein coding sequence
<400> 22
   gacgcccccg aggaagagga ccacgtcctg gtcctgcgaa agtctaactt cgccgaggcc      60
   ctggccgccc acaagtacct gctggtcgaa ttctacgccc cctggtgcgg ccactgcaag     120
   gccctcgctc ccgagtacgc caaggccgct ggcaagctga aggccgaggg ctctgagatc     180
   cgactggcca aggtggacgc caccgaggaa tctgacctgg cccagcagta cggcgtgcga     240
   ggctacccca ccatcaagtt cttccgaaac ggcgacaccg cctctcccaa ggagtacacc     300
   gccggacgag aggccgacga catcgtgaac tggctgaaga agcgaaccgg acccgccgct     360
   actactctgc ccgacggcgc tgccgccgag tctctggtcg agtcctctga ggtggccgtg     420
   atcggcttct tcaaggacgt cgagtctgac tctgccaagc agttcctgca ggccgccgag     480
   gccatcgacg acattccctt cggcatcacc tctaactctg acgtgttctc taagtaccag     540
   ctggacaagg acggcgtggt gctgttcaag aagttcgacg agggccgaaa caacttcgag     600
   ggcgaggtga ccaaggaaaa cctgctggac ttcatcaagc acaaccagct gcccctggtg     660
   atcgagttca ccgagcagac cgcccccaag attttcggcg gcgagatcaa gacccacatc     720
   ctgctgtttc tgcccaagtc tgtgtctgac tacgacggca agctgtctaa cttcaagacc     780
   gccgctgagt ctttcaaggg caagatcctg ttcatcttca tcgactctga ccacaccgac     840
   aaccagcgaa tcctcgagtt cttcggcctg aagaaagaag aatgtcccgc cgtccgactg     900
   atcaccctcg aggaagagat gaccaagtac aagcccgagt ctgaggaact gaccgccgag     960
   cgaatcaccg agttctgcca ccgattcctc gagggcaaga tcaagcccca cctgatgtct    1020
   caggaactgc ccgaggactg ggataagcag cccgtgaagg tgctggtggg caagaacttc    1080
   gaggacgtgg ccttcgacga gaagaagaac gttttcgtcg agttttacgc tccttggtgt    1140
   ggacactgta agcagctggc ccccatctgg gacaagctgg gcgagactta caaggaccac    1200
   gagaacatcg tgatcgccaa gatggactct accgccaacg aggtcgaggc cgtgaaggtc    1260
   cactcgttcc ccaccctgaa gttctttccc gcctctgccg accgaaccgt gatcgactac    1320
   aacggcgagc gaaccctgga cggcttcaag aagtttctcg agtctggcgg ccaggacggc    1380
   gctggcgacg acgacgacct cgaggatctc gaagaagccg aggaacccga catggaagaa    1440
   gacgacgacc agaaggccgt c                                              1461
<210> 23
   <211> 33
   <212> PRT
   <213> Artificial Sequence
<220>
   <223> hFGE leader sequence
<400> 23
   Met Ala Ala Pro Ala Leu Gly Leu Val Cys Gly Arg Cys Pro Glu Leu
   1               5                   10                  15
   Gly Leu Val Leu Leu Leu Leu Leu Leu Ser Leu Leu Cys Gly Ala Ala
               20                  25                  30
   Gly
<210> 24
   <211> 480
   <212> PRT
   <213> Homo sapiens sulfamidase protein mature sequence
<400> 24
   Arg Pro Arg Asn Ala Leu Leu Leu Leu Ala Asp Asp Gly Gly Phe Glu
   1               5                   10                  15
   Ser Gly Ala Tyr Asn Asn Ser Ala Ile Ala Thr Pro His Leu Asp Ala
               20                  25                  30
   Leu Ala Arg Arg Ser Leu Leu Phe Arg Asn Ala Phe Thr Ser Val Ser
           35                  40                  45
   Ser Cys Ser Pro Ser Arg Ala Ser Leu Leu Thr Gly Leu Pro Gln His
       50                  55                  60
   Gln Asn Gly Met Tyr Gly Leu His Gln Asp Val His His Phe Asn Ser
   65                  70                  75                  80
   Phe Asp Lys Val Arg Ser Leu Pro Leu Leu Leu Ser Gln Ala Gly Val
                   85                  90                  95
   Arg Thr Gly Ile Ile Gly Lys Lys His Val Gly Pro Glu Thr Val Tyr
               100                 105                 110
   Pro Phe Asp Phe Ala Tyr Thr Glu Glu Asn Gly Ser Val Leu Gln Val
           115                 120                 125
   Gly Arg Asn Ile Thr Arg Ile Lys Leu Leu Val Arg Lys Phe Leu Gln
   130 135 140
   Thr Gln Asp Asp Arg Pro Phe Phe Leu Tyr Val Ala Phe His Asp Pro
   145 150 155 160
   His Arg Cys Gly His Ser Gln Pro Gln Tyr Gly Thr Phe Cys Glu Lys
   165 170 175
   Phe Gly Asn Gly Glu Ser Gly Met Gly Arg Ile Pro Asp Trp Thr Pro
   180 185 190
   Gln Ala Tyr Asp Pro Leu Asp Val Leu Val Pro Tyr Phe Val Pro Asn
   195 200 205
   Thr Pro Ala Ala Arg Ala Asp Leu Ala Ala Gln Tyr Thr Thr Val Gly
   210 215 220
   Arg Met Asp Gln Gly Val Gly Leu Val Leu Gln Glu Leu Arg Asp Ala
   225 230 235 240
   Gly Val Leu Asn Asp Thr Leu Val Ile Phe Thr Ser Asp Asn Gly Ile
   245 250 255
   Pro Phe Pro Ser Gly Arg Thr Asn Leu Tyr Trp Pro Gly Thr Ala Glu
   260 265 270
   Pro Leu Leu Val Ser Ser Pro Glu His Pro Lys Arg Trp Gly Gln Val
   275 280 285
   Ser Glu Ala Tyr Val Ser Leu Leu Asp Leu Thr Pro Thr Ile Leu Asp
   290 295 300
   Trp Phe Ser Ile Pro Tyr Pro Ser Tyr Ala Ile Phe Gly Ser Lys Thr
   305 310 315 320
   Ile His Leu Thr Gly Arg Ser Leu Leu Pro Ala Leu Glu Ala Glu Pro
   325 330 335
   Leu Trp Ala Thr Val Phe Gly Ser Gln Ser His His Glu Val Thr Met
   340 345 350
   Ser Tyr Pro Met Arg Ser Val Gln His Arg His Phe Arg Leu Val His
   355 360 365
   Asn Leu Asn Phe Lys Met Pro Phe Pro Ile Asp Gln Asp Phe Tyr Val
   370 375 380
   Ser Pro Thr Phe Gln Asp Leu Leu Asn Arg Thr Thr Ala Gly Gln Pro
   385                 390                 395                 400
   Thr Gly Trp Tyr Lys Asp Leu Arg His Tyr Tyr Tyr Arg Ala Arg Trp
                   405                 410                 415
   Glu Leu Tyr Asp Arg Ser Arg Asp Pro His Glu Thr Gln Asn Leu Ala
               420                 425                 430
   Thr Asp Pro Arg Phe Ala Gln Leu Leu Glu Met Leu Arg Asp Gln Leu
           435                 440                 445
   Ala Lys Trp Gln Trp Glu Thr His Asp Pro Trp Val Cys Ala Pro Asp
       450                 455                 460
   Gly Val Leu Glu Glu Lys Leu Ser Pro Gln Cys Gln Pro Leu His Asn
   465                 470                 475                 480
<210> 25
   <211> 1440
   <212> DNA
   <213> coding sequence of mature sulfamidase (SGSH)
<400> 25
   cgaccccgaa acgccctcct cctcctcgct gatgatggcg gtttcgagtc gggtgcctac      60
   aacaactccg ctatcgctac ccctcacctc gacgctctgg ctcgacgatc tctgctcttc     120
   cgaaacgcct ttacctccgt gtcctcttgc tctccctcgc gagcttctct gctcactgga     180
   ctccctcagc accagaacgg aatgtacggc ctgcatcagg acgttcacca tttcaactct     240
   tttgataagg tccgatcgct ccctctgctc ctgtcccagg ctggtgttcg aaccggtatc     300
   attggaaaga agcacgtcgg acccgagacc gtgtaccctt tcgactttgc ttacactgag     360
   gagaacggct ccgttctgca ggtcggccga aacatcaccc gaattaagct cctggtccga     420
   aagttcctcc agactcagga cgatcgaccc ttctttctgt acgtggcctt tcacgaccct     480
   caccgatgcg gacactctca gcctcagtac ggtaccttct gtgagaagtt tggaaacggc     540
   gagtccggta tgggacgaat ccccgactgg acccctcagg cttacgaccc cctcgatgtc     600
   ctggtgcctt acttcgttcc caacacccct gctgctcgag ctgacctcgc tgctcagtac     660
   accactgtcg gccgaatgga tcagggcgtg ggtctcgttc tgcaggagct gcgagacgct     720
   ggtgtgctca acgataccct ggttatcttc acttctgaca acggtattcc ctttccttcg     780
   ggacgaacca acctgtactg gcccggaact gctgagcctc tcctggtctc gtcccctgag     840
   caccctaagc gatggggaca ggtttcggag gcttacgtct ccctcctgga cctcaccccc     900
   actatcctgg attggttctc tattccctac ccttcgtacg ccatctttgg atctaagacc     960
   attcatctga ctggacgatc cctcctgcct gctctcgagg ctgagcctct gtgggctacc    1020
   gtgttcggct cccagtctca ccatgaggtt actatgtcct accccatgcg atctgtccag    1080
   caccgacatt tccgactcgt gcacaacctg aacttcaaga tgccctttcc tatcgaccag    1140
   gatttctacg tctctcccac ctttcaggac ctcctgaacc gaaccactgc cggccagcct    1200
   accggttggt acaaggatct ccgacactac tactaccgag ctcgatggga gctgtacgac    1260
   cgatcccgag atccccatga gacccagaac ctggccactg accctcgatt cgctcagctc    1320
   ctggagatgc tccgagacca gctggccaag tggcagtggg agacccacga tccctgggtg    1380
   tgtgcccccg acggtgtgct cgaggagaag ctgtcccccc agtgtcagcc cctgcataac    1440
<210> 26
   <211> 163
   <212> PRT
   <213> MNS1 anchorage domain (AA 1-163 of XP_502939.1)
<400> 26
   Met Ser Phe Asn Ile Pro Lys Thr Thr Pro Asn Phe Ser Ala Lys Ala
   1               5                   10                  15
   Arg Lys Leu Glu Asp Gln Leu Trp Gln Ala Ser Gly Leu Glu Lys Ser
               20                  25                  30
   Lys Asp Ser Thr Leu Pro Leu Tyr Lys Asp Lys Pro Tyr Gly Glu Gly
           35                  40                  45
   Phe Val Ala Arg Thr Thr Ser Gly Arg Arg Arg Arg Asn Ile Ile Tyr
       50                  55                  60
   Gly Val Val Val Gly Leu Leu Phe Trp Ala Ile Tyr Thr Phe Ser Arg
   65                  70                  75                  80
   Ser Leu Asp Gly Asn Val Ser Leu Lys Asp Gly Ile Lys Asp Tyr Glu
                   85                  90                  95
   Phe Lys Gly Trp Lys Gly Arg Gly Lys Pro Lys Thr Asn Trp Val Ala
               100                 105                 110
   Glu Gln Asn Ala Val Lys Gln Ala Phe Val Asp Ser Trp Asn Gly Tyr
           115                 120                 125
   His Lys Tyr Ala Trp Gly Lys Asp Val Tyr Lys Pro Gln Thr Lys Thr
       130                 135                 140
   Gly Lys Asn Met Gly Pro Lys Pro Leu Gly Trp Phe Ile Val Asp Ser
   145                 150                 155                 160
   Leu Asp Ser
<210> 27
   <211> 489
   <212> DNA
   <213> Coding sequence for the MNS1 anchorage domain (AA 1-163 of
   XP_502939.1)
<400> 27
   atgtcgttca acattcccaa gaccaccccc aacttctcgg ctaaggctcg aaagctggag        60
   gatcagctct ggcaggcttc tggactcgag aagtccaagg actctaccct gcctctctac       120
   aaggataagc cctacggaga gggcttcgtg gctcgaacca cttccggccg acgacgacga       180
   aacatcatct acggcgtcgt ggttggtctg ctcttctggg ccatctacac cttttctcga       240
   tcgctggacg gtaacgtctc tctcaaggac ggaattaagg attacgagtt caagggctgg       300
   aagggtcgag gaaagcccaa gactaactgg gtggccgagc agaacgctgt taagcaggcc       360
   tttgtcgact cctggaacgg ctaccataag tacgcctggg gcaaggatgt gtacaagccc       420
   cagaccaaga ctggaaagaa catgggcccc aagcctctgg gatggttcat cgtggactct       480
   ctggattcc                                                               489
<210> 28
   <211> 118
   <212> PRT
   <213> WBP1 anchorage domain (AA 400-505 of XP_502492.1)
<400> 28
   Asp His Leu Pro Thr Thr Gly Phe Thr Met Leu Asn Pro Tyr Tyr Arg
   1               5                   10                  15
   Leu Thr Leu Glu Gln Thr Gly Thr Thr Asn Phe Ser Ala Ile Tyr Ser
               20                  25                  30
   Thr Thr Phe Lys Ile Pro Asp Gln His Gly Val Phe Thr Phe Asn Leu
           35                  40                  45
   Asp Tyr Lys Arg Pro Gly Tyr Thr Phe Ile Glu Glu Lys Thr Arg Ala
       50                  55                  60
   Thr Ile Arg His Thr Ala Asn Asp Glu Trp Pro Arg Ser Trp Glu Ile
   65                  70                  75                  80
   Thr Asn Ser Trp Val Tyr Leu Thr Ser Ala Val Met Val Val Ile Ala
                   85                  90                  95
   Trp Phe Leu Phe Val Val Phe Tyr Leu Phe Val Gly Lys Ala Asp Lys
               100                 105                 110
   Glu Ala Val His Lys Gln
           115
<210> 29
   <211> 354
   <212> DNA
   <213> Coding sequence for the WBP1 anchorage domain (AA 400-505 of
   XP_502492 .1)
<400> 29
   gatcacctcc ccaccactgg cttcaccatg ctgaacccct actaccgact gaccctcgag      60
   cagactggca ccactaactt ctccgccatc tactctacca cttttaagat tcctgaccag     120
   catggcgtgt tcacctttaa cctcgattac aagcgacccg gttacacctt catcgaggag     180
   aagacccgag ccactattcg acacaccgct aacgacgagt ggccccgatc ctgggagatc     240
   accaactctt gggtctacct gacttcggcc gtgatggtcg tgattgcttg gttcctcttc     300
   gtggtgttct acctgtttgt gggaaaggct gataaggaag ctgttcataa gcag           354
<210> 30
   <211> 373
   <212> PRT
   <213> ERp44 mature protein
<400> 30
   Glu Ile Thr Ser Leu Asp Thr Glu Asn Ile Asp Glu Ile Leu Asn Asn
   1               5                   10                  15
   Ala Asp Val Ala Leu Val Asn Phe Tyr Ala Asp Trp Cys Arg Phe Ser
               20                  25                  30
   Gln Met Leu His Pro Ile Phe Glu Glu Ala Ser Asp Val Ile Lys Glu
           35                  40                  45
   Glu Phe Pro Asn Glu Asn Gln Val Val Phe Ala Arg Val Asp Cys Asp
       50                  55                  60
   Gln His Ser Asp Ile Ala Gln Arg Tyr Arg Ile Ser Lys Tyr Pro Thr
   65                  70                  75                  80
   Leu Lys Leu Phe Arg Asn Gly Met Met Met Lys Arg Glu Tyr Arg Gly
                   85                  90                  95
   Gln Arg Ser Val Lys Ala Leu Ala Asp Tyr Ile Arg Gln Gln Lys Ser
               100                 105                 110
   Asp Pro Ile Gln Glu Ile Arg Asp Leu Ala Glu Ile Thr Thr Leu Asp
           115                 120                 125
   Arg Ser Lys Arg Asn Ile Ile Gly Tyr Phe Glu Gln Lys Asp Ser Asp
       130                 135                 140
   Asn Tyr Arg Val Phe Glu Arg Val Ala Asn Ile Leu His Asp Asp Cys
   145                 150                 155                 160
   Ala Phe Leu Ser Ala Phe Gly Asp Val Ser Lys Pro Glu Arg Tyr Ser
                   165                 170                 175
   Gly Asp Asn Ile Ile Tyr Lys Pro Pro Gly His Ser Ala Pro Asp Met
               180                 185                 190
   Val Tyr Leu Gly Ala Met Thr Asn Phe Asp Val Thr Tyr Asn Trp Ile
           195                 200                 205
   Gln Asp Lys Cys Val Pro Leu Val Arg Glu Ile Thr Phe Glu Asn Gly
       210                 215                 220
   Glu Glu Leu Thr Glu Glu Gly Leu Pro Phe Leu Ile Leu Phe His Met
   225                 230                 235                 240
   Lys Glu Asp Thr Glu Ser Leu Glu Ile Phe Gln Asn Glu Val Ala Arg
                   245                 250                 255
   Gln Leu Ile Ser Glu Lys Gly Thr Ile Asn Phe Leu His Ala Asp Cys
               260                 265                 270
   Asp Lys Phe Arg His Pro Leu Leu His Ile Gln Lys Thr Pro Ala Asp
           275                 280                 285
   Cys Pro Val Ile Ala Ile Asp Ser Phe Arg His Met Tyr Val Phe Gly
       290                 295                 300
   Asp Phe Lys Asp Val Leu Ile Pro Gly Lys Leu Lys Gln Phe Val Phe
   305                 310                 315                 320
   Asp Leu His Ser Gly Lys Leu His Arg Glu Phe His His Gly Pro Asp
                   325                 330                 335
   Pro Thr Asp Thr Ala Pro Gly Glu Gln Ala Gln Asp Val Ala Ser Ser
               340                 345                 350
   Pro Pro Glu Ser Ser Phe Gln Lys Leu Ala Pro Ser Glu Tyr Arg Tyr
           355                 360                 365
   Thr Leu Leu Arg Asp
   370
<210> 31
   <211> 1119
   <212> DNA
   <213> Coding sequence for the ERp44 mature protein
<400> 31
   gagattactt ccctggatac tgagaacatc gacgagattc tgaacaacgc cgacgtggcc      60
   ctggtcaact tctacgccga ctggtgccga ttttcccaga tgctccaccc catcttcgag     120
   gaggcttctg atgtgattaa ggaggagttc cctaacgaga accaggtcgt gtttgcccga     180
   gttgactgtg atcagcattc tgacatcgct cagcgatacc gaatttcgaa gtaccccacc     240
   ctgaagctct tccgaaacgg aatgatgatg aagcgagagt accgaggcca gcgatcggtt     300
   aaggccctgg ctgactacat ccgacagcag aagtccgacc ccatccagga gattcgagat     360
   ctggccgaga ttaccactct cgaccgatct aagcgaaaca tcattggtta cttcgagcag     420
   aaggactcgg ataactaccg agtgtttgag cgagttgcta acatcctgca cgacgattgc     480
   gccttcctct ctgcttttgg agacgtctcg aagcccgagc gatactccgg cgacaacatc     540
   atctacaagc cccctggaca ttctgcccct gacatggttt acctgggcgc tatgaccaac     600
   ttcgacgtca cttacaactg gattcaggat aagtgtgttc ccctcgtccg agagattacc     660
   tttgagaacg gcgaggagct gactgaggag ggtctccctt tcctgatcct ctttcacatg     720
   aaggaggata ccgagtccct ggagattttc cagaacgagg tggcccgaca gctgatctcc     780
   gagaagggaa ctattaactt cctccacgct gactgcgata agtttcgaca ccccctgctc     840
   catatccaga agacccccgc cgactgtcct gtcatcgcta ttgattcttt ccgacacatg     900
   tacgtcttcg gcgactttaa ggatgtgctg attcccggca agctgaagca gttcgtgttt     960
   gacctgcact ccggaaagct ccatcgagag ttccaccatg gccccgaccc taccgatact    1020
   gcccctggag agcaggccca ggacgttgct tcctctcccc ctgagtcgtc cttccagaag    1080
   ctggccccct ccgagtaccg atacaccctc ctgcgagac                         1119
<210> 32
   <211> 378
   <212> PRT
   <213> Artificial Sequence
<220>
   <223> Fusion construct: LIP2-BtFGE-6xHis-HDEL
<400> 32
   Met Lys Leu Ser Thr Ile Leu Phe Thr Ala Cys Ala Thr Leu Ala Ala
   1               5                   10                  15
   Ala Ala Gly Gly Glu Glu Ala Gly Pro Glu Ala Gly Ala Pro Ser Leu
                   20                  25                  30
   Val Gly Ser Cys Gly Cys Gly Asn Pro Gln Arg Pro Gly Ala Gln Gly
               35                  40                  45
   Ser Ser Ala Ala Ala His Arg Tyr Ser Arg Glu Ala Asn Ala Pro Gly
       50                  55                  60
   Ser Val Pro Gly Gly Arg Pro Ser Pro Pro Thr Lys Met Val Pro Ile
   65                  70                  75                  80
   Pro Ala Gly Val Phe Thr Met Gly Thr Asp Asp Pro Gln Ile Lys Gln
                   85                  90                  95
   Asp Gly Glu Ala Pro Ala Arg Arg Val Ala Ile Asp Ala Phe Tyr Met
               100                 105                 110
   Asp Ala Tyr Glu Val Ser Asn Ala Glu Phe Glu Lys Phe Val Asn Ser
           115                 120                 125
   Thr Gly Tyr Leu Thr Glu Ala Glu Lys Phe Gly Asp Ser Phe Val Phe
       130                 135                 140
   Glu Gly Met Leu Ser Glu Gln Val Lys Ser Asp Ile Gln Gln Ala Val
   145                 150                 155                 160
   Ala Ala Ala Pro Trp Trp Leu Pro Val Lys Gly Ala Asn Trp Arg His
                   165                 170                 175
   Pro Glu Gly Pro Asp Ser Thr Val Leu His Arg Pro Asp His Pro Val
               180                 185                 190
   Leu His Val Ser Trp Asn Asp Ala Val Ala Tyr Cys Thr Trp Ala Gly
           195                 200                 205
   Lys Arg Leu Pro Thr Glu Ala Glu Trp Glu Tyr Ser Cys Arg Gly Gly
       210                 215                 220
   Leu Gln Asn Arg Leu Phe Pro Trp Gly Asn Lys Leu Gln Pro Lys Gly
   225                 230                 235                 240
   Gln His Tyr Ala Asn Ile Trp Gln Gly Glu Phe Pro Val Thr Asn Thr
                   245                 250                 255
   Gly Glu Asp Gly Phe Arg Gly Thr Ala Pro Val Asp Ala Phe Pro Pro
               260                 265                 270
   Asn Gly Tyr Gly Leu Tyr Asn Ile Val Gly Asn Ala Trp Glu Trp Thr
   275 280 285
   Ser Asp Trp Trp Thr Val His His Ser Ala Glu Glu Thr Ile Asn Pro
   290 295 300
   Lys Gly Pro Pro Ser Gly Lys Asp Arg Val Lys Lys Gly Gly Ser Tyr
   305 310 315 320
   Met Cys His Lys Ser Tyr Cys Tyr Arg Tyr Arg Cys Ala Ala Arg Ser
   325 330 335
   Gln Asn Thr Pro Asp Ser Ser Ala Ser Asn Leu Gly Phe Arg Cys Ala
   340 345 350
   Ala Asp His Leu Pro Thr Thr Gly Ala Asp His Leu Pro Thr Thr Gly
   355 360 365
   His His His His His His His Asp Glu Leu
   370 375
<210> 33
   <211> 4
   <212> PRT
   <213> Artificial Sequence
<220>
   <223> RDEL tag
<400> 33
   Arg Asp Glu Leu
   1
<210> 34
   <211> 12
   <212> PRT
   <213> Artificial Sequence
<220>
   <223> Conserved sequence of Iduronate Sulfatase
<400> 34
   Cys Ala Pro Ser Arg Val Ser Phe Leu Thr Gly Arg
   1 5 10
<210> 35
   <211> 453
   <212> PRT
   <213> Artificial Sequence
<220>
   <223> MNS1-HpFGE- 6xHis fusion construct
<400> 35
   Met Ser Phe Asn Ile Pro Lys Thr Thr Pro Asn Phe Ser Ala Lys Ala
   1               5                   10                  15
   Arg Lys Leu Glu Asp Gln Leu Trp Gln Ala Ser Gly Leu Glu Lys Ser
               20                  25                  30
   Lys Asp Ser Thr Leu Pro Leu Tyr Lys Asp Lys Pro Tyr Gly Glu Gly
           35                  40                  45
   Phe Val Ala Arg Thr Thr Ser Gly Arg Arg Arg Arg Asn Ile Ile Tyr
       50                  55                  60
   Gly Val Val Val Gly Leu Leu Phe Trp Ala Ile Tyr Thr Phe Ser Arg
   65                  70                  75                  80
   Ser Leu Asp Gly Asn Val Ser Leu Lys Asp Gly Ile Lys Asp Tyr Glu
                   85                  90                  95
   Phe Lys Gly Trp Lys Gly Arg Gly Lys Pro Lys Thr Asn Trp Val Ala
               100                 105                 110
   Glu Gln Asn Ala Val Lys Gln Ala Phe Val Asp Ser Trp Asn Gly Tyr
           115                 120                 125
   His Lys Tyr Ala Trp Gly Lys Asp Val Tyr Lys Pro Gln Thr Lys Thr
       130                 135                 140
   Gly Lys Asn Met Gly Pro Lys Pro Leu Gly Trp Phe Ile Val Asp Ser
   145                 150                 155                 160
   Leu Asp Ser Met Gly Thr Asp Lys Ala Lys Ile Tyr Leu Asp Gly Glu
                   165                 170                 175
   Ser Pro Ser Arg Leu Val Thr Leu Asp Pro Tyr Tyr Phe Asp Val Tyr
               180                 185                 190
   Glu Val Ser Asn Ser Glu Phe Glu Leu Phe Val Asn Thr Thr Ser Tyr
           195                 200                 205
   Ile Thr Glu Ala Glu Lys Phe Gly Asp Ser Phe Val Leu Glu Ala Arg
       210                 215                 220
   Ile Ser Glu Glu Val Lys Lys Asp Ile Ser Gln Val Val Ala Ala Ala
   225                 230                 235                 240
   Pro Trp Trp Leu Pro Val Lys Gly Ala Glu Trp Arg His Pro Glu Gly
   245 250 255
   Pro Asp Ser Ser Ile Ser Ser Arg Met Asp His Pro Val Thr His Ile
   260 265 270
   Ser Trp Asn Asp Ala Thr Ala Tyr Cys Gln Trp Ala Gly Lys Arg Leu
   275 280 285
   Pro Thr Glu Ala Glu Trp Glu Asn Ala Ala Arg Gly Gly Leu Asn Asn
   290 295 300
   Arg Leu Phe Pro Trp Gly Asn Lys Leu Met Pro Lys Asp His His Arg
   305 310 315 320
   Val Asn Ile Trp Gln Gly Glu Phe Pro Lys Val Asn Thr Ala Glu Asp
   325 330 335
   Gly Tyr Glu Gly Thr Cys Pro Val Thr Ala Phe Glu Pro Asn Gly Tyr
   340 345 350
   Gly Leu Tyr Asn Thr Val Gly Asn Ala Trp Glu Trp Val Ala Asp Trp
   355 360 365
   Trp Thr Thr Val His Ser Pro Glu Ser Gln Asn Asn Pro Val Gly Pro
   370 375 380
   Asp Glu Gly Thr Asp Lys Val Lys Lys Gly Gly Ser Tyr Met Cys His
   385 390 395 400
   Ile Ser Tyr Cys Tyr Arg Tyr Arg Cys Glu Ala Arg Ser Gln Asn Ser
   405 410 415
   Pro Asp Ser Ser Ala Cys Asn Leu Gly Phe Arg Cys Ala Ala Thr Asn
   420 425 430
   Leu Pro Glu Asp Ile Pro Cys Ser Asn Cys Asn Asp Ser Thr Pro His
   435 440 445
   His His His His His
   450
<210> 36
   <211> 1362
   <212> DNA
   <213> Artificial Sequence
<220>
   <223> Coding sequence for MNS1-HpFGE- 6xHis fusion construct
<400> 36
   atgtcgttca acattcccaa gactacccct aacttctcgg ctaaggctcg aaagctggag      60
   gatcagctct ggcaggcttc tggactggag aagtccaagg actctaccct gcccctctac     120
   aaggataagc cttacggaga gggattcgtg gctcgaacca cctccggccg acgacgacga     180
   aacatcatct acggcgtcgt ggttggtctg ctcttctggg ctatctacac cttttcccga     240
   tctctggacg gcaacgtctc cctcaaggac ggtattaagg attacgagtt caagggatgg     300
   aagggccgag gcaagcccaa gaccaactgg gtggctgagc agaacgccgt gaagcaggct     360
   tttgttgact cttggaacgg ataccacaag tacgcctggg gcaaggatgt ctacaagccc     420
   cagaccaaga ctggaaagaa catgggcccc aagcctctgg gctggttcat cgtggactcg     480
   ctcgattcca tgggcaccga caaggccaag atctacctgg atggtgagtc gccctcccga     540
   ctggttactc tcgaccctta ctactttgat gtttacgagg tctctaactc ggagttcgag     600
   ctgtttgtca acaccacttc ttacatcacc gaggccgaga agttcggtga ctcctttgtc     660
   ctcgaggctc gaatctctga ggaagtcaag aaggatattt ctcaggtggt ggccgctgcc     720
   ccctggtggc tccctgttaa gggtgctgag tggcgacacc ctgagggacc tgactcctct     780
   atctcgtccc gaatggatca ccccgttacc catatttcct ggaacgacgc tactgcctac     840
   tgtcagtggg ctggcaagcg actgcctacc gaggctgagt gggagaacgc tgctcgaggc     900
   ggtctgaaca accgactctt cccctgggga aacaagctca tgcctaagga ccaccatcga     960
   gtgaacatct ggcagggcga gttccccaag gttaacaccg ccgaggacgg ttacgaggga    1020
   acctgccccg tgactgcttt tgagcctaac ggatacggcc tgtacaacac tgtcggaaac    1080
   gcctgggagt gggtggctga ctggtggacc actgttcact ctcccgagtc gcagaacaac    1140
   cccgttggtc ctgacgaggg aaccgataag gtcaagaagg gaggctcgta catgtgccat    1200
   atttcttact gttaccgata ccgatgcgag gcccgatccc agaactctcc cgactcttcg    1260
   gcttgtaacc tgggtttccg atgcgctgcc accaacctcc ctgaggacat tccctgctct    1320
   aactgtaacg actccactcc ccaccaccat caccatcact aa                       1362
<210> 37
   <211> 519
   <212> PRT
   <213> Artificial Sequence
<220>
   <223> MNS1-BtFGE-6xHis fusion construct
<400> 37
   Met Ser Phe Asn Ile Pro Lys Thr Thr Pro Asn Phe Ser Ala Lys Ala
   1               5                   10                  15
   Arg Lys Leu Glu Asp Gln Leu Trp Gln Ala Ser Gly Leu Glu Lys Ser
               20                  25                  30
   Lys Asp Ser Thr Leu Pro Leu Tyr Lys Asp Lys Pro Tyr Gly Glu Gly
           35                  40                  45
   Phe Val Ala Arg Thr Thr Ser Gly Arg Arg Arg Arg Asn Ile Ile Tyr
       50                  55                  60
   Gly Val Val Val Gly Leu Leu Phe Trp Ala Ile Tyr Thr Phe Ser Arg
   65                  70                  75                  80
   Ser Leu Asp Gly Asn Val Ser Leu Lys Asp Gly Ile Lys Asp Tyr Glu
                   85                  90                  95
   Phe Lys Gly Trp Lys Gly Arg Gly Lys Pro Lys Thr Asn Trp Val Ala
               100                 105                 110
   Glu Gln Asn Ala Val Lys Gln Ala Phe Val Asp Ser Trp Asn Gly Tyr
           115                 120                 125
   His Lys Tyr Ala Trp Gly Lys Asp Val Tyr Lys Pro Gln Thr Lys Thr
       130                 135                 140
   Gly Lys Asn Met Gly Pro Lys Pro Leu Gly Trp Phe Ile Val Asp Ser
   145                 150                 155                 160
   Leu Asp Ser Gly Gly Glu Glu Ala Gly Pro Glu Ala Gly Ala Pro Ser
                   165                 170                 175
   Leu Val Gly Ser Cys Gly Cys Gly Asn Pro Gln Arg Pro Gly Ala Gln
               180                 185                 190
   Gly Ser Ser Ala Ala Ala His Arg Tyr Ser Arg Glu Ala Asn Ala Pro
           195                 200                 205
   Gly Ser Val Pro Gly Gly Arg Pro Ser Pro Pro Thr Lys Met Val Pro
       210                 215                 220
   Ile Pro Ala Gly Val Phe Thr Met Gly Thr Asp Asp Pro Gln Ile Lys
   225                 230                 235                 240
   Gln Asp Gly Glu Ala Pro Ala Arg Arg Val Ala Ile Asp Ala Phe Tyr
                   245                 250                 255
   Met Asp Ala Tyr Glu Val Ser Asn Ala Glu Phe Glu Lys Phe Val Asn
               260                 265                 270
   Ser Thr Gly Tyr Leu Thr Glu Ala Glu Lys Phe Gly Asp Ser Phe Val
   275 280 285
   Phe Glu Gly Met Leu Ser Glu Gln Val Lys Ser Asp Ile Gln Gln Ala
   290 295 300
   Val Ala Ala Ala Pro Trp Trp Leu Pro Val Lys Gly Ala Asn Trp Arg
   305 310 315 320
   His Pro Glu Gly Pro Asp Ser Thr Val Leu His Arg Pro Asp His Pro
   325 330 335
   Val Leu His Val Ser Trp Asn Asp Ala Val Ala Tyr Cys Thr Trp Ala
   340 345 350
   Gly Lys Arg Leu Pro Thr Glu Ala Glu Trp Glu Tyr Ser Cys Arg Gly
   355 360 365
   Gly Leu Gln Asn Arg Leu Phe Pro Trp Gly Asn Lys Leu Gln Pro Lys
   370 375 380
   Gly Gln His Tyr Ala Asn Ile Trp Gln Gly Glu Phe Pro Val Thr Asn
   385 390 395 400
   Thr Gly Glu Asp Gly Phe Arg Gly Thr Ala Pro Val Asp Ala Phe Pro
   405 410 415
   Pro Asn Gly Tyr Gly Leu Tyr Asn Ile Val Gly Asn Ala Trp Glu Trp
   420 425 430
   Thr Ser Asp Trp Trp Thr Val His His Ser Ala Glu Glu Thr Ile Asn
   435 440 445
   Pro Lys Gly Pro Pro Ser Gly Lys Asp Arg Val Lys Lys Gly Gly Ser
   450 455 460
   Tyr Met Cys His Lys Ser Tyr Cys Tyr Arg Tyr Arg Cys Ala Ala Arg
   465 470 475 480
   Ser Gln Asn Thr Pro Asp Ser Ser Ala Ser Asn Leu Gly Phe Arg Cys
   485 490 495
   Ala Ala Asp His Leu Pro Thr Thr Gly Ala Asp His Leu Pro Thr Thr
   500 505 510
   Gly His His His His His His
   515
<210> 38
   <211> 1560
   <212> DNA
   <213> Artificial Sequence
<220>
   <223> Coding sequence for the MNS1-BtFGE-6xHis fusion construct
<400> 38
   atgtcgttca acattcccaa gaccaccccc aacttctcgg ctaaggctcg aaagctggag      60
   gatcagctct ggcaggcttc tggactcgag aagtccaagg actctaccct gcctctctac     120
   aaggataagc cctacggaga gggcttcgtg gctcgaacca cttccggccg acgacgacga     180
   aacatcatct acggcgtcgt ggttggtctg ctcttctggg ccatctacac cttttctcga     240
   tcgctggacg gtaacgtctc tctcaaggac ggaattaagg attacgagtt caagggctgg     300
   aagggtcgag gaaagcccaa gactaactgg gtggccgagc agaacgctgt taagcaggcc     360
   tttgtcgact cctggaacgg ctaccataag tacgcctggg gcaaggatgt gtacaagccc     420
   cagaccaaga ctggaaagaa catgggcccc aagcctctgg gatggttcat cgtggactct     480
   ctggattccg gcggcgagga agccggtcct gaggctggag ctccttctct ggttggctcg     540
   tgcggctgtg gaaaccccca gcgacctggt gctcagggct cctctgccgc tgcccaccga     600
   tactctcgag aggccaacgc tcccggttct gtgcctggag gccgaccttc gccccctacc     660
   aagatggtgc ccattcctgc tggagttttc accatgggca ctgacgatcc tcagatcaag     720
   caggacggag aggctcctgc tcgacgagtt gccattgacg ctttttacat ggatgcttac     780
   gaggtttcta acgccgagtt cgagaagttt gtcaactcga ccggatacct gactgaggcc     840
   gagaagttcg gagactcctt cgtctttgag ggcatgctct ccgagcaggt caagtctgac     900
   atccagcagg ctgtggctgc cgctccttgg tggctgcccg ttaagggtgc taactggcga     960
   catcctgagg gtcctgactc caccgtcctg caccgacccg atcatcctgt cctccacgtg    1020
   tcttggaacg acgccgtggc ttactgtacc tgggctggca agcgactgcc tactgaggct    1080
   gagtgggagt actcttgccg aggtggactg cagaaccgac tcttcccttg gggtaacaag    1140
   ctccagccca agggacagca ctacgccaac atttggcagg gcgagtttcc tgtcaccaac    1200
   actggcgagg acggtttccg aggaaccgct cccgtggatg cctttccccc taacggatac    1260
   ggcctgtaca acatcgtggg taacgcttgg gagtggacct ccgactggtg gactgttcac    1320
   cattctgccg aggagaccat taaccctaag ggccctccct ctggcaagga ccgagtcaag    1380
   aagggcggtt cgtacatgtg ccacaagtcc tactgttacc gataccgatg cgccgctcga    1440
   tcgcagaaca cccctgactc ttctgcttcc aacctcggct tccgatgtgc cgctgatcac    1500
   ctccccacca ctggcgctga ccacctgccc actactggac accaccacca ccaccattaa    1560
<210> 39
   <211> 485
   <212> PRT
   <213> Artificial Sequence
<220>
   <223> Lip2pre-6xHis-BtFGE-WBP1 fusion construct
<400> 39
   Met Lys Leu Ser Thr Ile Leu Phe Thr Ala Cys Ala Thr Leu Ala Ala
   1               5                   10                  15
   Ala His His His His His His Ala Gly Gly Glu Glu Ala Gly Pro Glu
               20                  25                  30
   Ala Gly Ala Pro Ser Leu Val Gly Ser Cys Gly Cys Gly Asn Pro Gln
           35                  40                  45
   Arg Pro Gly Ala Gln Gly Ser Ser Ala Ala Ala His Arg Tyr Ser Arg
       50                  55                  60
   Glu Ala Asn Ala Pro Gly Ser Val Pro Gly Gly Arg Pro Ser Pro Pro
   65                  70                  75                  80
   Thr Lys Met Val Pro Ile Pro Ala Gly Val Phe Thr Met Gly Thr Asp
                   85                  90                  95
   Asp Pro Gln Ile Lys Gln Asp Gly Glu Ala Pro Ala Arg Arg Val Ala
               100                 105                 110
   Ile Asp Ala Phe Tyr Met Asp Ala Tyr Glu Val Ser Asn Ala Glu Phe
           115                 120                 125
   Glu Lys Phe Val Asn Ser Thr Gly Tyr Leu Thr Glu Ala Glu Lys Phe
       130                 135                 140
   Gly Asp Ser Phe Val Phe Glu Gly Met Leu Ser Glu Gln Val Lys Ser
   145                 150                 155                 160
   Asp Ile Gln Gln Ala Val Ala Ala Ala Pro Trp Trp Leu Pro Val Lys
                   165                 170                 175
   Gly Ala Asn Trp Arg His Pro Glu Gly Pro Asp Ser Thr Val Leu His
               180                 185                 190
   Arg Pro Asp His Pro Val Leu His Val Ser Trp Asn Asp Ala Val Ala
           195                 200                 205
   Tyr Cys Thr Trp Ala Gly Lys Arg Leu Pro Thr Glu Ala Glu Trp Glu
       210                 215                 220
   Tyr Ser Cys Arg Gly Gly Leu Gln Asn Arg Leu Phe Pro Trp Gly Asn
   225                 230                 235                 240
   Lys Leu Gln Pro Lys Gly Gln His Tyr Ala Asn Ile Trp Gln Gly Glu
                   245                 250                 255
   Phe Pro Val Thr Asn Thr Gly Glu Asp Gly Phe Arg Gly Thr Ala Pro
               260                 265                 270
   Val Asp Ala Phe Pro Pro Asn Gly Tyr Gly Leu Tyr Asn Ile Val Gly
           275                 280                 285
   Asn Ala Trp Glu Trp Thr Ser Asp Trp Trp Thr Val His His Ser Ala
       290                 295                 300
   Glu Glu Thr Ile Asn Pro Lys Gly Pro Pro Ser Gly Lys Asp Arg Val
   305                 310                 315                 320
   Lys Lys Gly Gly Ser Tyr Met Cys His Lys Ser Tyr Cys Tyr Arg Tyr
                   325                 330                 335
   Arg Cys Ala Ala Arg Ser Gln Asn Thr Pro Asp Ser Ser Ala Ser Asn
               340                 345                 350
   Leu Gly Phe Arg Cys Ala Ala Asp His Leu Pro Thr Thr Gly Ala Asp
           355                 360                 365
   His Leu Pro Thr Thr Gly Phe Thr Met Leu Asn Pro Tyr Tyr Arg Leu
       370                 375                 380
   Thr Leu Glu Gln Thr Gly Thr Thr Asn Phe Ser Ala Ile Tyr Ser Thr
   385                 390                 395                 400
   Thr Phe Lys Ile Pro Asp Gln His Gly Val Phe Thr Phe Asn Leu Asp
                   405                 410                 415
   Tyr Lys Arg Pro Gly Tyr Thr Phe Ile Glu Glu Lys Thr Arg Ala Thr
               420                 425                 430
   Ile Arg His Thr Ala Asn Asp Glu Trp Pro Arg Ser Trp Glu Ile Thr
           435                 440                 445
   Asn Ser Trp Val Tyr Leu Thr Ser Ala Val Met Val Val Ile Ala Trp
       450                 455                 460
   Phe Leu Phe Val Val Phe Tyr Leu Phe Val Gly Lys Ala Asp Lys Glu
   465                 470                 475                 480
   Ala Val His Lys Gln
                   485
<210> 40
   <211> 1458
   <212> DNA
   <213> Artificial Sequence
<220>
   <223> Coding sequence for the Lip2pre-6xHis-BtFGE-WBP1 fusion construct
<400> 40
   atgaagctgt ctaccattct gtttactgct tgtgctaccc tggctgctgc ccaccaccat      60
   caccatcacg ctggcggaga agaggctgga cccgaggctg gagctccttc cctggtggga     120
   tcgtgtggat gtggaaaccc tcagcgacct ggagctcagg gttcttctgc cgctgcccat     180
   cgatactccc gagaggctaa cgctcctggt tctgtgcctg gcggacgacc ttctcctccc     240
   accaagatgg tccccatccc tgccggagtt ttcaccatgg gtactgacga tcctcagatc     300
   aagcaggacg gagaggctcc tgctcgacga gttgccattg acgcttttta catggatgcc     360
   tacgaggtct ctaacgctga gttcgagaag tttgttaact ccaccggata cctcactgag     420
   gccgagaagt tcggcgactc cttcgtcttt gagggaatgc tgtcggagca ggttaagtct     480
   gatattcagc aggctgtggc tgccgctcct tggtggctgc ccgtcaaggg agctaactgg     540
   cgacatcccg agggtcctga ctcgaccgtt ctgcaccgac ccgatcatcc tgttctccac     600
   gtgtcttgga acgacgctgt ggcttactgc acctgggctg gaaagcgact ccccactgag     660
   gctgagtggg agtactcttg tcgaggtggc ctgcagaacc gactcttccc ttggggtaac     720
   aagctgcagc ccaagggcca gcactacgcc aacatctggc agggagagtt tcctgttacc     780
   aacactggag aggacggatt ccgaggtacc gctcctgtgg atgcttttcc ccctaacggt     840
   tacggcctct acaacatcgt gggcaacgcc tgggagtgga cctcggactg gtggactgtc     900
   caccattctg ctgaggagac cattaacccc aagggtcccc cttctggcaa ggatcgagtg     960
   aagaagggag gttcctacat gtgtcacaag tcgtactgct accgataccg atgtgccgct    1020
   cgatcccaga acacccctga ctcgtctgcc tcgaacctgg gattccgatg cgccgctgac    1080
   catctgccta ccactggcgc tgatcacctc cccaccactg gcttcaccat gctgaacccc    1140
   tactaccgac tgaccctcga gcagactggc accactaact tctctgccat ctactccacc    1200
   acttttaaga ttcctgacca gcatggtgtc ttcaccttta acctcgatta caagcgaccc    1260
   ggctacactt tcatcgagga gaagacccga gccactattc gacacaccgc taacgacgag    1320
   tggccccgat cttgggagat caccaactcc tgggtgtacc tgacttcggc cgtcatggtg    1380
   gtcattgctt ggttcctgtt cgtcgtgttt tacctgttcg ttggcaaggc tgacaaggaa    1440
   gctgttcata agcagtaa                                                  1458
<210> 41
   <211> 380
   <212> PRT
   <213> Artificial Sequence
<220>
   <223> Chimeric Lip2pre-BtFGE-HpFGE-6xHis-HDEL fusion construct
<400> 41
   Met Lys Leu Ser Thr Ile Leu Phe Thr Ala Cys Ala Thr Leu Ala Ala
   1               5                   10                  15
   Ala Ala Gly Gly Glu Glu Ala Gly Pro Glu Ala Gly Ala Pro Ser Leu
               20                  25                  30
   Val Gly Ser Cys Gly Cys Gly Asn Pro Gln Arg Pro Gly Ala Gln Gly
           35                  40                  45
   Ser Ser Ala Ala Ala His Arg Tyr Ser Arg Glu Ala Asn Ala Pro Gly
       50                  55                  60
   Ser Val Pro Gly Gly Arg Pro Ser Pro Pro Thr Lys Met Val Pro Ile
   65                  70                  75                  80
   Pro Ala Gly Val Phe Thr Met Gly Thr Asp Lys Ala Lys Ile Tyr Leu
                   85                  90                  95
   Asp Gly Glu Ser Pro Ser Arg Leu Val Thr Leu Asp Pro Tyr Tyr Phe
               100                 105                 110
   Asp Val Tyr Glu Val Ser Asn Ser Glu Phe Glu Leu Phe Val Asn Thr
           115                 120                 125
   Thr Ser Tyr Ile Thr Glu Ala Glu Lys Phe Gly Asp Ser Phe Val Leu
       130                 135                 140
   Glu Ala Arg Ile Ser Glu Glu Val Lys Lys Asp Ile Ser Gln Val Val
   145                 150                 155                 160
   Ala Ala Ala Pro Trp Trp Leu Pro Val Lys Gly Ala Glu Trp Arg His
                   165                 170                 175
   Pro Glu Gly Pro Asp Ser Ser Ile Ser Ser Arg Met Asp His Pro Val
               180                 185                 190
   Thr His Ile Ser Trp Asn Asp Ala Thr Ala Tyr Cys Gln Trp Ala Gly
   195 200 205
   Lys Arg Leu Pro Thr Glu Ala Glu Trp Glu Asn Ala Ala Arg Gly Gly
   210 215 220
   Leu Asn Asn Arg Leu Phe Pro Trp Gly Asn Lys Leu Met Pro Lys Asp
   225 230 235 240
   His His Arg Val Asn Ile Trp Gln Gly Glu Phe Pro Lys Val Asn Thr
   245 250 255
   Ala Glu Asp Gly Tyr Glu Gly Thr Cys Pro Val Thr Ala Phe Glu Pro
   260 265 270
   Asn Gly Tyr Gly Leu Tyr Asn Thr Val Gly Asn Ala Trp Glu Trp Val
   275 280 285
   Ala Asp Trp Trp Thr Thr Val His Ser Pro Glu Ser Gln Asn Asn Pro
   290 295 300
   Val Gly Pro Asp Glu Gly Thr Asp Lys Val Lys Lys Gly Gly Ser Tyr
   305 310 315 320
   Met Cys His Ile Ser Tyr Cys Tyr Arg Tyr Arg Cys Glu Ala Arg Ser
   325 330 335
   Gln Asn Ser Pro Asp Ser Ser Ala Cys Asn Leu Gly Phe Arg Cys Ala
   340 345 350
   Ala Thr Asn Leu Pro Glu Asp Ile Pro Cys Ser Asn Cys Asn Asp Ser
   355 360 365
   Thr Pro His His His His His His His Asp Glu Leu
   370 375 380
<210> 42
   <211> 1143
   <212> DNA
   <213> Artificial Sequence
<220>
   <223> Coding sequence for Chimeric Lip2pre-BtFGE-HpFGE-6xHis-HDEL fusion construct
<400> 42
   atgaagctgt ctactattct gtttactgct tgcgctactc tggctgccgc tgccggaggc 60
   gaggaagctg gtcccgaggc tggtgctccc tctctggtgg gttcgtgcgg ctgtggaaac 120
   ccccagcgac ctggtgctca gggctcctct gccgctgccc accgatactc tcgagaggct 180
   aacgctcctg gatcggtccc tggcggtcga ccctctcccc ctaccaagat ggtgcccatc 240
   cctgccggtg ttttcaccat gggaactgac aaggctaaga tctacctgga tggcgagtcg 300
   ccttcccgac tggtcaccct cgacccctac tactttgatg tttacgaggt ctctaactcg 360
   gagttcgagc tgtttgtgaa caccacttct tacatcactg aggccgagaa gttcggtgac 420
   tcctttgtcc tcgaggctcg aatctctgag gaagtcaaga aggatatttc tcaggtggtg 480
   gctgccgctc cttggtggct ccccgttaag ggtgctgagt ggcgacaccc tgagggtcct 540
   gactcgtcca tctcttcgcg aatggatcac cctgtcaccc atatttcctg gaacgacgcc 600
   actgcttact gtcagtgggc tggcaagcga ctgcccaccg aggctgagtg ggagaacgct 660
   gctcgaggcg gcctgaacaa ccgactcttc ccttggggaa acaagctcat gcccaaggac 720
   caccatcgag tgaacatttg gcagggcgag ttccccaagg ttaacaccgc tgaggacgga 780
   tacgagggta cctgccctgt gactgctttt gagcccaacg gatacggcct ctacaacact 840
   gtcggaaacg cctgggagtg ggtggctgac tggtggacca ctgttcactc ccccgagtct 900
   cagaacaacc ccgttggacc tgacgagggc accgataagg tcaagaaggg cggctcctac 960
   atgtgccata tctcttactg ttaccgatac cgatgcgagg cccgatcgca gaactcccct 1020
   gactcctctg cttgtaacct gggtttccga tgcgccgcta ccaacctccc cgaggatatt 1080
   ccctgttcca actgtaacga ttccacccct caccaccatc accatcatca cgacgagctg 1140
   taa 1143
<210> 43
   <211> 338
   <212> PRT
   <213> Tupaia chinensis FGE
<400> 43
   Glu Glu Ala Arg Thr Gly Ala Gly Ala Thr Ser Ala Gln Gly Pro Cys
   1 5 10 15
   Gly Cys Gly Thr Pro Gln Arg Pro Gly Ser His Gly Ser Ser Ala Ala
   20 25 30
   Ala His Arg Tyr Ser Arg Glu Ala Asn Val Pro Gly Pro Val Pro Gly
   35 40 45
   Glu Arg Gln Pro Glu Ala Thr Lys Met Val Pro Ile Pro Ala Gly Val
   50 55 60
   Phe Thr Met Gly Thr Asp Asp Pro Gln Ile Lys Gln Asp Gly Glu Ala
   65 70 75 80
   Pro Ala Arg Arg Val Ala Ile Asp Ala Phe Tyr Met Asp Ala Tyr Glu
                   85                  90                  95
   Val Ser Asn Ala Glu Phe Glu Lys Phe Val Asn Ser Thr Gly Tyr Leu
               100                 105                 110
   Thr Glu Ala Glu Lys Phe Gly Asp Ser Phe Val Phe Glu Gly Met Leu
           115                 120                 125
   Ser Glu Gln Val Lys Thr Gly Ile Gln Gln Ala Val Ala Ala Ala Pro
       130                 135                 140
   Trp Trp Leu Pro Val Lys Gly Ala Asn Trp Arg His Pro Glu Gly Pro
   145                 150                 155                 160
   Asp Ser Thr Ile Leu His Arg Ala Asp His Pro Val Leu His Val Ser
                   165                 170                 175
   Trp Asn Asp Ala Val Ala Tyr Cys Thr Trp Ala Gly Lys Arg Leu Pro
               180                 185                 190
   Thr Glu Ala Glu Trp Glu Tyr Ser Cys Arg Gly Gly Leu Gln Asn Arg
           195                 200                 205
   Leu Phe Pro Trp Gly Asn Lys Leu Gln Pro Arg Gly Gln His Tyr Ala
       210                 215                 220
   Asn Ile Trp Gln Gly Glu Phe Pro Val Thr Asn Thr Ala Glu Asp Gly
   225                 230                 235                 240
   Phe Gln Gly Thr Ala Pro Val Asp Ala Phe Pro Pro Asn Gly Tyr Gly
                   245                 250                 255
   Leu Tyr Asn Ile Val Gly Asn Ala Trp Glu Trp Thr Ser Asp Trp Trp
               260                 265                 270
   Thr Val Tyr His Ser Val Glu Glu Thr Leu Asn Pro Lys Gly Pro Pro
           275                 280                 285
   Ser Gly Lys Asp Arg Val Lys Lys Gly Gly Ser Tyr Met Cys His Lys
       290                 295                 300
   Ser Tyr Cys Tyr Arg Tyr Arg Cys Ala Ala Arg Ser Gln Asn Thr Pro
   305                 310                 315                 320
   Asp Ser Ser Ala Ser Asn Leu Gly Phe Arg Cys Ala Ala Asp Arg Leu
                   325                 330                 335
   Pro Thr
<210> 44
   <211> 1014
   <212> DNA
   <213> Coding sequence for Tupaia chinensis FGE
<400> 44
   gaggaagccc gaactggtgc tggtgctact tctgctcagg gaccctgcgg ttgcggtact      60
   cctcagcgac ccggttctca cggctcgtct gccgctgccc accgatactc tcgagaggct     120
   aacgttcctg gacctgtccc cggagagcga cagcctgagg ccaccaagat ggtccctatc     180
   cccgctggcg tgttcaccat gggtactgac gatcctcaga tcaagcagga cggtgaagct     240
   cctgctcgac gagttgccat tgacgctttt tacatggatg cctacgaggt gtccaacgct     300
   gagttcgaga agtttgttaa ctctaccgga tacctgactg aggccgagaa gttcggagac     360
   tccttcgtct ttgagggcat gctctctgag caggttaaga ccggcatcca gcaggctgtg     420
   gctgccgctc cttggtggct gcctgtgaag ggagctaact ggcgacatcc tgagggtccc     480
   gactccacta ttctgcaccg agctgatcat cctgtcctcc acgtgtcttg gaacgacgcc     540
   gtcgcttact gtacctgggc tggcaagcga ctgcctactg aggctgagtg ggagtactcc     600
   tgccgaggcg gtctgcagaa ccgactcttc ccttggggta acaagctcca gccccgagga     660
   cagcactacg ccaacatctg gcagggagag tttcctgtca ccaacactgc tgaggacgga     720
   ttccagggca ccgctcctgt ggatgctttt ccccctaacg gttacggact gtacaacatt    780
   gttggaaacg cctgggagtg gacctcggac tggtggactg tgtaccattc cgttgaggag     840
   accctcaacc ccaagggtcc cccttctgga aaggatcgag tgaagaaggg aggctcgtac     900
   atgtgccaca agtcctactg ttaccgatac cgatgcgccg ctcgatctca gaacaccccc     960
   gactcctctg cctcgaacct cggattccga tgtgctgctg accgactgcc cact          1014
<210> 45
   <211> 341
   <212> PRT
   <213> Monodelphis domestica FGE
<400> 45
   Ala Ala Arg Gly Leu Gly Ser Glu Ala Gly Ser Ala Ala Ala Asp Ala
   1               5                   10                  15
   Ala His Pro Ala Gly Thr Cys Gly Cys Gly Ser Pro Gln Arg Pro Gly
               20                  25                  30
   Thr Ala Ala His Arg Tyr Ser Arg Glu Ala Asn Val Ala Glu Pro Ala
   35 40 45
   Ser Ala Glu Arg Pro Val Leu Thr Ser Gln Met Ala His Ile Pro Ala
   50 55 60
   Gly Val Phe Thr Met Gly Thr Asp Glu Pro Gln Ile Lys Gln Asp Gly
   65 70 75 80
   Glu Gly Pro Ala Arg Arg Val Arg Ile Asn Ser Phe Tyr Met Asp Leu
   85 90 95
   Tyr Glu Val Ser Asn Ala Glu Phe Glu Arg Phe Val Asn Ser Thr Gly
   100 105 110
   Tyr Val Thr Glu Ala Glu Lys Phe Gly Asp Ser Phe Val Phe Asp Ser
   115 120 125
   Met Leu Ser Asp Gln Val Lys Ser Asp Ile His Gln Ala Val Ala Ala
   130 135 140
   Ala Pro Trp Trp Leu Pro Val Lys Gly Ala Asn Trp Arg His Pro Glu
   145 150 155 160
   Gly Pro Asp Ser Ser Ile Leu His Arg Arg Asp His Pro Val Leu His
   165 170 175
   Val Ser Trp Asn Asp Ala Val Ala Tyr Cys Thr Trp Ala Gly Lys Arg
   180 185 190
   Leu Pro Thr Glu Ala Glu Trp Glu Tyr Ser Cys Arg Gly Gly Leu Glu
   195 200 205
   Asn Arg Leu Phe Pro Trp Gly Asn Lys Leu Gln Pro Lys Gly Gln His
   210 215 220
   Tyr Ala Asn Ile Trp Gln Gly Glu Phe Pro Val Ser Asn Thr Gly Glu
   225 230 235 240
   Asp Gly Tyr Gln Gly Thr Ala Pro Val Thr Ala Phe Pro Pro Asn Gly
   245 250 255
   Tyr Gly Leu Tyr Asn Ile Val Gly Asn Ala Trp Glu Trp Thr Ser Asp
   260 265 270
   Trp Trp Thr Val His His Ser Ala Asp Glu Thr Leu Asp Pro Lys Gly
   275 280 285
   Pro Pro Ser Gly Ser Asp Arg Val Lys Lys Gly Gly Ser Tyr Met Cys
       290                 295                 300
   His Lys Ser Tyr Cys Tyr Arg Tyr Arg Cys Ala Ala Arg Ser Gln Asn
   305                 310                 315                 320
   Thr Pro Asp Ser Ser Ala Ser Asn Leu Gly Phe Arg Cys Ala Ala Asp
                   325                 330                 335
   Arg Leu Pro Asp Thr
               340
<210> 46
   <211> 1023
   <212> DNA
   <213> Coding sequence for Monodelphis domestica FGE
<400> 46
   gccgcccgag gtctgggttc cgaggccggt tccgccgccg ccgacgccgc tcaccctgct      60
   ggcacttgtg gttgtggttc ccctcagcga cccggcaccg ccgctcaccg atactctcga     120
   gaggctaacg tggctgagcc tgcttctgcc gagcgacctg tgctgacttc gcagatggct     180
   cacatccccg ccggtgtctt caccatggga actgacgagc cccagatcaa gcaggatgga     240
   gagggacctg cccgacgagt tcgaattaac tcgttttaca tggacctcta cgaggtctcc     300
   aacgctgagt tcgagcgatt tgttaactcc accggttacg tcactgaggc cgagaagttc     360
   ggagactctt tcgtttttga ttccatgctg tctgaccagg tgaagtccga tatccatcag     420
   gctgtggccg ctgccccctg gtggctccct gtcaagggag ctaactggcg acaccctgag     480
   ggacctgact cctctattct gcaccgacga gatcatcccg tcctccacgt gtcttggaac     540
   gacgctgtgg cctactgtac ctgggctgga aagcgactgc ctactgaggc tgagtgggag     600
   tactcctgcc gaggcggtct ggagaaccga ctctttccct ggggcaacaa gctccagcct     660
   aagggtcagc actacgctaa catctggcag ggcgagttcc ccgtctccaa caccggagag     720
   gacggctacc agggcaccgc tcctgtgact gcctttcccc ctaacggcta cggtctgtac     780
   aacattgtgg gtaacgcttg ggagtggacc tccgactggt ggactgttca ccattctgcc     840
   gacgagaccc tcgatcccaa gggaccccct tctggctcgg atcgagttaa gaagggaggc     900
   tcgtacatgt gccacaagtc ctactgttac cgataccgat gcgctgcccg atctcagaac     960
   acccctgact cttccgcctc taacctgggc ttccgatgtg ctgctgaccg actgcctgac    1020
   act                                                                  1023
<210> 47
   <211> 329
   <212> PRT
   <213> Gallus gallus FGE
<400> 47
   Gly Lys Glu Thr Ala Pro Gly Gly Asn Cys Gly Cys Ser Ala Ser Arg
   1               5                   10                  15
   Ser Arg Gly Gly Glu Arg Glu Ala Val Ala Thr Val Arg Arg Tyr Ser
               20                  25                  30
   Ala Ala Ala Asn Asp Gly Arg Ser Ser Gly Arg Gly Pro Met Val Ala
           35                  40                  45
   Ile Pro Gly Gly Val Phe Thr Met Gly Thr Asp Glu Pro Glu Ile Gln
       50                  55                  60
   Gln Asp Gly Glu Trp Pro Ala Arg Arg Val His Val Asn Ser Phe Tyr
   65                  70                  75                  80
   Met Asp Gln Tyr Glu Val Ser Asn Gln Glu Phe Glu Arg Phe Val Asn
                   85                  90                  95
   Ser Thr Gly Tyr Leu Thr Glu Ala Glu Lys Phe Gly Asp Ser Phe Val
               100                 105                 110
   Phe Glu Gly Met Leu Ser Glu Glu Val Lys Ala Glu Ile His Gln Ala
           115                 120                 125
   Val Ala Ala Ala Pro Trp Trp Leu Pro Val Lys Gly Ala Asn Trp Arg
       130                 135                 140
   Gln Pro Glu Gly Pro Gly Ser Ser Ile Leu Ser Arg Met Asp His Pro
   145                 150                 155                 160
   Val Leu His Val Ser Trp Asn Asp Ala Val Ala Phe Cys Thr Trp Ala
                   165                 170                 175
   Gly Lys Arg Leu Pro Thr Glu Ala Glu Trp Glu Tyr Gly Cys Arg Gly
               180                 185                 190
   Gly Leu Glu Lys Arg Leu Phe Pro Trp Gly Asn Lys Leu Gln Pro Lys
           195                 200                 205
   Gly Gln His Tyr Ala Asn Ile Trp Gln Gly Val Phe Pro Thr Asn Asn
       210                 215                 220
   Thr Ala Glu Asp Gly Tyr Lys Gly Thr Ala Pro Val Thr Ala Phe Pro
   225                 230                 235                 240
   Pro Asn Gly Tyr Gly Leu Tyr Asn Ile Val Gly Asn Ala Trp Glu Trp
                   245                 250                 255
   Thr Ser Asp Trp Trp Ala Val His His Ser Ala Asp Glu Ala His Asn
               260                 265                 270
   Pro Lys Gly Pro Ser Ser Gly Thr Asp Arg Val Lys Lys Gly Gly Ser
           275                 280                 285
   Tyr Met Cys His Lys Ser Tyr Cys Tyr Arg Tyr Arg Cys Ala Ala Arg
       290                 295                 300
   Ser Gln Asn Thr Pro Asp Ser Ser Ala Ser Asn Leu Gly Phe Arg Cys
   305                 310                 315                 320
   Ala Ala Asp Ala Leu Pro Asp Pro Gln
                   325
<210> 48
   <211> 987
   <212> DNA
   <213> Coding sequence for Gallus gallus FGE
<400> 48
   ggcaaggaga ctgcccctgg cggtaactgc ggttgttctg cttcccgatc ccgaggtgga       60
   gagcgagagg ccgttgctac tgtccgacga tactccgccg ctgccaacga cggccgatcc      120
   tctggccgag gtcccatggt ggctatccct ggcggtgttt tcaccatggg aactgacgag      180
   cccgagattc agcaggatgg cgagtggcct gctcgacgag tccacgtgaa ctcgttttac      240
   atggaccagt acgaggtttc taaccaggag ttcgagcgat ttgtcaactc taccggatac      300
   ctgactgagg ccgagaagtt cggcgactct ttcgtttttg agggaatgct ctcggaggaa      360
   gtcaaggccg agatccatca ggctgttgct gccgctcctt ggtggctgcc tgtgaagggt      420
   gctaactggc gacagcctga gggacctggc tcgtccattc tgtcccgaat ggaccacccc      480
   gttctccatg tctcttggaa cgatgccgtc gctttctgta cctgggctgg caagcgactg      540
   cctactgagg ctgagtggga gtacggatgc cgaggcggcc tggagaagcg actctttccc      600
   tggggcaaca agctccagcc taagggtcag cactacgcca acatctggca gggcgtcttc      660
   cccaccaaca acactgctga ggacggctac aagggcaccg cccctgtgac tgcttttccc      720
   cctaacggtt acggactgta caacattgtg ggtaacgcct gggagtggac ctctgactgg      780
   tgggctgttc accattctgc cgatgaggct cacaacccca agggaccttc ttcgggcacc      840
   gaccgagtga agaagggtgg atcgtacatg tgccataagt cctactgtta ccgataccga      900
   tgcgccgctc gatcccagaa cacccccgat tcctctgcct ctaacctcgg tttccgatgt      960
   gccgccgacg ccctccccga ccctcag                                          987
<210> 49
   <211> 312
   <212> PRT
   <213> Dendroctonus ponderosa FGE
<400> 49
   Ile Cys Asp Cys Gly Cys Ser Leu Asn Arg Asp Gly Gln Cys Asn Ser
   1               5                   10                  15
   Glu Asp Asn Glu Ile Asn Pro Ser Gln Lys Tyr Lys Arg Asp Leu Asn
               20                  25                  30
   Glu Asn Pro Ala Asp Asn Phe Asp Lys Ser Gln Met Ala Leu Ile Gly
           35                  40                  45
   Lys Gly Ile Phe Glu Met Gly Thr Asn Lys Pro Val Phe Pro Ser Asp
       50                  55                  60
   Phe Glu Gly Pro Ala Arg Asn Val Thr Ile Glu Asn Ser Phe Tyr Leu
   65                  70                  75                  80
   Asp Leu Tyr Glu Val Ser Asn Gln Gln Phe Tyr Asp Phe Val Arg Thr
                   85                  90                  95
   Thr Asn Tyr Lys Thr Glu Ala Glu Gln Phe Gly Asp Ser Phe Val Phe
               100                 105                 110
   Glu Met Ser Leu Pro Glu Asn Gln Arg Asn Glu His Gln Asp Ile Arg
           115                 120                 125
   Ala Ala Gln Ala Pro Trp Trp Ile Lys Leu Pro Asp Ala Tyr Trp Lys
       130                 135                 140
   His Pro Glu Gly Pro Lys Ser Thr Ile Glu Asp Arg Met Asn His Pro
   145                 150                 155                 160
   Val Ala His Val Ser Trp Asn Asp Ala Val Ala Tyr Cys Glu Tyr Val
                   165                 170                 175
   Gly Lys Arg Leu Pro Thr Glu Ala Glu Trp Glu Met Ala Cys Arg Gly
               180                 185                 190
   Gly Leu Arg Gln Lys Met Tyr Pro Trp Gly Asn Lys Leu Gln Pro Lys
           195                 200                 205
   Gly Gln His Trp Ala Asn Ile Trp Gln Gly Glu Phe Pro Lys Glu Asn
       210                 215                 220
   Thr Ala Glu Asp Gly Tyr Ile Phe Thr Cys Pro Val Asp Lys Phe Pro
   225                 230                 235                 240
   Pro Asn Gln Phe Gly Leu Tyr Asn Met Ala Gly Asn Val Trp Glu Trp
                   245                 250                 255
   Val Gln Asp Asp Trp Gln Thr Asp Pro Gln Asn Ser Arg Val Lys Lys
               260                 265                 270
   Gly Gly Ser Phe Leu Cys His Gln Ser Tyr Cys Trp Arg Tyr Arg Cys
           275                 280                 285
   Ala Ala Arg Ser Phe Asn Thr Lys Asp Ser Ser Ala Ala Asn Leu Gly
       290                 295                 300
   Phe Arg Cys Ala Ala Asp Ala Arg
   305                 310
<210> 50
   <211> 936
   <212> DNA
   <213> Coding sequence for the Dendroctonus ponderosa FGE
<400> 50
   atttgcgact gcggctgctc cctgaaccga gacggccagt gtaactccga ggacaacgag      60
   attaacccct cccagaagta caagcgagac ctgaacgaga accccgccga caacttcgat     120
   aagtctcaga tggctctcat cggcaaggga atttttgaga tgggcaccaa caagcccgtt     180
   ttcccttcgg actttgaggg tcctgcccga aacgtcacta tcgagaactc cttctacctg     240
   gacctctacg aggtctctaa ccagcagttc tacgattttg tgcgaaccac taactacaag     300
   accgaggctg agcagttcgg tgactcgttc gtctttgaga tgtccctgcc cgagaaccag     360
   cgaaacgagc accaggacat ccgagctgct caggctcctt ggtggattaa gctccctgat     420
   gcttactgga agcatcccga gggacctaag tcgaccattg aggaccgaat gaaccacccc     480
   gtcgcccatg tgtcctggaa cgatgccgtg gcttactgtg agtacgttgg caagcgactg     540
   cctactgagg ctgagtggga gatggcttgc cgaggcggtc tgcgacagaa gatgtacccc     600
   tggggaaaca agctccagcc taagggccag cactgggcca acatctggca gggagagttc     660
   cccaaggaga acaccgctga ggacggatac atttttactt gtcctgtgga taagttccct     720
   cccaaccagt ttggcctcta caacatggcc ggtaacgttt gggagtgggt ccaggacgat     780
   tggcagaccg acccccagaa ctcccgagtt aagaagggag gctctttcct gtgccatcag     840
   tcgtactgtt ggcgataccg atgcgccgct cgatctttca acaccaagga ctcctctgcc     900
   gctaacctcg gattccgatg tgctgctgac gcccga                               936
<210> 51
   <211> 284
   <212> PRT
   <213> Columba livia FGE
<400> 51
   Met Val Val Ile Pro Gly Gly Val Phe Thr Met Gly Thr Asp Glu Pro
   1               5                   10                  15
   Ala Ile Gln Gln Asp Gly Glu Trp Pro Val Arg Lys Val His Val Asn
               20                  25                  30
   Ser Phe Tyr Met Asp Arg Tyr Glu Val Ser Asn Glu Asp Phe Glu Arg
           35                  40                  45
   Phe Val Asn Ser Thr Gly Tyr Val Thr Glu Ala Glu Lys Phe Gly Asp
       50                  55                  60
   Ser Phe Val Phe Glu Gly Met Leu Ser Glu Glu Val Lys Ala Glu Ile
   65                  70                  75                  80
   His Gln Ala Val Ala Ala Ala Pro Trp Trp Leu Pro Val Lys Gly Ala
                   85                  90                  95
   Asn Trp Lys His Pro Glu Gly Pro Asp Ser Asn Ile Ser Asn Arg Met
               100                 105                 110
   Asp His Pro Val Leu His Val Ser Trp Asn Asp Ala Val Ala Phe Cys
           115                 120                 125
   Thr Trp Ala Gly Lys Arg Leu Pro Thr Glu Ala Glu Trp Glu Tyr Ser
       130                 135                 140
   Cys Arg Gly Gly Leu Glu Asn Arg Leu Phe Pro Trp Gly Asn Lys Leu
   145                 150                 155                 160
   Gln Pro Lys Gly Gln His Tyr Ala Asn Ile Trp Gln Gly Val Phe Pro
                   165                 170                 175
   Thr Asn Asn Thr Ala Glu Asp Gly Tyr Lys Gly Thr Ala Pro Val Thr
               180                 185                 190
   Ala Phe Pro Pro Asn Gly Tyr Gly Leu Tyr Asn Ile Val Gly Asn Ala
           195                 200                 205
   Trp Glu Trp Thr Ala Asp Trp Trp Ala Val His His Ser Thr Glu Glu
       210                 215                 220
   Val His Asn Pro Lys Gly Pro Ser Ser Gly Thr Asp Arg Val Lys Lys
   225 230 235 240
   Gly Gly Ser Tyr Met Cys His Lys Ser Tyr Cys Tyr Arg Tyr Arg Cys
   245 250 255
   Ala Ala Arg Ser Gln Asn Thr Pro Asp Ser Ser Ala Ser Asn Leu Gly
   260 265 270
   Phe Arg Cys Ala Ala Asp Ala Ser Pro Glu Leu Pro
   275 280
<210> 52
   <211> 852
   <212> DNA
   <213> Coding sequence for Columba livia FGE
<400> 52
   atggtcgtta ttcccggagg agtttttact atgggtactg atgagcccgc tatccagcag 60
   gacggagagt ggcccgtgcg aaaggttcac gttaactctt tctacatgga ccgatacgag 120
   gtctcgaacg aggatttcga gcgatttgtt aactccaccg gctacgtcac tgaggctgag 180
   aagtttggtg actcgttcgt ctttgaggga atgctgtccg aggaagtcaa ggctgagatc 240
   caccaggctg tggccgctgc cccctggtgg ctccctgtga agggagctaa ctggaagcat 300
   cccgagggcc ctgactctaa catttcgaac cgaatggatc accccgtcct gcatgtgtcc 360
   tggaacgatg ctgttgcctt ctgtacctgg gctggcaagc gactgcctac tgaggccgag 420
   tgggagtact cttgccgagg cggtctggag aaccgactct ttccctgggg caacaagctg 480
   cagcctaagg gtcagcacta cgctaacatc tggcagggtg tgttccccac caacaacact 540
   gccgaggacg gctacaaggg caccgctcct gtgactgcct ttccccctaa cggttacgga 600
   ctctacaaca ttgttggaaa cgcttgggag tggaccgctg actggtgggc tgtgcaccat 660
   tctactgagg aagtccacaa ccccaaggga ccttcctctg gcaccgatcg agtcaagaag 720
   ggaggctcct acatgtgcca taagtcttac tgttaccgat accgatgcgc tgcccgatcc 780
   cagaacaccc ccgactcgtc cgcctctaac ctgggattcc gatgtgctgc cgacgcttcg 840
   cctgagctgc cc 852
<210> 53
   <211> 365
   <212> PRT
   <213> Artificial Sequence
<220>
   <223> Tupaia chinensis Lip2-TupFGE-His6-HDEL fusion construct
<210> 54
   <211> 1098
   <212> DNA
   <213> Artificial Sequence
<220>
   <223> Coding sequence for the Lip2-TupFGE-His6-HDEL fusion protein
<400> 54
   atgaagcttt ccaccatcct cttcacagcc tgcgctaccc tggctgccgc cgaggaagcc      60
   cgaactggtg ctggtgctac ttctgctcag ggaccctgcg gttgcggtac tcctcagcga     120
   cccggttctc acggctcgtc tgccgctgcc caccgatact ctcgagaggc taacgttcct     180
   ggacctgtcc ccggagagcg acagcctgag gccaccaaga tggtccctat ccccgctggc     240
   gtgttcacca tgggtactga cgatcctcag atcaagcagg acggtgaagc tcctgctcga     300
   cgagttgcca ttgacgcttt ttacatggat gcctacgagg tgtccaacgc tgagttcgag     360
   aagtttgtta actctaccgg atacctgact gaggccgaga agttcggaga ctccttcgtc     420
   tttgagggca tgctctctga gcaggttaag accggcatcc agcaggctgt ggctgccgct     480
   ccttggtggc tgcctgtgaa gggagctaac tggcgacatc ctgagggtcc cgactccact     540
   attctgcacc gagctgatca tcctgtcctc cacgtgtctt ggaacgacgc cgtcgcttac     600
   tgtacctggg ctggcaagcg actgcctact gaggctgagt gggagtactc ctgccgaggc     660
   ggtctgcaga accgactctt cccttggggt aacaagctcc agccccgagg acagcactac     720
   gccaacatct ggcagggaga gtttcctgtc accaacactg ctgaggacgg attccagggc     780
   accgctcctg tggatgcttt tccccctaac ggttacggac tgtacaacat tgttggaaac     840
   gcctgggagt ggacctcgga ctggtggact gtgtaccatt ccgttgagga gaccctcaac     900
   cccaagggtc ccccttctgg aaaggatcga gtgaagaagg gaggctcgta catgtgccac     960
   aagtcctact gttaccgata ccgatgcgcc gctcgatctc agaacacccc cgactcctct    1020
   gcctcgaacc tcggattccg atgtgctgct gaccgactgc ccactcacca ccaccaccac    1080
   caccacgacg agctgtaa                                                  1098
<210> 55
   <211> 368
   <212> PRT
   <213> Artificial Sequence
<220>
   <223> Monodelphis domestica Lip2-MdFGE-His6-HDEL fusion construct
<400> 55
   Met Lys Leu Ser Thr Ile Leu Phe Thr Ala Cys Ala Thr Leu Ala Ala
   1               5                   10                  15
   Ala Ala Ala Arg Gly Leu Gly Ser Glu Ala Gly Ser Ala Ala Ala Asp
               20                  25                  30
   Ala Ala His Pro Ala Gly Thr Cys Gly Cys Gly Ser Pro Gln Arg Pro
           35                  40                  45
   Gly Thr Ala Ala His Arg Tyr Ser Arg Glu Ala Asn Val Ala Glu Pro
       50                  55                  60
   Ala Ser Ala Glu Arg Pro Val Leu Thr Ser Gln Met Ala His Ile Pro
   65                  70                  75                  80
   Ala Gly Val Phe Thr Met Gly Thr Asp Glu Pro Gln Ile Lys Gln Asp
                   85                  90                  95
   Gly Glu Gly Pro Ala Arg Arg Val Arg Ile Asn Ser Phe Tyr Met Asp
               100                 105                 110
   Leu Tyr Glu Val Ser Asn Ala Glu Phe Glu Arg Phe Val Asn Ser Thr
           115                 120                 125
   Gly Tyr Val Thr Glu Ala Glu Lys Phe Gly Asp Ser Phe Val Phe Asp
       130                 135                 140
   Ser Met Leu Ser Asp Gln Val Lys Ser Asp Ile His Gln Ala Val Ala
   145 150 155 160
   Ala Ala Pro Trp Trp Leu Pro Val Lys Gly Ala Asn Trp Arg His Pro
   165 170 175
   Glu Gly Pro Asp Ser Ser Ile Leu His Arg Arg Asp His Pro Val Leu
   180 185 190
   His Val Ser Trp Asn Asp Ala Val Ala Tyr Cys Thr Trp Ala Gly Lys
   195 200 205
   Arg Leu Pro Thr Glu Ala Glu Trp Glu Tyr Ser Cys Arg Gly Gly Leu
   210 215 220
   Glu Asn Arg Leu Phe Pro Trp Gly Asn Lys Leu Gln Pro Lys Gly Gln
   225 230 235 240
   His Tyr Ala Asn Ile Trp Gln Gly Glu Phe Pro Val Ser Asn Thr Gly
   245 250 255
   Glu Asp Gly Tyr Gln Gly Thr Ala Pro Val Thr Ala Phe Pro Pro Asn
   260 265 270
   Gly Tyr Gly Leu Tyr Asn Ile Val Gly Asn Ala Trp Glu Trp Thr Ser
   275 280 285
   Asp Trp Trp Thr Val His His Ser Ala Asp Glu Thr Leu Asp Pro Lys
   290 295 300
   Gly Pro Pro Ser Gly Ser Asp Arg Val Lys Lys Gly Gly Ser Tyr Met
   305 310 315 320
   Cys His Lys Ser Tyr Cys Tyr Arg Tyr Arg Cys Ala Ala Arg Ser Gln
   325 330 335
   Asn Thr Pro Asp Ser Ser Ala Ser Asn Leu Gly Phe Arg Cys Ala Ala
   340 345 350
   Asp Arg Leu Pro Asp Thr His His His His His His His Asp Glu Leu
   355 360 365
<210> 56
   <211> 1107
   <212> DNA
   <213> Artificial Sequence
<220>
   <223> Coding sequence for the Lip2-MdFGE-His6-HDEL fusion protein
<400> 56
   atgaagcttt ccaccatcct cttcacagcc tgcgctaccc tggctgccgc cgccgcccga     60
   ggtctgggtt ccgaggccgg ttccgccgcc gccgacgccg ctcaccctgc tggcacttgt    120
   ggttgtggtt cccctcagcg acccggcacc gccgctcacc gatactctcg agaggctaac    180
   gtggctgagc ctgcttctgc cgagcgacct gtgctgactt cgcagatggc tcacatcccc    240
   gccggtgtct tcaccatggg aactgacgag ccccagatca agcaggatgg agagggacct    300
   gcccgacgag ttcgaattaa ctcgttttac atggacctct acgaggtctc caacgctgag    360
   ttcgagcgat ttgttaactc caccggttac gtcactgagg ccgagaagtt cggagactct    420
   ttcgtttttg attccatgct gtctgaccag gtgaagtccg atatccatca ggctgtggcc    480
   gctgccccct ggtggctccc tgtcaaggga gctaactggc gacaccctga gggacctgac    540
   tcctctattc tgcaccgacg agatcatccc gtcctccacg tgtcttggaa cgacgctgtg    600
   gcctactgta cctgggctgg aaagcgactg cctactgagg ctgagtggga gtactcctgc    660
   cgaggcggtc tggagaaccg actctttccc tggggcaaca agctccagcc taagggtcag    720
   cactacgcta acatctggca gggcgagttc cccgtctcca acaccggaga ggacggctac    780
   cagggcaccg ctcctgtgac tgcctttccc cctaacggct acggtctgta caacattgtg    840
   ggtaacgctt gggagtggac ctccgactgg tggactgttc accattctgc cgacgagacc    900
   ctcgatccca agggaccccc ttctggctcg gatcgagtta agaagggagg ctcgtacatg    960
   tgccacaagt cctactgtta ccgataccga tgcgctgccc gatctcagaa cacccctgac   1020
   tcttccgcct ctaacctggg cttccgatgt gctgctgacc gactgcctga cactcatcac   1080
   catcatcacc accacgacga gctgtaa                                       1107
<210> 57
   <211> 356
   <212> PRT
   <213> Artificial Sequence
<220>
   <223> Gallus gallus Lip2-GgFGE-His6-HDEL fusion construct
<400> 57
   Met Lys Leu Ser Thr Ile Leu Phe Thr Ala Cys Ala Thr Leu Ala Ala
   1               5                   10                  15
   Ala Gly Lys Glu Thr Ala Pro Gly Gly Asn Cys Gly Cys Ser Ala Ser
               20                  25                  30
   Arg Ser Arg Gly Gly Glu Arg Glu Ala Val Ala Thr Val Arg Arg Tyr
           35                  40                  45
   Ser Ala Ala Ala Asn Asp Gly Arg Ser Ser Gly Arg Gly Pro Met Val
           50                  55                  60
   Ala Ile Pro Gly Gly Val Phe Thr Met Gly Thr Asp Glu Pro Glu Ile
   65                  70                  75                  80
   Gln Gln Asp Gly Glu Trp Pro Ala Arg Arg Val His Val Asn Ser Phe
                   85                  90                  95
   Tyr Met Asp Gln Tyr Glu Val Ser Asn Gln Glu Phe Glu Arg Phe Val
               100                 105                 110
   Asn Ser Thr Gly Tyr Leu Thr Glu Ala Glu Lys Phe Gly Asp Ser Phe
           115                 120                 125
   Val Phe Glu Gly Met Leu Ser Glu Glu Val Lys Ala Glu Ile His Gln
       130                 135                 140
   Ala Val Ala Ala Ala Pro Trp Trp Leu Pro Val Lys Gly Ala Asn Trp
   145                 150                 155                 160
   Arg Gln Pro Glu Gly Pro Gly Ser Ser Ile Leu Ser Arg Met Asp His
                   165                 170                 175
   Pro Val Leu His Val Ser Trp Asn Asp Ala Val Ala Phe Cys Thr Trp
               180                 185                 190
   Ala Gly Lys Arg Leu Pro Thr Glu Ala Glu Trp Glu Tyr Gly Cys Arg
           195                 200                 205
   Gly Gly Leu Glu Lys Arg Leu Phe Pro Trp Gly Asn Lys Leu Gln Pro
       210                 215                 220
   Lys Gly Gln His Tyr Ala Asn Ile Trp Gln Gly Val Phe Pro Thr Asn
   225                 230                 235                 240
   Asn Thr Ala Glu Asp Gly Tyr Lys Gly Thr Ala Pro Val Thr Ala Phe
                   245                 250                 255
   Pro Pro Asn Gly Tyr Gly Leu Tyr Asn Ile Val Gly Asn Ala Trp Glu
               260                 265                 270
   Trp Thr Ser Asp Trp Trp Ala Val His His Ser Ala Asp Glu Ala His
           275                 280                 285
   Asn Pro Lys Gly Pro Ser Ser Gly Thr Asp Arg Val Lys Lys Gly Gly
       290                 295                 300
   Ser Tyr Met Cys His Lys Ser Tyr Cys Tyr Arg Tyr Arg Cys Ala Ala
   305                 310                 315                 320
   Arg Ser Gln Asn Thr Pro Asp Ser Ser Ala Ser Asn Leu Gly Phe Arg
                   325                 330                 335
   Cys Ala Ala Asp Ala Leu Pro Asp Pro Gln His His His His His His
               340                 345                 350
   His Asp Glu Leu
           355
<210> 58
   <211> 1071
   <212> DNA
   <213> Artificial Sequence
<220>
   <223> Coding sequence for the Lip2-GgFGE-His6-HDEL fusion protein
<400> 58
   atgaagcttt ccaccatcct cttcacagcc tgcgctaccc tggctgccgc cggcaaggag      60
   actgcccctg gcggtaactg cggttgttct gcttcccgat cccgaggtgg agagcgagag     120
   gccgttgcta ctgtccgacg atactccgcc gctgccaacg acggccgatc ctctggccga     180
   ggtcccatgg tggctatccc tggcggtgtt ttcaccatgg gaactgacga gcccgagatt     240
   cagcaggatg gcgagtggcc tgctcgacga gtccacgtga actcgtttta catggaccag     300
   tacgaggttt ctaaccagga gttcgagcga tttgtcaact ctaccggata cctgactgag     360
   gccgagaagt tcggcgactc tttcgttttt gagggaatgc tctcggagga agtcaaggcc     420
   gagatccatc aggctgttgc tgccgctcct tggtggctgc ctgtgaaggg tgctaactgg     480
   cgacagcctg agggacctgg ctcgtccatt ctgtcccgaa tggaccaccc cgttctccat     540
   gtctcttgga acgatgccgt cgctttctgt acctgggctg gcaagcgact gcctactgag     600
   gctgagtggg agtacggatg ccgaggcggc ctggagaagc gactctttcc ctggggcaac     660
   aagctccagc ctaagggtca gcactacgcc aacatctggc agggcgtctt ccccaccaac     720
   aacactgctg aggacggcta caagggcacc gcccctgtga ctgcttttcc ccctaacggt     780
   tacggactgt acaacattgt gggtaacgcc tgggagtgga cctctgactg gtgggctgtt     840
   caccattctg ccgatgaggc tcacaacccc aagggacctt cttcgggcac cgaccgagtg     900
   aagaagggtg gatcgtacat gtgccataag tcctactgtt accgataccg atgcgccgct     960
   cgatcccaga acacccccga ttcctctgcc tctaacctcg gtttccgatg tgccgccgac    1020
   gccctccccg accctcagca tcaccatcac catcatcacg acgagctgta g             1071
<210> 59
   <211> 339
   <212> PRT
   <213> Artificial Sequence
<220>
   <223> Dendroctonus ponderosa Lip2-DpFGE-His6-HDEL fusion construct
<400> 59
   Met Lys Leu Ser Thr Ile Leu Phe Thr Ala Cys Ala Thr Leu Ala Ala
   1               5                   10                  15
   Ala Ile Cys Asp Cys Gly Cys Ser Leu Asn Arg Asp Gly Gln Cys Asn
               20                  25                  30
   Ser Glu Asp Asn Glu Ile Asn Pro Ser Gln Lys Tyr Lys Arg Asp Leu
           35                  40                  45
   Asn Glu Asn Pro Ala Asp Asn Phe Asp Lys Ser Gln Met Ala Leu Ile
       50                  55                  60
   Gly Lys Gly Ile Phe Glu Met Gly Thr Asn Lys Pro Val Phe Pro Ser
   65                  70                  75                  80
   Asp Phe Glu Gly Pro Ala Arg Asn Val Thr Ile Glu Asn Ser Phe Tyr
                   85                  90                  95
   Leu Asp Leu Tyr Glu Val Ser Asn Gln Gln Phe Tyr Asp Phe Val Arg
               100                 105                 110
   Thr Thr Asn Tyr Lys Thr Glu Ala Glu Gln Phe Gly Asp Ser Phe Val
           115                 120                 125
   Phe Glu Met Ser Leu Pro Glu Asn Gln Arg Asn Glu His Gln Asp Ile
       130                 135                 140
   Arg Ala Ala Gln Ala Pro Trp Trp Ile Lys Leu Pro Asp Ala Tyr Trp
   145                 150                 155                 160
   Lys His Pro Glu Gly Pro Lys Ser Thr Ile Glu Asp Arg Met Asn His
                   165                 170                 175
   Pro Val Ala His Val Ser Trp Asn Asp Ala Val Ala Tyr Cys Glu Tyr
               180                 185                 190
   Val Gly Lys Arg Leu Pro Thr Glu Ala Glu Trp Glu Met Ala Cys Arg
           195                 200                 205
   Gly Gly Leu Arg Gln Lys Met Tyr Pro Trp Gly Asn Lys Leu Gln Pro
       210                 215                 220
   Lys Gly Gln His Trp Ala Asn Ile Trp Gln Gly Glu Phe Pro Lys Glu
   225 230 235 240
   Asn Thr Ala Glu Asp Gly Tyr Ile Phe Thr Cys Pro Val Asp Lys Phe
   245 250 255
   Pro Pro Asn Gln Phe Gly Leu Tyr Asn Met Ala Gly Asn Val Trp Glu
   260 265 270
   Trp Val Gln Asp Asp Trp Gln Thr Asp Pro Gln Asn Ser Arg Val Lys
   275 280 285
   Lys Gly Gly Ser Phe Leu Cys His Gln Ser Tyr Cys Trp Arg Tyr Arg
   290 295 300
   Cys Ala Ala Arg Ser Phe Asn Thr Lys Asp Ser Ser Ala Ala Asn Leu
   305 310 315 320
   Gly Phe Arg Cys Ala Ala Asp Ala Arg His His His His His His His
   325 330 335
   Asp Glu Leu
<210> 60
   <211> 1020
   <212> DNA
   <213> Artificial Sequence
<220>
   <223> Coding sequence for the Lip2-DpFGE-His6-HDEL fusion protein
<400> 60
   atgaagcttt ccaccatcct cttcacagcc tgcgctaccc tggctgccgc catttgcgac 60
   tgcggctgct ccctgaaccg agacggccag tgtaactccg aggacaacga gattaacccc 120
   tcccagaagt acaagcgaga cctgaacgag aaccccgccg acaacttcga taagtctcag 180
   atggctctca tcggcaaggg aatttttgag atgggcacca acaagcccgt tttcccttcg 240
   gactttgagg gtcctgcccg aaacgtcact atcgagaact ccttctacct ggacctctac 300
   gaggtctcta accagcagtt ctacgatttt gtgcgaacca ctaactacaa gaccgaggct 360
   gagcagttcg gtgactcgtt cgtctttgag atgtccctgc ccgagaacca gcgaaacgag 420
   caccaggaca tccgagctgc tcaggctcct tggtggatta agctccctga tgcttactgg 480
   aagcatcccg agggacctaa gtcgaccatt gaggaccgaa tgaaccaccc cgtcgcccat 540
   gtgtcctgga acgatgccgt ggcttactgt gagtacgttg gcaagcgact gcctactgag 600
   gctgagtggg agatggcttg ccgaggcggt ctgcgacaga agatgtaccc ctggggaaac      660
   aagctccagc ctaagggcca gcactgggcc aacatctggc agggagagtt ccccaaggag      720
   aacaccgctg aggacggata catttttact tgtcctgtgg ataagttccc tcccaaccag      780
   tttggcctct acaacatggc cggtaacgtt tgggagtggg tccaggacga ttggcagacc      840
   gacccccaga actcccgagt taagaaggga ggctctttcc tgtgccatca gtcgtactgt      900
   tggcgatacc gatgcgccgc tcgatctttc aacaccaagg actcctctgc cgctaacctc      960
   ggattccgat gtgctgctga cgcccgacac caccaccacc accaccacga cgagctgtag     1020
<210> 61
   <211> 311
   <212> PRT
   <213> Artificial Sequence
<220>
   <223> Columba livia Lip2-ClFGE-His6-HDEL fusion construct
<400> 61
   Met Lys Leu Ser Thr Ile Leu Phe Thr Ala Cys Ala Thr Leu Ala Ala
   1               5                   10                  15
   Ala Met Val Val Ile Pro Gly Gly Val Phe Thr Met Gly Thr Asp Glu
               20                  25                  30
   Pro Ala Ile Gln Gln Asp Gly Glu Trp Pro Val Arg Lys Val His Val
           35                  40                  45
   Asn Ser Phe Tyr Met Asp Arg Tyr Glu Val Ser Asn Glu Asp Phe Glu
       50                  55                  60
   Arg Phe Val Asn Ser Thr Gly Tyr Val Thr Glu Ala Glu Lys Phe Gly
   65                  70                  75                  80
   Asp Ser Phe Val Phe Glu Gly Met Leu Ser Glu Glu Val Lys Ala Glu
                   85                  90                  95
   Ile His Gln Ala Val Ala Ala Ala Pro Trp Trp Leu Pro Val Lys Gly
               100                 105                 110
   Ala Asn Trp Lys His Pro Glu Gly Pro Asp Ser Asn Ile Ser Asn Arg
           115                 120                 125
   Met Asp His Pro Val Leu His Val Ser Trp Asn Asp Ala Val Ala Phe
       130                 135                 140
   Cys Thr Trp Ala Gly Lys Arg Leu Pro Thr Glu Ala Glu Trp Glu Tyr
   145                 150                 155                 160
   Ser Cys Arg Gly Gly Leu Glu Asn Arg Leu Phe Pro Trp Gly Asn Lys
                   165                 170                 175
   Leu Gln Pro Lys Gly Gln His Tyr Ala Asn Ile Trp Gln Gly Val Phe
               180                 185                 190
   Pro Thr Asn Asn Thr Ala Glu Asp Gly Tyr Lys Gly Thr Ala Pro Val
           195                 200                 205
   Thr Ala Phe Pro Pro Asn Gly Tyr Gly Leu Tyr Asn Ile Val Gly Asn
       210                 215                 220
   Ala Trp Glu Trp Thr Ala Asp Trp Trp Ala Val His His Ser Thr Glu
   225                 230                 235                 240
   Glu Val His Asn Pro Lys Gly Pro Ser Ser Gly Thr Asp Arg Val Lys
                   245                 250                 255
   Lys Gly Gly Ser Tyr Met Cys His Lys Ser Tyr Cys Tyr Arg Tyr Arg
               260                 265                 270
   Cys Ala Ala Arg Ser Gln Asn Thr Pro Asp Ser Ser Ala Ser Asn Leu
           275                 280                 285
   Gly Phe Arg Cys Ala Ala Asp Ala Ser Pro Glu Leu Pro His His His
       290                 295                 300
   His His His His Asp Glu Leu
   305                 310
<210> 62
   <211> 936
   <212> DNA
   <213> Artificial Sequence
<220>
   <223> Coding sequence for the Lip2-ClFGE-His6-HDEL fusion protein
<400> 62
   atgaagcttt ccaccatcct cttcacagcc tgcgctaccc tggctgccgc catggtcgtt      60
   attcccggag gagtttttac tatgggtact gatgagcccg ctatccagca ggacggagag     120
   tggcccgtgc gaaaggttca cgttaactct ttctacatgg accgatacga ggtctcgaac     180
   gaggatttcg agcgatttgt taactccacc ggctacgtca ctgaggctga gaagtttggt     240
   gactcgttcg tctttgaggg aatgctgtcc gaggaagtca aggctgagat ccaccaggct     300
   gtggccgctg ccccctggtg gctccctgtg aagggagcta actggaagca tcccgagggc     360
   cctgactcta acatttcgaa ccgaatggat caccccgtcc tgcatgtgtc ctggaacgat      420
   gctgttgcct tctgtacctg ggctggcaag cgactgccta ctgaggccga gtgggagtac      480
   tcttgccgag gcggtctgga gaaccgactc tttccctggg gcaacaagct gcagcctaag      540
   ggtcagcact acgctaacat ctggcagggt gtgttcccca ccaacaacac tgccgaggac      600
   ggctacaagg gcaccgctcc tgtgactgcc tttcccccta acggttacgg actctacaac      660
   attgttggaa acgcttggga gtggaccgct gactggtggg ctgtgcacca ttctactgag      720
   gaagtccaca accccaaggg accttcctct ggcaccgatc gagtcaagaa gggaggctcc      780
   tacatgtgcc ataagtctta ctgttaccga taccgatgcg ctgcccgatc ccagaacacc      840
   cccgactcgt ccgcctctaa cctgggattc cgatgtgctg ccgacgcttc gcctgagctg      900
   ccccaccacc accatcacca tcacgacgag ctgtaa                                936
<210> 63
   <211> 447
   <212> PRT
   <213> Artificial Sequence
<220>
   <223> MNS1-ClFGE fusion construct
<400> 63
   Met Ser Phe Asn Ile Pro Lys Thr Thr Pro Asn Phe Ser Ala Lys Ala
   1               5                   10                  15
   Arg Lys Leu Glu Asp Gln Leu Trp Gln Ala Ser Gly Leu Glu Lys Ser
               20                  25                  30
   Lys Asp Ser Thr Leu Pro Leu Tyr Lys Asp Lys Pro Tyr Gly Glu Gly
           35                  40                  45
   Phe Val Ala Arg Thr Thr Ser Gly Arg Arg Arg Arg Asn Ile Ile Tyr
       50                  55                  60
   Gly Val Val Val Gly Leu Leu Phe Trp Ala Ile Tyr Thr Phe Ser Arg
   65                  70                  75                  80
   Ser Leu Asp Gly Asn Val Ser Leu Lys Asp Gly Ile Lys Asp Tyr Glu
                   85                  90                  95
   Phe Lys Gly Trp Lys Gly Arg Gly Lys Pro Lys Thr Asn Trp Val Ala
               100                 105                 110
   Glu Gln Asn Ala Val Lys Gln Ala Phe Val Asp Ser Trp Asn Gly Tyr
           115                 120                 125
   His Lys Tyr Ala Trp Gly Lys Asp Val Tyr Lys Pro Gln Thr Lys Thr
       130                 135                 140
   Gly Lys Asn Met Gly Pro Lys Pro Leu Gly Trp Phe Ile Val Asp Ser
   145                 150                 155                 160
   Leu Asp Ser Met Val Val Ile Pro Gly Gly Val Phe Thr Met Gly Thr
                   165                 170                 175
   Asp Glu Pro Ala Ile Gln Gln Asp Gly Glu Trp Pro Val Arg Lys Val
               180                 185                 190
   His Val Asn Ser Phe Tyr Met Asp Arg Tyr Glu Val Ser Asn Glu Asp
           195                 200                 205
   Phe Glu Arg Phe Val Asn Ser Thr Gly Tyr Val Thr Glu Ala Glu Lys
       210                 215                 220
   Phe Gly Asp Ser Phe Val Phe Glu Gly Met Leu Ser Glu Glu Val Lys
   225                 230                 235                 240
   Ala Glu Ile His Gln Ala Val Ala Ala Ala Pro Trp Trp Leu Pro Val
                   245                 250                 255
   Lys Gly Ala Asn Trp Lys His Pro Glu Gly Pro Asp Ser Asn Ile Ser
               260                 265                 270
   Asn Arg Met Asp His Pro Val Leu His Val Ser Trp Asn Asp Ala Val
           275                 280                 285
   Ala Phe Cys Thr Trp Ala Gly Lys Arg Leu Pro Thr Glu Ala Glu Trp
       290                 295                 300
   Glu Tyr Ser Cys Arg Gly Gly Leu Glu Asn Arg Leu Phe Pro Trp Gly
   305                 310                 315                 320
   Asn Lys Leu Gln Pro Lys Gly Gln His Tyr Ala Asn Ile Trp Gln Gly
                   325                 330                 335
   Val Phe Pro Thr Asn Asn Thr Ala Glu Asp Gly Tyr Lys Gly Thr Ala
               340                 345                 350
   Pro Val Thr Ala Phe Pro Pro Asn Gly Tyr Gly Leu Tyr Asn Ile Val
           355                 360                 365
   Gly Asn Ala Trp Glu Trp Thr Ala Asp Trp Trp Ala Val His His Ser
       370                 375                 380
   Thr Glu Glu Val His Asn Pro Lys Gly Pro Ser Ser Gly Thr Asp Arg
   385                 390                 395                 400
   Val Lys Lys Gly Gly Ser Tyr Met Cys His Lys Ser Tyr Cys Tyr Arg
                   405                 410                 415
   Tyr Arg Cys Ala Ala Arg Ser Gln Asn Thr Pro Asp Ser Ser Ala Ser
               420                 425                 430
   Asn Leu Gly Phe Arg Cys Ala Ala Asp Ala Ser Pro Glu Leu Pro
           435                 440                 445
<210> 64
   <211> 1341
   <212> DNA
   <213> Artificial Sequence
<220>
   <223> Coding sequence for the MNS1-ClFGE fusion protein
<400> 64
   atgtcgttca acattcccaa gaccaccccc aacttctcgg ctaaggctcg aaagctggag      60
   gatcagctct ggcaggcttc tggactcgag aagtccaagg actctaccct gcctctctac     120
   aaggataagc cctacggaga gggcttcgtg gctcgaacca cttccggccg acgacgacga     180
   aacatcatct acggcgtcgt ggttggtctg ctcttctggg ccatctacac cttttctcga     240
   tcgctggacg gtaacgtctc tctcaaggac ggaattaagg attacgagtt caagggctgg     300
   aagggtcgag gaaagcccaa gactaactgg gtggccgagc agaacgctgt taagcaggcc     360
   tttgtcgact cctggaacgg ctaccataag tacgcctggg gcaaggatgt gtacaagccc     420
   cagaccaaga ctggaaagaa catgggcccc aagcctctgg gatggttcat cgtggactct     480
   ctggattcca tggtcgttat tcccggagga gtttttacta tgggtactga tgagcccgct     540
   atccagcagg acggagagtg gcccgtgcga aaggttcacg ttaactcttt ctacatggac     600
   cgatacgagg tctcgaacga ggatttcgag cgatttgtta actccaccgg ctacgtcact     660
   gaggctgaga agtttggtga ctcgttcgtc tttgagggaa tgctgtccga ggaagtcaag     720
   gctgagatcc accaggctgt ggccgctgcc ccctggtggc tccctgtgaa gggagctaac     780
   tggaagcatc ccgagggccc tgactctaac atttcgaacc gaatggatca ccccgtcctg     840
   catgtgtcct ggaacgatgc tgttgccttc tgtacctggg ctggcaagcg actgcctact     900
   gaggccgagt gggagtactc ttgccgaggc ggtctggaga accgactctt tccctggggc     960
   aacaagctgc agcctaaggg tcagcactac gctaacatct ggcagggtgt gttccccacc    1020
   aacaacactg ccgaggacgg ctacaagggc accgctcctg tgactgcctt tccccctaac  1080
   ggttacggac tctacaacat tgttggaaac gcttgggagt ggaccgctga ctggtgggct    1140
   gtgcaccatt ctactgagga agtccacaac cccaagggac cttcctctgg caccgatcga    1200
   gtcaagaagg gaggctccta catgtgccat aagtcttact gttaccgata ccgatgcgct    1260
   gcccgatccc agaacacccc cgactcgtcc gcctctaacc tgggattccg atgtgctgcc    1320
   gacgcttcgc ctgagctgcc c                                               1341
<210> 65
   <211> 10
   <212> PRT
   <213> Artificial Sequence
<220>
   <223> c-myc protein tag
<400> 65
   Glu Gln Lys Leu Ile Ser Glu Glu Asp Leu
   1               5                   10
<210> 66
   <211> 33
   <212> DNA
   <213> Artificial Sequence
<220>
   <223> Coding sequence for the c-myc protein tag
<400> 66
   gaacaaaaac tcatctcaga agaggatctg taa 33
<210> 67
   <211> 457
   <212> PRT
   <213> Artificial Sequence
<220>
   <223> MNS1-ClFGE-c-myc fusion construct
<400> 67
   Met Ser Phe Asn Ile Pro Lys Thr Thr Pro Asn Phe Ser Ala Lys Ala
   1               5                   10                  15
   Arg Lys Leu Glu Asp Gln Leu Trp Gln Ala Ser Gly Leu Glu Lys Ser
               20                  25                  30
   Lys Asp Ser Thr Leu Pro Leu Tyr Lys Asp Lys Pro Tyr Gly Glu Gly
           35                  40                  45
   Phe Val Ala Arg Thr Thr Ser Gly Arg Arg Arg Arg Asn Ile Ile Tyr
       50                  55                  60
   Gly Val Val Val Gly Leu Leu Phe Trp Ala Ile Tyr Thr Phe Ser Arg
   65                  70                  75                  80
   Ser Leu Asp Gly Asn Val Ser Leu Lys Asp Gly Ile Lys Asp Tyr Glu
                   85                  90                  95
   Phe Lys Gly Trp Lys Gly Arg Gly Lys Pro Lys Thr Asn Trp Val Ala
               100                 105                 110
   Glu Gln Asn Ala Val Lys Gln Ala Phe Val Asp Ser Trp Asn Gly Tyr
           115                 120                 125
   His Lys Tyr Ala Trp Gly Lys Asp Val Tyr Lys Pro Gln Thr Lys Thr
       130                 135                 140
   Gly Lys Asn Met Gly Pro Lys Pro Leu Gly Trp Phe Ile Val Asp Ser
   145                 150                 155                 160
   Leu Asp Ser Met Val Val Ile Pro Gly Gly Val Phe Thr Met Gly Thr
                   165                 170                 175
   Asp Glu Pro Ala Ile Gln Gln Asp Gly Glu Trp Pro Val Arg Lys Val
               180                 185                 190
   His Val Asn Ser Phe Tyr Met Asp Arg Tyr Glu Val Ser Asn Glu Asp
           195                 200                 205
   Phe Glu Arg Phe Val Asn Ser Thr Gly Tyr Val Thr Glu Ala Glu Lys
       210                 215                 220
   Phe Gly Asp Ser Phe Val Phe Glu Gly Met Leu Ser Glu Glu Val Lys
   225                 230                 235                 240
   Ala Glu Ile His Gln Ala Val Ala Ala Ala Pro Trp Trp Leu Pro Val
                   245                 250                 255
   Lys Gly Ala Asn Trp Lys His Pro Glu Gly Pro Asp Ser Asn Ile Ser
               260                 265                 270
   Asn Arg Met Asp His Pro Val Leu His Val Ser Trp Asn Asp Ala Val
           275                 280                 285
   Ala Phe Cys Thr Trp Ala Gly Lys Arg Leu Pro Thr Glu Ala Glu Trp
       290                 295                 300
   Glu Tyr Ser Cys Arg Gly Gly Leu Glu Asn Arg Leu Phe Pro Trp Gly
   305                 310                 315                 320
   Asn Lys Leu Gln Pro Lys Gly Gln His Tyr Ala Asn Ile Trp Gln Gly
                   325                 330                 335
   Val Phe Pro Thr Asn Asn Thr Ala Glu Asp Gly Tyr Lys Gly Thr Ala
               340                 345                 350
   Pro Val Thr Ala Phe Pro Pro Asn Gly Tyr Gly Leu Tyr Asn Ile Val
           355                 360                 365
   Gly Asn Ala Trp Glu Trp Thr Ala Asp Trp Trp Ala Val His His Ser
       370                 375                 380
   Thr Glu Glu Val His Asn Pro Lys Gly Pro Ser Ser Gly Thr Asp Arg
   385                 390                 395                 400
   Val Lys Lys Gly Gly Ser Tyr Met Cys His Lys Ser Tyr Cys Tyr Arg
                   405                 410                 415
   Tyr Arg Cys Ala Ala Arg Ser Gln Asn Thr Pro Asp Ser Ser Ala Ser
               420                 425                 430
   Asn Leu Gly Phe Arg Cys Ala Ala Asp Ala Ser Pro Glu Leu Pro Glu
           435                 440                 445
   Gln Lys Leu Ile Ser Glu Glu Asp Leu
       450                 455
<210> 68
   <211> 1374
   <212> DNA
   <213> Artificial Sequence
<220>
   <223> Coding sequence for the MNS1-ClFGE-c-myc fusion protein
<400> 68
   atgtcgttca acattcccaa gaccaccccc aacttctcgg ctaaggctcg aaagctggag      60
   gatcagctct ggcaggcttc tggactcgag aagtccaagg actctaccct gcctctctac     120
   aaggataagc cctacggaga gggcttcgtg gctcgaacca cttccggccg acgacgacga     180
   aacatcatct acggcgtcgt ggttggtctg ctcttctggg ccatctacac cttttctcga     240
   tcgctggacg gtaacgtctc tctcaaggac ggaattaagg attacgagtt caagggctgg     300
   aagggtcgag gaaagcccaa gactaactgg gtggccgagc agaacgctgt taagcaggcc     360
   tttgtcgact cctggaacgg ctaccataag tacgcctggg gcaaggatgt gtacaagccc     420
   cagaccaaga ctggaaagaa catgggcccc aagcctctgg gatggttcat cgtggactct     480
   ctggattcca tggtcgttat tcccggagga gtttttacta tgggtactga tgagcccgct     540
   atccagcagg acggagagtg gcccgtgcga aaggttcacg ttaactcttt ctacatggac     600
   cgatacgagg tctcgaacga ggatttcgag cgatttgtta actccaccgg ctacgtcact     660
   gaggctgaga agtttggtga ctcgttcgtc tttgagggaa tgctgtccga ggaagtcaag     720
   gctgagatcc accaggctgt ggccgctgcc ccctggtggc tccctgtgaa gggagctaac     780
   tggaagcatc ccgagggccc tgactctaac atttcgaacc gaatggatca ccccgtcctg     840
   catgtgtcct ggaacgatgc tgttgccttc tgtacctggg ctggcaagcg actgcctact     900
   gaggccgagt gggagtactc ttgccgaggc ggtctggaga accgactctt tccctggggc     960
   aacaagctgc agcctaaggg tcagcactac gctaacatct ggcagggtgt gttccccacc    1020
   aacaacactg ccgaggacgg ctacaagggc accgctcctg tgactgcctt tccccctaac    1080
   ggttacggac tctacaacat tgttggaaac gcttgggagt ggaccgctga ctggtgggct    1140
   gtgcaccatt ctactgagga agtccacaac cccaagggac cttcctctgg caccgatcga    1200
   gtcaagaagg gaggctccta catgtgccat aagtcttact gttaccgata ccgatgcgct    1260
   gcccgatccc agaacacccc cgactcgtcc gcctctaacc tgggattccg atgtgctgcc    1320
   gacgcttcgc ctgagctgcc cgaacaaaaa ctcatctcag aagaggatct gtaa          1374